# FASCICULE DE BREVET EUROPEEN

(11) **EP 1 091 928 B1**
(45) Date de publication et mention de la délivrance du brevet: **28.02.2007**
(21) Numéro de dépôt: 99957636.6
(22) Date de dépôt: 23.06.1999
(51) Int. Cl.: C07C 237/00, C07F 9/09, A61K 31/66, A61P 37/02

(54) **NOUVEAUX PSEUDODIPEPTIDES ACYLES, LEUR MODE DE PREPARATION ET LES COMPOSITIONS PHARMACEUTIQUES EN RENFERMANT**
NEUE ACYLIERTE PSEUDODIPEPTIDE, VERFAHREN ZU IHRER HERSTELLUNG, UND DIESE ENTHALTENDE PHARMAZEUTISCHE ZUSAMMENSETZUNGEN
NOVEL ACYL PSEUDODIPEPTIDES, PREPARATION METHOD AND PHARMACEUTICAL COMPOSITIONS CONTAINING SAME

(30) Priorité: 30.06.1998 WO PCT/FR98/01396
(43) Date de publication de la demande: 18.04.2001
(73) Titulaire: OM Pharma, 1217 Meyrin 2 (CH)
(72) Inventeur: BAUER, Jacques, CH-1162 Saint-Prex (CH); MARTIN, Olivier, Richard, F-45100 Orléans (FR)
(74) Mandataire: Burtin, Jean-François
(86) Numéro de dépôt international: PCT/IB1999/001170
(87) Numéro de publication internationale: WO 2000/000462

(56) Documents cités:
- EP-A- 0 224 260
- EP-A- 0 668 289
- WO-A-95/14026
- PATENT ABSTRACTS OF JAPAN vol. 011, no. 069 (C-407), 3 mars 1987 (1987-03-03) & JP 61 227586 A (DAI ICHI SEIYAKU CO LTD), 9 octobre 1986 (1986-10-09)
- CHEMICAL ABSTRACTS, vol. 127, no. 8, 25 août 1997 (1997-08-25) Columbus, Ohio, US; abstract no. 109122h, MIYAJIMA, K. ET AL.: "Lipid A and related compounds XXXIII." page 624; XP002095371 & CHEM. PHARM. BULL., vol. 45, no. 6, 1997, pages 1089-1093,
- CHEMICAL ABSTRACTS, vol. 126, no. 18, 5 mai 1997 (1997-05-05) Columbus, Ohio, US; abstract no. 238583n, MIYAJIMA, K. ET AL.: "Lipid A and related compounds XXXII." page 645; XP002095372 & CHEM. PHARM. BULL., vol. 45, no. 2, 1997, pages 312-320,
- CHEMICAL ABSTRACTS, vol. 123, no. 3, 17 juillet 1995 (1995-07-17) Columbus, Ohio, US; abstract no. 33547v, SUHARA, Y. ET AL.: "Lipid A and related compounds XXIX." page 902; XP002095373 & CHEM. PHARM. BULL., vol. 42, no. 12, 1994, pages 2526-2531,

## Description

La présente invention se rapporte au domaine de la chimie et plus particulièrement au domaine de la chimie thérapeutique.

Elle a plus particulièrement pour objet des pseudodipeptides dérivés d'acides aminés hydroxylés, dont les fonctions amine libres sont amidifiées par des acides gras.

Elle a spécifiquement pour objet des pseudodipeptides N-acylés dont au moins un groupe hydroxyle est estérifié par un groupement acide sous forme neutre ou chargée, répondant à la formule générale I. 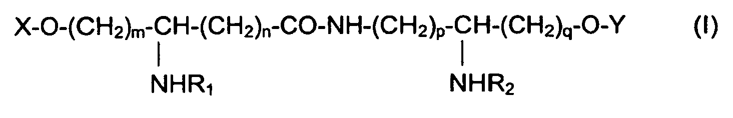 dans laquelle R₁ et R₂ représentent chacun un groupe acyle dérivé d'un acide carboxylique ayant de 2 à 24 atomes de carbone, saturé ou insaturé, linéaire ou ramifié, non-substitué ou portant un ou des substituants choisis parmi les hydroxyle, alkyle, alkoxy, acyloxy, amino, acylamino, acylthio et (alkyl en C₁ - C₂₄) thio le descripteur m- possède une valeur allant de 1 à 4
le descripteur n possède la valeur 0
le descripteur p possède la valeur 3 ou 4 et q est égal à 1
X et Y représentent chacun un hydrogène ou un groupe phosphorique sous forme neutre ou chargée,
avec la limitation que l'un au moins des substituants X et Y représente un groupe acide sous forme neutre ou chargée,

Lorsque les substituants X et/ou Y représentent un groupe acide sous forme neutre, il s'agit de la forme phosphorique libre. Lorsqu'il s'agit d'un groupe acide sous forme chargée il s'agit de la forme phosphorique salifiée, notamment par addition d'une base minérale ou organique, de préférence thérapeutiquement compatible. Lorsque les bases ne sont pas thérapeutiquement compatibles, elles peuvent servir de moyen d'identification, de purification ou de dédoublement.

Parmi les bases salifiantes thérapeutiquement compatibles on citera notamment les bases alcalines comme les hydroxydes de sodium, de potassium, de lithium, les sels d'ammonium ; les bases alcalinoterreuses comme les hydroxydes de calcium ou de strontium, les sels de magnésium, les sels de métaux ferreux et similaires, les bases organiques comme celles dérivées d'amines primaires, secondaires ou tertiaires comme la méthylamine, la diéthylamine, la monoéthanolamine, la diéthanolamine, la benzylamine, la N-méthylbenzylamine, la vératrylamine, la triméthoxybenzylamine, des aminoacides à réaction basique comme la lysine ou l'ornithine ou des sucres aminés.

Des bases non utilisables thérapeutiquement sont par exemple la brucine, la strychnine, l'agmatine, l'homarine, la glucosamine, la N-méthylglucosamine ou la N-méthylmorpholine. Comme indiqué ci-dessus les sels en découlant serviront comme moyen de séparation ou d'identification.

Lorsque m est égal à 1 et n est égal à 0, la molécule dérive de la sérine. Lorsque m est égal à 2 et n est égal à 0, la molécule dérive de l'homosérine. Lorsque m est égal à 3 et n est égal à 0, il s'agit d'un dérivé de la pentahomosérine. Lorsque m est égal à 4 et n ést égal à 0, il s'agit d'un dérivé de l'hexahomosérine.

Lorsque p est égal à 3 et q est égal à 1, il peut s'agir d'un dérivé de la citrulline ou de l'ornithine ou de l'arginine. Lorsque p est égal à 4 et q est égal à 1, il peut s'agir d'un dérivé de l'homoarginine ou de la lysine.

Parmi les pseudodipeptides objet de l'invention, on retiendra particulièrement comme composés actuellement préférés :
- les 1 et/ou 10-dihydrogénophosphate de 3-(3-dodécanoyloxytétradécanoylamino) 9-(3-hydroxytétradécanoylamino) 4-oxo-5-azadécane-1,10-diols et leurs sels d'addition avec une base minérale ou organique.
- le 1,10-bis(dihydrogénophosphate) de 3-(3-dodécanoyloxytétradécanoylamino) 9-(3-hydroxytétradécanoylamino) 4-oxo-5-azadécane-1,10-diol et ses sels d'addition avec une base minérale ou organique.
- le 1,10-bis(dihydrogénophosphate) de 3-(3-hydroxytétradécanoylamino) 9-(3-dodécanoyloxytétradécanoylamino) 4-oxo-5-azadécane 1,10-diol et ses sels d'addition avec une base minérale ou organique.
- le 1-dihydrogénophosphate de 3-(3-dodécanoyloxytétradécanoylamino) 9-(3-hydroxytétradécanoylamino) 4-oxo-5-azadécane-1,10-diol et ses sels d'addition avec une base minérale ou organique.
- le 1-dihydrogénophosphate de 3-(3-hydroxytétradécanoylamino) 9-(3-dodécanoyloxytétradécanoylamino) 4-oxo-5-azadecane 1,10-diol et ses sels d'addition avec une base minérale ou organique.
- le 10-dihydrogénophosphate de 3-(3-hydroxytétradécanoylamino) 9-(3-dodécanoyloxytétradécanoylamino) 4-oxo-5-azadecane 1,10-diol et ses sels d'addition avec une base minérale ou organique.

La définition de R₁ et R₂ englobe des dérivés acylés à chaîne de longueur variable, identiques ou différents, ramifiés ou en chaîne droite, saturés ou insaturés, pouvant porter un ou plusieurs substituants choisis dans le groupe formé par un alkyle, amino, acylamino, hydroxyle, alkoxyle, acyloxy, acylthio et alkylthio.

Des exemples de tels dérivés acylés substitués, sont le radical ricinoléyle, 12-hydroxystéaroyle, 2-hydroxy-3-méthylbutyroyle, 3-hydroxy-2-aminopentanoyle, palmitoléyle, élaidyle, éléostéaroyle, arachidoyle, arachidonyle, gadoléyle, béhényle, érucyle, 8-méthyldécanoyle, 9-méthyldécanoyle, docosahexaénoyle ou eicosapentaénoyle.

Parmi les groupements acyle concernés, l'acide 3-hydroxymyristique et l'acide 3-lauryloxymyristique sont ceux actuellement préférés.

Les composés de formule générale I et spécialement les composés mono- et diphosphorylés désignés sous le nom de code OM-294-MP (MP) et OM-294-DP (DP), respectivement, se distinguent par des propriétés pharmacologiques intéressantes, notamment immunomodulatrices. Ils trouvent un intérêt particulier dans la thérapeutique des maladies liées à une déficience des défenses immunitaires ou à une exagération des réponses immunitaires, selon les doses utilisées.

L'invention a donc pour objet des pseudodipeptides dérivés d'acides aminés hydroxylés, dont les fonctions amine libres sont amidifiées par des acides gras et qui présentent des propritétés biologiques, notamment immuno modulatrices améliorées par rapport à l'art antérieur.

La présente invention a précisément pour objet des pseudodipeptides conçus comme analogues de lipides A. Les lipides A sont des entités moléculaires de type glycolipide qui forment l'ancre lipidique du lipopolysaccharide (LPS) dans la membrane cellulaire des bactéries Gram(-). Celles-ci sont responsables de la très forte activité immunostimulante du LPS (Rietschel et al. *Curr. Top. Microbiol. Immunol.* **1996,** 216, 39 ; Zaehringer, U. et al. *Adv. Carbohydr. Chem. Biochem.* **1994,** 50, 211). Les lipides A et leurs analogues possèdent des activités biologiques intéressantes comme immunomodulateurs et anticancéreux (Shiba & Kotani, (Daiichi Seiyaku Co.), brevet JP 61 227 586 A ; Hasegawa et al. (Toho Pharmaceutical Industries) brevet EP 224 260 ; Kodama et al. (Suntory Ltd), brevet EP 688 289) mais sont généralement caractérisés par une forte endotoxicité. Le dérivé de lipides A connu sous le nom de code OM-174 (lipide A de *E. coli* des-O-acylé) est d'un intérêt particulier en raison de sa très faible toxicité et des activités immunomodulatrice, antitumorale et comme adjuvant importantes (Davies, Bauer, Hirt, Schulthess (Laboratoires OM S.A.) brevet WO 95 14026). La complexité des lipides A et leur toxicité ont stimulé la recherche d'analogues synthétiques simplifiés comme par exemple les composées hybrides *O*-glycosyl-amino acides *N-* et *O-*acylés décrits par Achiwa et al. (*Chem. Pharm. Bull.* **1994,** 42, 2526 ; **1997,** 45, 312 ; **1997**, 45, 1089). Les pseudodipeptides N-acylés de l'invention constituent une famille nouvelle d'analogues synthétiques de lipides A dépourvus de toxicité et présentant à un degré élevé des propriétés immunomodulatrices.

II paraît à cet égard important de signaler le rôle important que joue l'oxyde nitrique (NO) et les espèces oxygénées réactives telles que l'anion superoxyde dans la régulation des phénomènes immunologiques. Ces activités ont déjà été largement décrits dans la littérature (Bogdan, C. *Nature Immunol.* **2001**, 2, 907-916 et ref. citées).

L'oxyde nitrique induit de multiples effets modulateurs sur l'inflammation et sur les réponses immunitaires.
Dans les tissus animaux, NO est généré par des synthases (NOS) qui oxydent la L-arginine en L-citrulline. Il existe différentes isoformes de NOS, dont la NOS inductible (iNOS ou NOS II) présente dans les macrophages, les monocytes et autres types cellulaires participant à l'inflammation. L'inducteur de NOS est le LPS.
L'interaction des Lipopolysaccharides (LPS) avec leur récepteur TLR4 déclenche une cascade d'évènements qui mènent à l'activation de NFκB, qui est responsable de la transcription de l'enzyme iNOS. L'oxyde nitrique NO produit par les macrophages activés immunologiquement ou chimiquement, présente une activité antimicrobienne, également attribuée à la formation de peroxynitrite (ONOO).

En plus de son rôle effecteur direct, NO sert de puissant facteur immuno régulateur. Il est principalement décrit comme inhibiteur de l'expression de gènes impliqués dans la prolifération cellulaire et, en particulier, du sous-type Th1 de cellules T d'aide (T Helper cells). NO inhiberait aussi la sécrétion de cytokines par ces lymphocytes T dans les processus inflammatoires.

Une revue générale a été publiée par JW. Coleman (*International Immunopharmacology* **2001,** 2, 1397-1406). Elle fait référence à des publications bien antérieures au dépôt de la présente demande de brevet et notamment :
- Wink (*Curr. Top. Cell. Regul.* **1996**, *34*, 159-87).
- Taylor-Robinson (*Eur. J. Immunol.* **1994,** *24,* 980-4).
- Bauer H (*Immunology* **1997,** *90,* 205-11).
- Fehsel K (J. Immunol. **1995,** *155,* 2858-65).

Du fait, en particulier, de leur activité stimulatrice de la production de NO, les composés de la présente demande doivent être considérés comme des agents capables de moduler la réponse immunitaire de l'organisme et c'est dans ce domaine que les composés de l'invention trouvent une utilisation comme médicament.

L'invention concerne un procédé d'obtention des phosphopseudodipepdides de formule générale I. 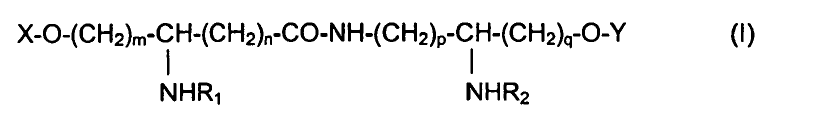 dans laquelle R₁ et R₂ représentent chacun un groupe acyle dérivé d'un acide carboxylique ayant de 2 à 24 atomes de carbone, saturé ou insaturé, linéaire ou ramifié, non-substitué ou portant un ou plusieurs substituants hydroxyle, alkyle, alkoxy, acyloxy, amino, acylamino, acylthio et (alkyl en C₁ - C₂₄) thio
le descripteur m prend une valeur allant de 1 à 4
le descripteur n prend la valeur 0
le descripteur p possède une valeur égale à 3 ou 4 et q est égal à 1
dans laquelle X et Y représentent chacun un hydrogène ou un groupe dihydroxyphosphoryle
qui consiste en ce qu'on bloque les fonctions amine en position (q+1) et en ω d'un acide diaminé de formule H₂N(CH₂)ₚCHNH₂(CH₂)_{q-1}COOH par des réactifs de blocage labiles par acidolyse et/ou hydrogénolyse, respectivement, soumet la fonction carboxylique restée libre à l'action d'un agent réducteur pour former l'alcool correspondant, libère la fonction amine en (q+1) que l'on acyle à l'aide d'un dérivé fonctionnel d'un acide carboxylique de formule R₂OH dans laquelle R₂ est défini comme précédemment, puis libère la fonction amine terminale par hydrogénolyse pour obtenir l'amino alcool de formule générale II 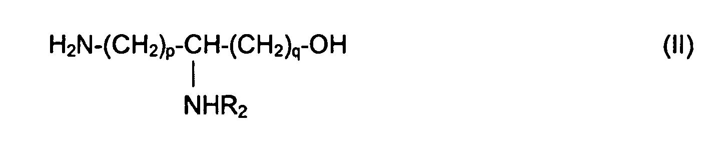 dans laquelle R₂ représente un groupe acyle dérivé d'acide carboxylique ayant de 2 à 24 atomes de carbone, saturé ou non-saturé, non substitué ou portant un ou plusieurs substituants définis comme ci-dessus,
p possède une valeur de 3 ou 4 et q est égal à 1
que l'on condense en présence d'un agent de condensation peptidique dans un solvant inerte avec un dérivé fonctionnel d'ω-hydroxy amino acide de formule générale III : 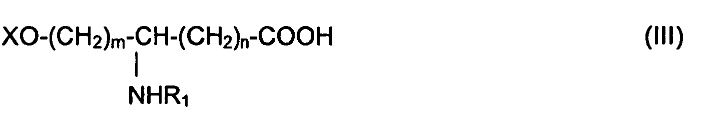 dans laquelle R₁ est un groupe acyle dérivé d'un acide carboxylique ayant de 2 à 24 atomes de carbone, saturé ou non-saturé, non substitué ou portant un ou plusieurs substituants
m est un nombre entier variant de 1 à 4
n est un nombre égal à 0
et X est un radical diaryloxyphosphoryle de formule 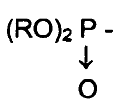 dans laquelle R est un radical aryle labile par hygrogénolise
pour former le pseudodipeptide de formule générale IV 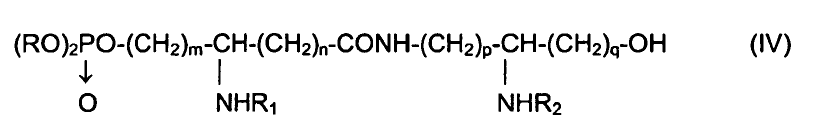 dans laquelle les substituants R₁, R₂ et les descripteurs m, n, p et q sont définis comme précédemment, et R est un radical labile par hydrogénolyse, défini comme ci-dessus, dont on peut -si désiré- phosphoryler l'autre fonction alcool par un agent de phosphorylation en présence d'un agent de couplage, si nécessaire, et soumettre à une hydrogénation catalytique d'une part pour débloquer la fonction alcool éventuellement présente sur le groupe acyle R₂ et d'autre part libérer la fonction phosphate puis débloquer par hydrogénolyse la deuxième fonction phosphate éventuellement présente, de façon à obtenir le dérivé de formule générale V 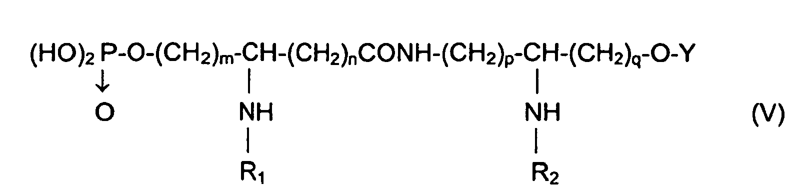 dans laquelle Y représente soit un hydrogène soit un groupe phosphono
et si désiré, on effectue l'étape supplémentaire de salification à l'aide d'une base minérale ou organique.

La stéréochimie des centres porteurs de groupes acylamino est déterminée par la configuration des acides aminés de départ et celle des groupes acylamino par la configuration des acides gras de départ. On peut partir d'un diamino acide de configuration L ou D ou racémique. On peut partir d'un acide aminé hydroxylé de configuration L, D ou racémique. Tous ces stéréoisomères ou diastéréoisomères font partie de l'invention.

Le procédé selon l'invention peut encore être défini par les modalités d'exécution suivantes, actuellement préférées figurées aux Schémas de synthèse 1 et 2:
1. Le blocage de la fonction amine en ω sur la chaîne d'un dérivé d'ornithine est effectué par N-benzyloxycarbonylation après réaction initiale de la fonction acide avec un sel de cuivre, en milieu alcalin, réaction de ce carboxylate de cuivre avec du chloroformiate de benzyle et libération de la fonction carboxylique par chélation du cuivre en milieu acide, pour obtenir le dérivé N-benzyloxycarbonylé, selon une méthode décrite dans « Organic Preparations and Procedures International 23 (1992) 191-194 ».
2. Le blocage de la fonction amine en α du carboxyle du dérivé de l'ornithine est effectué par terbutyloxycarbonylation au moyen d'un pyrocarbonate d'alkyle comme le pyrocarbonate de terbutyle en milieu basique.
   Le pyrocarbonate de terbutyle réagit avec la fonction amine proximale pour former le dérivé ω-benzyloxycarbonylamino α-terbutyloxycarbonylamino carboxylique.
3. La conversion de la fonction carboxylique en fonction alcool primaire est réalisée en appliquant la méthode décrite dans Tetrahedron Letters 32 (1991) 923-926 qui consiste en ce que l'on fait réagir le dérivé carboxylique avec un chloroformiate d'alkyle, comme le chloroformiate d'isobutyle, pour former un anhydride mixte que l'on réduit au moyen d'un borohydrure de métal alcalin ou alcalino-terreux, pour conduire au dérivé hydroxylé correspondant, porteur d'une fonction alcool primaire.
4. L'élimination du groupe terbutyloxycarbonyle en α est effectuée par action de l'acide trifluoroacétique qui conduit en même temps à la formation du trifluoroacétate de la fonction amine.
5. L'acylation de la fonction amine en α de la fonction alcool est effectuée au départ du sel trifluoroacétique au moyen d'un anhydride mixte préparé à partir de l'acide R₂OH et d'un chloroformiate d'alkyle.
6. La libération de la fonction amine terminale est réalisée par hydrogénolyse en présence d'un catalyseur à base de métal noble comme le palladium sur charbon ou l'iridium.
7. Le couplage peptidique entre le composé aminé de formule II et le dérivé phosphorylé de formule III est effectué en présence d'un agent de couplage tel que la 1-isobutyloxy 2-isobutyloxycarbonyl-1,2-dihydroquinoléine dans un solvant inerte tel qu'un solvant halogéné, ou en utilisant un carbodiimide.
   On obtient ainsi un pseudodipeptide de formule générale (V) dont la fonction hydroxyle éventuellement portée par le groupe acyle R₂, est bloquée.
8. La libération de la fonction hydroxyle du groupe acyle R₂ intervient par hydrogénolyse en présence d'un métal noble comme le palladium sur charbon.
9. La libération du groupe phosphorique X est effectuée par hydrogénation catalytique en présence d'oxyde de métal noble comme l'oxyde de platine.
10. La phosphorylation du dérivé pseudodipeptique IV est effectuée en deux étapes (Helv. Chim. Acta 70 (1987), 175). Dans une première étape le composé IV est soumis à l'action d'un N, N-dialkyl phosphoramidite de dialkyle ou de diaryle, en présence d'un agent de couplage tel que le [1H]-tétrazole dans un solvant polaire tel que le tétrahydrofurane ; le phosphite ainsi formé est ensuite oxydé en phosphate à l'aide d'un acide peroxycarboxylique aromatique comme par exemple l'acide peroxyphthalique, l'acide m-chloroperbenzoïque ou l'acide nitroperbenzoïque. La libération du groupe phosphorique Y est réalisée par hydrogénation catalytique en présence d'un métal noble comme le palladium sur charbon.
11. La phosphorylation du dérivé de l'homosérine est effectuée à l'aide d'un halogénure de diphénylphosphoryle en présence de pyridine et d'une N, N-dialkylaminopyridine (Helv. Chim. Acta 58 (1975), 518), après bloquage de la fonction amine par terbutoxycarbonylation au moyen du pyrocarbonate de terbutyle en milieu basique et bloquage de la fonction carboxyle après formation d'un sel de césium, et benzylation à l'aide d'un halogénure de benzyle dans le diméthylformamide ou le diméthylacétamide.
12. L'acylation de l'azote du dérivé d'homosérine s'effectue après déprotection de la fonction amine par l'acide trifluoroacétique pour obtenir le sel trifluoroacétique de l'amine, et réaction avec un anhydride mixte résultant de la réaction entre l'acide carboxylique R₁OH et un chloroformiate d'alkyle en présence d'une amine réactive telle que la N-méthylmorpholine.

L'invention concerne encore les produits intermédiaires de formule générale II, et de formule générale III, sous forme énantiomériquement pure ou sous forme racémique.

L'invention concerne encore les compositions pharmaceutiques renfermant à titre de principe actif au moins un composé de formule générale I, comme définie précédemment, sous forme neutre ou chargée, en association ou en mélange avec un excipient ou un véhicule inerte, non toxique, pharmaceutiquement acceptable.

L'invention concerne plus particulièrement les compositions pharmaceutiques renfermant à titre de principe actif au moins un sel d'un composé de formule générale I, avec une base minérale ou organique thérapeutiquement compatible.

L'invention concerne encore les compositions pharmaceutiques à base d'un composé de formule générale I, sous forme énantiomériquement pure ou sous forme racémique, en association ou en mélange avec un excipient ou un véhicule pharmaceutique.

Parmi les formes pharmaceutiques envisagées on pourra citer celles qui conviennent pour la voie digestive, parentérale, par inhalation, par voie topique, transdermique ou permuqueuse, comme par exemple les comprimés, les dragées, les gélules, les solutés ou suspensions injectables, les aérosols, les gels, les emplâtres ou les solutés pénétrants.

Les compositions pharmaceutiques selon l'invention trouvent une utilisation comme vecteur de molécule d'intérêt thérapeutique par leurs propriétés d'association non-covalente de nature hydrophile et hydrophobe. Leur caractère amphiphile favorise les formulations et le transport des molécules d'intérêt thérapeutique vers les récepteurs membranaires, ainsi que vers les parois et le cytoplasme cellulaires. Ils peuvent être utilisés seuls ou en association avec une molécule d'intérêt thérapeutique par voie orale, parentérale, rectale, topique, percutanée, ou permuqueuse. Ils peuvent être utilisés seuls ou en association avec une molécule d'intérêt thérapeutique par incubation extratemporanément *ex-vivo* avec des cellules sanguines afin de rendre les cellules immunocompétentes, avant de les réinoculer *in-vivo* par voie parentérale.
Les molécules MP et DP montrent des propriétés semblables, en tant qu'adjuvants du système immunitaire utilisés par exemple pour la vaccination, en association avec les antigènes appropriés, contre des maladies d'origine virale, parasitaire, microbienne ou fongique. Par contre, les composés selon l'invention montrent des propriétés fondamentalement différentes dans leur capacité à induire la production de cytokines ou la maturation des cellules souches immunocompétentes provenant des organes hématopoiétiques et lymphoides.
Le composé MP favorise la maturation et la différenciation des monocytes en cellules dendritiques fonctionnelles, en présence ou en absence de l'antigène approprié et contribue ainsi à renforcer l'immunité humorale et cellulaire. Le composé DP montre de son côté des propriétés anti-tumorales.
Les compositions selon l'invention sont particulièrement intéressantes du fait de leur faible toxicité. Elles sont utilisées en thérapeutique humaine ou animale à des doses qui varient de 1 à 100 mg de principe actif par prise unitaire et de 1 à 300 mg de principe actif par jour.

Les exemples suivants illustrent l'invention sans toutefois la limiter. Ils sont présentés dans les Schémas de synthèse 1 à 6.

### EXEMPLE I

### Acide 4-(diphényloxyphosphoryloxy)-2-[(R)3-dodécanoyloxytétradécanoylamino] butanoïque

### 1. N_{α} - Terbutyloxycarbonyl-DL-homosérine

2 g d'homosérine (16.78 mmol) ont été dissouts dans 20ml d'eau et on additionne cette solution de 16.78ml de NaOH 1M et de 3.006g de carbonate de césium (9.23 mmol). Après 5 minutes d'agitation, la solution est refroidie dans un bain d'eau et de glace. On ajoute alors 60 ml de dioxane et du pyrocarbonate de terbutyle. Le mélange réactionnel est maintenu sous agitation dans un bain d'eau glacée pendant une heure puis à température ambiante pendant 5 heures. Le solvant a été ensuite éliminé sous vide. Le résidu sec a été employé directement pour l'étape suivante.

### 2. N_{α}-Terbutyloxycarbonyl-DL-homosérinate de benzyle

Au résidu du stade 1, on ajoute 20 ml de diméthylformamide et on effectue l'évaporation à siccité puis on additionne le milieu réactionnel de 60 ml de diméthylformamide et de 4.5 ml de bromure de benzyle (20.13 mmol). Il se forme alors un précipité blanc. Le mélange est maintenu sous agitation pendant 16 heures. Le solvant a été ensuite chassé sous vide. Le résidu a été épuisé avec 2 fois 20 ml d'acétate d'éthyle. La phase organique a été lavée avec de l'eau (20 ml) puis avec une solution saline (20 ml) respectivement, puis séchée sur sulfate de magnésium anhydre. Le solvant est évaporé et le résidu est utilisé tel quel pour l'étape suivante.

### 3. N_{α}-Terbutyloxycarbonyl-O-(diphényloxyphosphoryl)-DL-homosérinate de benzyle

Le résidu de l'étape précédente a été séché sous vide poussé puis dissout dans le chlorure de méthylène (60 ml). On ajoute alors 4.11 g de 4-diméthylaminopyridine (33.56 mmol) dans la solution, le mélange réactionnel a été agité pendant 10 minutes, et on ajoute alors 12 ml de pyridine et 6.95 ml de chlorophosphate de diphényle (33.56 mmol). La solution a été agitée à température ambiante pendant 18 heures puis lavée avec de l'acide chlorhydrique N (5 x 20 ml), de l'eau (30 ml) et par une solution saline (30 ml). La phase organique a été séchée sur sulfate de magnésium anhydre, le solvant a été chassé sous vide. Le résidu est purifié par chromatographie flash (hexane/acétate d'éthyle = 4 : 1). La fraction principale a été concentrée et le résidu cristallisé. On obtient ainsi 7.49 g de produit phosphorylé soit un rendement de 82.4 %. Point de fusion : 63.5 - 64.0° C.

### 4. O-(Diphényloxyphosphoryl)-DL-homosérinate de benzyle

Le produit phosphorylé de l'étape précédente (7.88 g soit 15.4 mmol) a été dissout dans 15 ml d'acide trifluoroacétique et la solution a été maintenue sous agitation à température ordinaire pendant 2.5 heures. Le solvant a été alors chassé sous vide poussé, le résidu sec a été purifié par chromatographie flash (MeOH / CH₂Cl₂ = 10 : 1). La fraction principale a été concentrée et le résidu a été cristallisé à température ordinaire. On obtient ainsi 7.17 g de produit phosphorylé (rendement 88.9 %). Il est utilisé sans autre purification pour l'étape suivante.

### 5. 2-[(R)-3-Dodécanoyloxytétradécanoylamino]-4-(diphényloxyphosphoryloxy)-butanoate de benzyle

4.284 g (10.07 mmol) d'acide (R) 3-dodécanoyloxytétradécanoïque préparé selon la méthode décrite dans Bull. Chem. Soc. Jpn 60 (1987), 2205-2214, ont été dissouts dans 30ml de tétrahydrofurane et la solution a été mise à refroidir jusqu'à -15°C dans un bain de saumure glacée. On a alors ajouté 1.108 ml (10.07 mmol) de N-méthylmorpholine et 1.31 ml (10.07 mmol) de chloroformiate d'isobutyle. On a poursuivi l'agitation pendant 30 minutes. On a alors ajouté au mélange réactionnel 5.724 g (10.07 mmol) de O-(diphényloxyphosphoryl)-DL-homosérinate de benzyle dans un mélange de 30ml de tétrahydrofurane et de 5 ml de triéthylamine. Après agitation pendant une nuit à température ambiante, le solvant a été chassé sous vide et on a ajouté 20 ml d'eau au résidu. Le mélange a été ensuite épuisé avec de l'acétate d'éthyle (2 x 30 ml). Les phases organiques ont été combinées, lavées successivement avec de l'eau (20 ml) et avec de la saumure (20 ml) et séchées sur sulfate de magnésium. Le solvant a été évaporé et le résidu a été purifié par chromatographie flash (hexane - acétate d'éthyle 2:1, Rf = 0.29); rendement 7.455g soit 87,1 %. PF = 31,0° - 32,1°C. ¹H-RMN (CDCl₃, 250 MHz), δ en ppm : 7.4-7.1 (m, 15H), 6.90 (2d, 1H, ³*J* = 7.6 Hz, NH), 5.3-5.1 (m, 3H), 4.7 (m, 1 H), 4.35 (m, 2H), 2.45 (m, 2H), 2.4-2.1 (m, 4H), 1.6 (m, 4H), 1.4-1.1 (m, 34H), 0.9 (t, 6H). ¹³C-RMN (CDCl₃, 63 MHz), δ en ppm : 173.01, 171.08, 169.66, 150.18 (d, ²*J*_{P,C}=7.1 Hz), 135.01, 129.60, 128.33, 128.14, 127.96, 125.21, 119.80 (d, ³*J*_{P,C}=5.0 Hz), 70.69, 67.05, 65.19 (d, ²*J*_{P,C}=5.6 Hz), 49.13, 40.97, 40.77 (2 diast.), 34.20, 33.98, 33.82, 31.70, 29.42, 29.34, 29.14, 28.94, 25.01, 24.77, 22.47, 13.91.

### 6. Acide 4-(diphényloxyphosphoryloxy)-2-[(R)-3-dodécanoyloxytétradécanoylamino] butanoïque

On prépare une solution de l'ester benzylique obtenu à l'étape 5 (2.23 g soit 2.6 mmol) dans 300 ml de méthanol HPLC dans un ballon à trois tubulures et on y ajoute 1.0 g de charbon palladié à 10 % de palladium. On purge le contenu du ballon pour chasser l'air, sous vide, puis le ballon a été chargé avec de l'hydrogène sous pression atmosphérique.
Le mélange réactionnel a été agité à température ambiante pendant 1 heure, le catalyseur a été ensuite éliminé rapidement par filtration sur membrane et le filtrat a été concentré pour fournir un sirop incolore. Celui-ci est homogène en chromatographie en couche mince et en RMN, et a été utilisé directement sans purification supplémentaire pour l'étape de couplage; Rf = 0.75 (dichlorométhane - méthanol - triéthylamine, 10:1:0.5). ¹H-RMN (CDCl₃, 250 MHz), δ en ppm : 7.4-7.1 (m, 10H), 6.85 (d, 1H, N*H*), 5.15 (m, 1H), 4.6 (m, 1 H), 4.35 (m, 2H), 2.45 (m, 2H), 2.4-2.15 (m, 4H), 1.6 (m, 4H), 1.4-1.1 (m, 34H), 0.9 (t, 6H). ¹³C-RMN (CDCl₃, 63 MHz), δ en ppm: 173.35, 173.30 (2 diast.), 172.75, 170.37, 150.0 (d, ²*J*_{P,C}=7.5 Hz), 129.55, 125.28, 119.71 (d, ³*J*_{P,C}=4.4 Hz), 70.78, 65.65 (d, ²*J*_{P,C}=5.9 Hz), 49.00, 40.77, 40.63 (2 diast.), 34.13, 33.86, 33.76, 31.59, 29.31, 29.25, 29.03, 28.82, 24.88, 24.68, 22.36, 13.76.

L' acide 4-(diphényloxyphosphoryloxy)-2-[(R)-3-benzyloxytétradécanoylamino] butanoïque peut être obtenu par la même séquence de réactions en remplaçant dans l'étape 5 de l'exemple I, l'acide (R)-3-dodécanoyloxytétradécanoïque par l'acide (R)-3-benzyloxytétradécanoique.

### EXEMPLE II

### (2R)-5-amino-2-[(R)-3-benzyloxytétradécanoylamino]-pentan-1-ol

### 1. Sel de cuivre de la D-ornithine

A une solution de D-ornithine (5.25 g soit 30 mmol) dans 30 ml d'hydroxyde de sodium M, on a ajouté 50 ml d'une solution de sulfate cuivrique pentahydraté (3.814 g soit 15.3 mmol) dans de l'eau. L'agitation a été poursuivie pendant 2 heures. On évapore alors le solvant à siccité. On ajoute 60ml de méthanol pour former un solide de couleur pourpre que l'on sépare, lave au dioxane et au méthanol respectivement.

### 2. (2R)-2-Amino-5-(benzyloxycarbonylamino) pentanoate de cuivre

Le solide pourpre a été dissout dans 40 ml de soude M et 70 ml de dioxane, la solution a été refroidie dans un bain d'eau glacée et on y ajoute 5.14 ml (soit 36 mmol) de chloroformiate de benzyle. L'agitation est poursuvie dans un bain d'eau glacée pendant 3 heures et ensuite à température ordinaire pendant 15 heures. Le précipité pourpre est rassemblé puis lavé à l'éthanol à 95 % (40 ml), à l'eau (50 ml) et à l'éthanol (60 ml) respectivement. Le précipité a été séché à l'étuve (T< 45° C, sous vide); le rendement en deux étapes est de 8.27 g, soit 93 % par rapport à la théorie.

### 3. Acide (2R)-5-(benzyloxycarbonylamino)-2-(terbutyloxycarbonylamino)pentanoïgue

Le sel de cuivre obtenu au stade 2 a été dissout dans de l'acide chlorhydrique 2M (400 ml) et on y a ajouté de l'EDTA (8.15 g, 27.8 mmol). On agite le mélange pendant 2.5 heures, puis on le neutralise à pH7 en ajoutant de la soude 5M (environ 160 ml). Il se forme un précipité blanc. Le mélange a été agité pendant 2.5 heures dans un bain d'eau glacée. Le précipité a été filtré, lavé à l'eau froide jusqu'à ce que l'effluant soit incolore, puis séché à l'étuve en dessous de 60°. Ce solide a été dissout dans 156ml de NaOH M et la solution refroidie au bain d'eau glacée. On a ajouté à cette solution 7.7g (35.2 mmol) de pyrocarbonate de terbutyle dans le dioxane (160 ml). Le mélange réactionnel a été agité à 0° C pendant 45 minutes puis pendant 16 heures à température ambiante. Le solvant organique a été évaporé et on ajoute au résidu 70ml d'acétate d'éthyle. On acidifie ensuite la phase aqueuse en ajoutant de l'acide chlorhydrique 2N jusqu'à pH ~3. La couche aqueuse a été épuisée encore une fois avec 100 ml d'acétate d'éthyle. Les phases organiques ont été combinées et lavées à l'eau (30 ml) et avec une solution saline (30 ml). Le solvant a été éliminé sous vide de façon à fournir une huile incolore après purification par chromatographie flash (Rdt : 8.42g en deux étapes soit 76.7 % de la théorie) (Rf = 0.19, dichlorométhane - MeOH 20 : 1).

### 4. (2R)-5-(Benzyloxycarbonylamino)-2-(terbutyloxycarbonylamino)pentan-1-ol

A une solution froide (-15° C) du dérivé de l'acide diamino pentanoïque obtenu au stade 3 (5.45 g soit 14.8 mmol) dans 60ml de THF, on a ajouté 1.654 ml (soit 14.8 mmol) de N-méthylmorpholine et 9,6ml (soit 14.8 mmol) de chloroformiate d'isobutyle (IBCF). La solution a été agitée à -15° C pendant 1 minute puis on y a ajouté une solution de borohydrure de sodium (5.104 g soit 44.6 mmol) dans 10 ml d'eau. L'agitation a été maintenue à -15° C pendant encore 10 minutes puis on a ajouté 400 ml d'eau pour arrêter la réaction. La solution a été épuisée avec de l'acétate d'éthyle (100 ml x 2). Les phases organiques ont été combinées et lavées avec 50 ml d'eau et avec 60 ml de solution saline puis séchées sur sulfate de magnésium anhydre. Le solvant a été chassé et le résidu a été cristallisé du mélange acétate d'éthyle/hexane (4.94 g, rendement 94.9 %) PF = 47.5 - 48° C.

### 5. Déblocage du dérivé du 2,5-diaminopentan-1-ol

6.32 g (18 mmol) de (2R)-5-(benzyloxycarbonylamino)-2-(terbutyloxycarbonylamino)-pentan-1-ol obtenu au stade 4 ont été dissouts dans 25 ml d'acide trifluoroacétique puis agités pendant 2.5 heures à température ambiante. Le solvant a été ensuite évaporé et le résidu a été purifié par chromatographie flash (MeOH / CH₂Cl₂ = 10:1). On obtient ainsi une masse vitreuse incolore qui fond à température ambiante. Le rendement est de 5.45 g de sel trifluoroacétique (rendement = 82.7 %). Le chlorhydrate fond à 133.0 - 134.3° C (recristallisation du méthanol).

### 6. (2R)-5-(Benzyloxycarbonylamino)-2-[(R)-3-benzyloxytétradecanoylamino]pentan-1-ol

On a ajouté à une solution refroidie à -15° C de 5.27 g (15.8 mmol) d'acide (R)-3-benzyloxytétradécanoïque (Bull. Chem. Soc. Jpn, 60 (1987), 2197-2204) dans 30 ml de tétrahydrofurane, 1.89 ml (15.8 mmol) de N-méthylmorpholine et 2.21 ml d'IBCF (15.8 mmol). Le mélange réactionnel a été maintenu sous agitation à -15° C pendant 30 minutes. On ajoute alors 5.25 g de sel trifluoroacétique de l'exemple précédent (14.4 mmol) dans 30 ml de tétrahydrofurane et 1.44 ml de triéthylamine à la solution. L'agitation a été poursuivie à température ambiante pendant 16 heures puis on a ajouté 30ml d'eau et 60ml d'acétate d'éthyle; la phase organique a été séparée et la phase aqueuse a été épuisée une fois encore avec de l'acétate d'éthyle (60 ml). Les phases organiques ont été combinées et lavées à l'eau (30 ml) et avec une solution saline (30 ml) puis séchées sur sulfate de magnésium anhydre. Le solvant a été 5 évaporé et le résidu a été recristallisé d'un mélange acétate d'éthyle/hexane (5.824 g, soit un rendement de 71,2 %), PF = 117,5°- 118° C. Rf = 0.32, acétate d'éthyle - éther de pétrole 3:1. ¹H-RMN (CDCl₃, 250 MHz), δ en ppm : 7.4-7.2 (m, 10H), 6.5 (d, 1H, N*H*), 5.1 (s, 2H), 4.9 (m, 1 H, N*H*), 4.5 (2d, AB, 2H), 3.8 (m, 2H), 3.5 (m, 2H), 3.1 (m, 2H), 2.4 (m, 2H), 1.6-1.4 (m, 6H), 1.4-1.2 (m, 18H), 0.9 (t, 3H). ¹³C-RMN (CDCl₃, 63 MHz), δ en ppm : 172.24, 156.49, 138.06, 136.53, 128.46, 128.04, 127.87, 76.76, 71.39, 66.60, 65.44, 51.54, 41.43, 40.65, 33.76, 31.87, 29.61, 29.30, 28.01, 26.47, 25.05, 22.65, 14.09.

### 7. (2R)-5-Amino-2-[(R)-3-benzyloxytétradecanoylamino]pentan-1-ol

Dans un ballon à trois tubulures, 150 mg de palladium sur charbon à 20 % ont été ajoutés à la solution de (2R)-5-(benzyloxycarbonylamino)-2-[(R)-3-benzyloxytétradécanoylamino] pentan-1-ol (3.0 g, soit 5.27 mmol) et 6ml de triéthylamine dans 300 ml d'éthanol HPLC. On a chassé l'air par mise sous vide puis le ballon a été chargé d'hydrogène. Le mélange réactionnel a été agité à température ambiante pendant 2 heures puis le catalyseur a été séparé par filtration sur membrane et le filtrat a été concentré pour fournir un solide blanc homogène en CCM, utilisé tel quel pour le stade suivant sans autre purification. Rf = 0.2, dichlorométhane - méthanol - triéthylamine 5:1:0.5, PF = 47 - 48°C.
¹H-RMN (CDCl₃, 250 MHz), δ en ppm: 7.4-7.2 (m, 5H), 6.75 (d, 1H, N*H*), 4.5 (2d, AB, 2H), 3.9 (m, 2H), 3.5 (m, 2H), 2.3 - 2.6 (m, 7H), 1.7-1.2 (m, 24H), 0.9 (t, 3H). ¹³C-RMN (CDCl₃, 63 MHz), δ en ppm: 171.86, 138.13, 128.37, 127.87, 127.75, 76.81, 71.50, 64.57, 51.38, 41.51, 41.17, 33.89, 31.82, 29,26, 28.57, 28.03, 25.07, 22.60, 14.04.

Le (2R)-5-amino-2-[(R)-3-dodécanoytoxytétradecanoylamino]pentan-1-ol peut être obtenu par la même séquence de réactions en remplaçant dans l'étape 6 de l'exemple II, l'acide (R)-3-benzyloxytétradécanoique par l'acide (R)-3-dodécanoyloxytétradécanoïque.

### EXEMPLE III

### 3-[(R)-3-Dodécanoyloxytétradécanoylamino]-4-oxo-5-aza-9-[(R)-3-hydroxytétradécanoylaminol-décane-1,10-diol 1-dihydrogénophosphate

### 1. Couplage Peptidique

On disperse dans une solution d'acide (2RS)-4-(diphényloxyphosphoryloxy)-2-[(R)-3-dodécanoyloxytétradécanoytamino]butanoïque (1.0 mmol) obtenu à l'exemple I, dissous dans 20ml de chlorure de méthylène, 363.6 mg (1.2 mmol) d'IIDQ (1-isobutyloxy-2-isobutyfoxycarbonyl-1,2-dihydroquinoléine). On ajoute après 15 minutes d'agitation 1.0 mmol de (2R)-5-amino-2-[(R)-benzyloxytétradécanoylamino]pentan-1-ol obtenu à l'exemple II, dissout dans 10 ml de chlorure de méthylène et le mélange réactionnel a été maintenu sous agitation pendant 4h.
La solution a été concentrée et le résidu purifié par chromatographie flash (CH₂Cl₂ / acétone = 5:2, Rf 0.23). Le solvant a été chassé et on obtient ainsi un sirop incolore (0.620 g soit un rendement de 52.7 %) de pseudodipeptide phosphorylé. Rf = 0.49, dichlorométhane - méthanol - triéthylamine, 10:1:0.5. ¹H-RMN (CDCl₃, 250 MHz), δ en ppm: 7.40-7.15 (m, 15H), 7.00 (m, 1H), 6.90 et 6.80 (2d, 2 diast, 1H), 6.65 (d, 1H) (3 x NH), 5.15 (m, 1H), 4.50 (m, 3H), 4.30 (m, 2H), 3.85 (m, 2H), 3.45 (m, 2H), 3.15 (m, 2H), 2.41-2.14 (m, 8H), 1.6-1.4 (m, 8H), 1.4-1.1 (m, 54H), 0.9 (t, 9H, 3*CH₃*). ¹³C-RMN (CDCl₃, 63 MHz), δ en ppm : 173.11, 171.68, 170.52 (2 diast.), 169.94 (2 diast.), 150.0 (d, ²*J*_{P,C}=7.2 Hz), 138.20 (2diast.), 129.58, 127.99, 127.49, 127.26, 125.24, 119.73 (t, ³*J*_{P,C}: 5.0 Hz), 76.48, 71.12, 70.71, 65.86 (élargi), 64.22, 50.96, 49.71 (élargi), 41.46, 41.05, 39.07, 34.13, 34.00, 32.70, 31.61, 29.34, 29.06, 28.87, 27.98, 25.25 24.92, 24.72, 22.38, 13.80.

### 2. 1-(Diphényloxyphosphoryloxy)-3-[(R)-3-dodécanoyloxytétradecanoylamino]-4-oxo-5-aza-9-[(R)-3-hydroxytétradécanoylamino]décan-10-ol.

La solution de pseudodipeptide phosphorylé (488 mg soit 0.42 mmol) obtenu ci-dessus et d'acide acétique (1.9 ml) dans 65 ml d'éthanol HPLC a été placée dans un ballon à trois tubulures et on y ajoute 200 mg de palladium sur charbon à 10 % de Pd. On purge l'air par mise sous vide puis le ballon a été chargé avec de l'hydrogène. Le mélange réactionnel a été agité à température ambiante pendant 2h, puis le catalyseur a été séparé par filtration sur membrane, le solvant a été chassé sous vide, ce qui fournit le produit brut avec un rendement de 92%. Un échantillon de produit est purifié par chromatographie flash (CH₂Cl₂ / acétone 5:4, Rf = 0.24). On obtient ainsi un solide vitreux. Rf = 0.68, chlorure de méthylène - méthanol 5:2. ¹³C-RMN (CDCl₃, 63 MHz), δ en ppm (quelques signaux dédoublés par la présence de diastéréoisomères) : 173.60, 173.15, 170.67, 170.60, 170.27, 170.07, 150.24 (d), 129.92, 125.66, 120.05 et 119.90 (2d), 71.11, 71.05, 68.83, 66.21 (élargi), 64.71, 51-38, 50.32, 50.12, 43.25, 43.12, 41.66, 41.57, 39.30, 37.26, 34.45, 32.84, 31.86, 29.62, 29.5, 29.29, 29.13, 28.08, 25.57, 25.19, 24.97, 22.62, 14.03.

### 3. 3-[(R)-3-Dodecanoyloxytétradecanoylamino]-4-oxo-5-aza-9-[(R)-3-hydroxytétradécanoylamino]-décan-1,10-diol 1-dihydrogénophosphate

Dans un ballon à trois tubulures, on préactive de l'oxyde de platine (137 mg) dans de l'éthanol absolu (5 ml) avec de l'hydrogène pendant 10 min. On ajoute ensuite la solution de 1-(diphényloxyphosphoryloxy)-3-[(R)-3-dodécanoyloxytétradécanoylamino]-4-oxo-5-aza-9-[(R)-3-hydroxytétradecanoylamino) décan-10-ol (411 mg soit 0,38 mmol) dans de l'éthanol absolu (20 ml). On chasse l'air par mise sous vide poussé, puis le ballon a été chargé avec de l'hydrogène. Le mélange réactionnel a été agité à température ambiante pendant 2 à 3 h, le catalyseur a été séparé par filtration sur membrane, le solvant chassé sous vide. On obtient finalement le produit brut sous la forme d'un solide blanc (rendement brut de 98%). Rf = 0.50, chloroforme - méthanol - eau, 6:4:0.6.

Le 3-[(R)-3-hydroxytétradecanoylamino]-4-oxo-5-aza-9-[(R)-3-dodécanoyloxytétradécanoylamino]-décan-1,10-diol 1-dihydrogénophosphate peut être obtenu à partir de l'acide 4-(diphényloxyphosphoryloxy)-2-[(R)-3-benzyloxytétradécanoylamino] butanoïque et du (2R)-5-amino-2-[(R)-3-dodécanoyloxytétradecanoylamino]pentan-1-ol par la même séquence de réactions (Schéma 3) (Fig 35).

Alternativement, le 3-[(R)-3-dodécanoyloxytétradécanoylamino]-4-oxo-5-aza-9-[(R)-3-hydroxytétradécanoylamino]-décan-1,10-diol 1-dihydrogénophosphate est obtenu à partir de l'acide aspartique par la séquence de réactions suivantes (Schémas de synthèse 1, 5 et 6) (Fig 33, 37 et 38): protection de la fonction OH libre du (2R)-5-(benzyloxycarbonylamino)-2-[(R)-3-benzyloxytétradecanoylamino]pentan-1-ol par un groupement benzyloxyméthyle, libération de la fonction 5-amino de ce composé par hydrogénolyse, couplage peptidique de cette amine avec un dérivé monoestérifié de l'acide D ou L-aspartique portant sur sa fonction amine soit un groupe protecteur soit un groupe (R)-3-dodecanoyloxytétradecanoyle, libération et réduction de la fonction carboxylique terminale par l'intermédiaire d'un anhydride mixte, déprotection si nécessaire de la fonction amine issue de l'acide aspartique puis N-acylation avec un dérivé de l'acide (R)-3-dodécanoyloxytétradecanoique, phosphorylation de la fonction hydroxyle en C-1, et finalement déblocage des fonctions phosphate et hydroxyles par hydrogénolyse.

### EXEMPLE IV

### Préparation du 3-[(R)-3-dodécanoyl-oxytétradécanoylamino]-4-oxo-5-aza-9-[(R)-3-hydroxytétradécanoylamino]décane-1,10-diol, 1,10-bis(dihydrogénophosphate).

Le 1-(diphényloxyphosphoryloxy)-3-[(R)-3-dodécanoyloxytétradécanoylamino]-4-oxo-5-aza-9-[(R)-3-benzyloxytétradécanoylamino]décan-10-ol (985 mg soit 0.84 mmol) est traité avec du N,N-diéthylphosphoramidite de dibenzyle (0.58 ml, pureté 85%), en présence de [1 H]-tétrazole (182 mg) dans le tétrahydrofurane (35 ml) pendant 30 min à température ambiante. Le phosphite intermédiaire est oxydé par addition d' une solution d'acide m-chloroperoxybenzoïque (535 mg) dans 25 ml de chlorure de méthylène entre 0° et -20°C. Après 20 min, on ajoute une solution de Na₂S₂O₃ (20 ml) pour détruire l'excès d'oxydant, puis on dilue la phase organique avec de l'éther. La phase organique est séparée, lavée successivement avec une solution aqueuse de Na₂S₂O₃ (5 x 20 ml), puis une solution de NaHCO₃ (2 x 20 ml), puis avec de l'acide chlorhydrique aqueux (20 ml), séchée sur MgSO₄ et concentrée. Le produit brut est purifié par chromatographie flash sur gel de silice (CH₂Cl₂ - acétone 10:3). Le dérivé diphosphorylé protégé ainsi obtenu (900 mg, rendement 75%)(Rf 0.64, dichlorométhane - acétone 5:2) est soumis à une hydrogénation catalytique dans le méthanol HPLC (100 ml) en présence de palladium sur charbon à 10% de palladium (300 mg) sous pression atmosphérique, pendant 4h à température ambiante. Le catalyseur est éliminé par filtration sur membrane et le filtrat concentré sous pression réduite, ce qui fournit le 10-(dihydroxyphosphoryloxy)-1-(diphenyloxyphosphoryloxy)-3-[(R)-3-dodécanoyloxytétradecanoylamino]-4-oxo-5-aza-9-[(R)-3-hydroxytétradécanoylamino]décane brut (Rf = 0.63, chloroforme-méthanol-eau 6:4:0.6) avec un rendement de 89%. Ce produit est ensuite soumis à une hydrogénation catalytique sur oxyde de platine (380 mg) dans l'éthanol HPLC (130 ml) pendant 24h à température ambiante, sous pression atmosphérique. Le catalyseur est éliminé par filtration sur membrane et le filtrat concentré pour fournir composé bis dihydrogénophosphate libre (Rf = 0.20, chloroforme - MeOH - eau, 6:4:0.6).

Le 3-[(R)-3-hydroxytétradecanoylamino]-4-oxo-5-aza-9-[(R)-3-dodécanoyloxytétradécanoylamino]-décan-1,10-diol 1,10-bis(dihydrogénophosphate) peut être obtenu à partir de l'acide 4-(diphényloxyphosphoryloxy)-2-[(R)-benzyloxytétradécanoylamino] butanoïque et du (2R)-5-amino-2-[(R)-3-dodécanoyloxytétradecanoylamino]pentan-1-ol par la même séquence de réactions (Schéma 3) (Fig 35).

Alternativement, le 3-[(R)-3-dodécanoyloxytétradécanoylamino]-4-oxo-5-aza-9-[(R)-3-hydroxytétradécanoylamino]-décan-1,10-diol 1,10-bis(dihydrogénophosphate) est obtenu à partir de l'acide aspartique par la séquence de réactions suivantes (Schémas de synthèse 1, 4 et 6): libération de la fonction 5-amino du (2R)-5-(benzyloxycarbonylamino)-2-[(R)-3-benzyloxytétradecanoylamino]pentan-1-ol par hydrogénolyse, couplage peptidique de cette amine avec un dérivé monoestérifié de l'acide D ou L-aspartique portant sur sa fonction amine soit un groupe protecteur soit un groupe (R)-3-dodecanoyloxytétradecanoyle, libération et réduction de la fonction carboxylique terminale par l'intermédiaire d'un anhydride mixte, déprotection si nécessaire de la fonction amine issue de l'acide aspartique puis N-acylation avec un dérivé de l'acide (R)-3-dodécanoyloxytétradecanoique, phosphorylation des fonctions hydroxyles en C-1 et en C-10, et finalement déblocage des fonctions phosphates et hydroxyle par hydrogénolyse.

### EXEMPLE V - PURIFICATION ET ANALYSE DES COMPOSES SELON L'INVENTION

### 1. Purification des composés monophosphorylé et diphosphorylé

Les produits de synthèse monophosphorylé et diphosphorylé sont solubilisés dans un mélange eau - isopropanol (1:1 v/v) avec 0.1% de triéthylamine pour obtenir un pH entre 8 et 9. La quantité nécessaire de bicarbonate d'ammonium 2M est ensuite ajoutée pour atteindre une concentration de 25 mM.

La purification est effectuée par HPLC préparative en phase inverse dans les conditions suivantes :

| | |
|---|---|
| Colonne: | Bondapack C18 PrepPak, 40 x 200 mm, 15-20 µm, 300 Å. Waters |
| Phase mobile: | A: isopropanol - eau (1:1, v/v), 50mM bicarbonate d'ammonium |
| | B: isopropanol - eau (2:8, v/v), 50mM bicarbonate d'ammonium |
| Débit: | 40 ml/min |
| Elution: | Adsorption isocratique sur la colonne : 40% B (60% A), 10 minutes |
| | Gradient A:B : 40 à 80% B en 10 minutes |
| | Elution isocratique 80% B, 30 minutes |
| | Lavage : 100% B, 10 minutes |
| Détection: | UV, 210 nm |

Dans ces conditions d'élution, le temps de rétention du composé monophosphorylé est compris entre 25 et 30 min et celui du composé diphosphorylé entre 18 et 25 minutes. Au cas où la présence de produits monophénylés serait observée (déprotection incomplète lors de la déphénylation finale), une purification plus fine doit être effectuée. Cette purification supplémentaire est réalisée dans les conditions suivantes :

| | | | |
|---|---|---|---|
| Colonne: | Kromasil C18, 21 x 250 mm, 5 µm, 100 Å, Macherey-Nagel | | |
| Phase mobile: | A: isopropanol - eau (1:1, v/v), 50mM bicarbonate d'ammonium | | |
| | B: isopropanol - eau (2:8, v/v), 50mM bicarbonate d'ammonium | | |
| Débit: | 10 ml/min | | |
| Elution: | Adsorption isocratique sur la colonne: 40% B (60% A), 10 minutes | | |
| | Elution isocratique : | | |
| | | composé monophosphorylé : | 80% B, 30 minutes |
| | | composé diphosphorylé : | 74% B, 30 minutes |
| | Lavage : 100% B, 10 minutes | | |
| Détection: | UV, 210 et 254 nm | | |

Les fractions contenant les composés monophosphorylé ou diphosphorylé sous forme de sel d'ammonium sont rassemblées et concentrées par adsorption sur phase C18 Bondapack, 15-20 µm, 300 Å, Waters. Le sel de sodium des composés monophosphorylé ou diphosphorylé est obtenu par lavage au moyen d'une solution à 10 g/L NaCl dans l'eau - isopropanol (9:1, v/v). Après élimination de l'excédent de NaCl par passage de 5 volumes d'un mélange eau - isopropanol (9:1, v/v) sur la colonne, le composé est élué avec de l'isopropanol pur. Ce solvant est ensuite évaporé à sec au Rotavapor. Une solubilisation finale est réalisée dans le volume nécessaire d'eau (additionnée de 0.1 % de triéthanolamine dans le cas du composé monophosphorylé) pour atteindre une concentration cible de 2 mg/ml. Une filtration stérilisante est ensuite effectuée sur filtre 0.2 µm, Express Membrane, Millipore (si volume inférieur à 50 ml: système Steriflip, si volume supérieur à 50 ml: système Steritop).
Dans le cas du composé monophosphorylé, une sonication de la solution (3 fois 10 secondes) à température ambiante est effectuée avant la filtration stérilisante.

### 2. Suivi et rendement de la purification

Après chaque étape, les fractions sont analysées par chromatographie analytique HPLC en phase inverse selon les conditions suivantes :

| | |
|---|---|
| Colonne: | Supelcosil C18, 3 µm, 4.6 x 150mm, 100 Å, Supelco |
| Phase mobile: | A: eau - acétonitrile (1:1, v/v), 5mM TBAP |
| | B: eau - isopropanol (1:9, v/v), 5mM TBAP |
| | TBAP : phosphate de tétrabutylammonium |
| Débit: | 1 ml/min |
| Elution: | Gradient A:B (75:25 à 0:100) en 37.5 minutes |
| Détection: | UV, 210 et 254 nm |

Dans ces conditions chromatographiques, les temps de rétention observés pour les composés mono- et diphosphorylés sont de 25.5 ±0.5 et 20.8 ±0.5 minutes, respectivement. Les rendements de purification obtenus varient entre 57 et 94% pour le composé monophosphorylé et entre 71 et 92% pour le composé diphosphorylé. 311 mg et 189 mg de composés mono et diphosphorylé, respectivement, ont été produits.

### 3. Dosage et analyse de pureté des produits finaux

Le dosage et le contrôle de pureté des produits obtenus sont réalisés par HPLC/UV dans les conditions chromatographiques décrites ci-dessus. Selon ces analyses les puretés obtenues pour les différents lots de composés mono- et diphosphorylés produits varient entre 99 et 100%. Afin de mettre en évidence la présence d'impuretés inactives en UV, des analyses LC/ES-MS ont été réalisées (ionisation de type electrospray, mode positif). Pour ces dernières, le phosphate de tétrabutylammonium (5 mM) a été remplacé par l'acétate d'ammonium (25 mM) pour satisfaire aux conditions d'ionisation de l'interface electrospray.

Des méthodes alternatives de dosage des solutions finales ont été utilisées. On citera pour exemple l'analyse quantitative des phosphates totaux (adapté de Ames, B.N., Methods in Enzymology VII (1966), 115-117), des acides aminés (adapté de Hughes et al., J. Chromatogr. 389 (1987), 327-333) et des chaînes acyles (adapté de Miller, L.T., Hewlett-Packard Application Note (1984), 228-237).

### 4. Analyses spectroscopiques

### 4.1. Spectrométrie de masse

Les spectres ES-MS (modes positif et négatif) des composés mono- et diphosphosphorylés ont été mesurés sur trois types de spectromètres de masse (Finnigan LCQ, ion trap; Micromass Quattro II, triple stage quadrupole; Hewlett-Packard MSD, single quadrupole). Des analyses complémentaires de type MS/MS ont également été réalisées. Les spectres attestant de l'identité et de la pureté des produits sont joints en annexe.

### Spectres ES-MS (mode positif)

### Composé diphosphorylé :

### (Micromass Quattro II : Spectre 1; HP-MSD : Spectre 3) (Fig 39 et 41)

A basse énergie, un ion pseudomoléculaire majoritaire à *m*/*z* 1014.6 [M+H]⁺ est observé. Des adducts de sodium à *m*/*z* 1036.6 [M+Na]⁺, 1058.6 [M-H+2Na]⁺ et 1080.5 [M-2H+3Na]⁺ sont également visibles.

Selon le degré de fragmentation choisi, deux fragments à *m*/*z* 916.5 [M-98+H]⁺ et 818.6 [M-98+H]⁺ sont observés, attestant de la présence de deux groupes phosphoryles sur la molécule. Comme le montre le spectre 3 (Fig 41), l'intensité relative des ions observés varie considérablement en fonction de l'énergie appliquée.

### Composé monophosphorylé :

### (Micromass Quattro Il : Spectre 2) (Fig 40)

Un schéma d'ionisation quelque peu différent est obtenu pour le composé monophosphorylé du fait de la présence de triéthanolamine (TeoA) dans les solution analysées. On observe un ion pseudomoléculaire majoritaire à *m*/*z* 934.4 [M+H]⁺ ainsi que des adducts de sodium [M+Na]⁺ et de potassium [M+K]⁺ à *m*/*z* 956.3 et 972.3, respectivement. Un second groupe d'adducts à *m*/*z* 1083.4 [M+TeoA+H]⁺, *m*/*z* 1105.3 [M+TeoA+Na]⁺ et *m*/*z* 1121.3 [M+TeoA+K]⁺ est également visible. La présence d'un groupe phosphoryle sur la molécule est confirmée par un fragment observé à haute énergie à *m*/*z* 836.4 [M-98+H]⁺.

### Spectres ES-MS (mode négatif)

Les ions observés sur les spectres ES-MS des composés mono- et diphosphorylés en mode négatif sont tout à fait en accord avec les résultats obtenus en mode positif.

Des analyses en ionisation FAB (mode positif) ont également été réalisées. A basse résolution, les composés mono- et diphosphosphorylés présentent des adducts de sodium [M+Na⁺] à *m*/*z* 956.5 et 1036.5, respectivement.

A haute résolution (matrice d'alcool 3-nitro benzylique), un pic à *m*/*z* 956.667 est observé pour le monophosphate, ce qui correspond à la formule brute C₄₉H₉₆O₁₁N₃PNa attendue (masse calculée : 956.668 uma).

Pour le diphosphate, un pic à *m*/*z* 1036.635 est mesuré, ce qui correspond à la formule brute C₄₉H₉₇O₁₄N₃P₂Na attendue (masse calculée : 1036.634 uma).

Toutes les analyses MS ont mis en évidence le haut degré de pureté des produits obtenus.

### 4.2. Résonance magnétique nucléaire

Les spectres ¹H-RMN et ¹³C-RMN des composés mono- et diphosphosphorylés ont été mesurés sur des appareils de type Bruker DPX à 250.13 et 62.89 MHz, respectivement, et Varian Unity Inova 500 à 499.87 et 125.7 MHz, respectivement. Les spectres ³¹P-RMN ont été enregistrés à 121.6 MHz (Bruker DPX). Les spectres attestant de l'identité et de la pureté des produits sont joints en annexe.

Spectres ¹H-RMN (Spectres 4 et 5) (Fig 42 et 43)
- composé monophosphorylé : sur le spectre enregistré dans CDCl₃ + 0.1% triéthanolamine (TEoA) (Spectre 4), on observe les signaux correspondant aux trois protons portés par les atomes d'azote N(5), N(2a) et N(2b) entre 7 et 9.5 ppm (voir aggrandissement de la fenêtre spectrale). Les signaux attribués à H-N(2a) et H-N(2b) apparaissent sous la forme de deux doublets qui mettent en évidence la présence d'un mélange de stéréoisomères. On observe également une prédominance de l'un des diastéréoisomères (conséquence des différentes étapes de purification).
- composé diphosphorylé : sur le spectre enregistré dans CDCl₃-CD₃OD (3:1, v/v) (Spectre 5), les signaux correspondant à H-N(5), H-N(2a) et H-N(2b) ne sont plus observés suite à un échange en présence de CD₃OD.
   Des informations supplémentaires pour l'attribution des différents signaux ont été obtenues par des expériences de corrélations homo- et hétéronucléaires (¹H-¹H - RMN: COSY, ¹H-¹³C-RMN: HSQC et HMBC).
   Spectres ¹³C-RMN (Spectres 6 et 7) (Fig 44 et 45)
   L'enregistrement de spectres ¹³C-RMN est rendu particulièrement difficile par la solubilité relativement faible des composés mono- et diphosphorylés.
   Spectres ³¹P-RMN (Spectres 8 et 9) (Fig 46 et 47)
   Pour les deux composés mono- et diphosphorylés, un pic unique est observé.

### EXEMPLE V - ETUDES PHARMACOLOGIQUES DES COMPOSES SELON L'INVENTION

### 1. Détermination de l'endotoxicité par le test chromogénique au Limulus.

L'endotoxicité est déterminée par le test *Limulus Amoebocyte Lysate* chromogénique (LAL-Chromogénique de Charles River Endosafe , lot # EK4121E, Charleston, USA). Ce test est basé sur l'activation par le lipopolysaccharide (LPS) ou les produits de structure comparable, d'une cascade enzymatique présente dans le LAL. Cette activation enzymatique est mise en évidence par le clivage d'un chromogène lié à un peptide par la protéase, en bout de chaîne de la cascade enzymatique selon la réaction suivante :

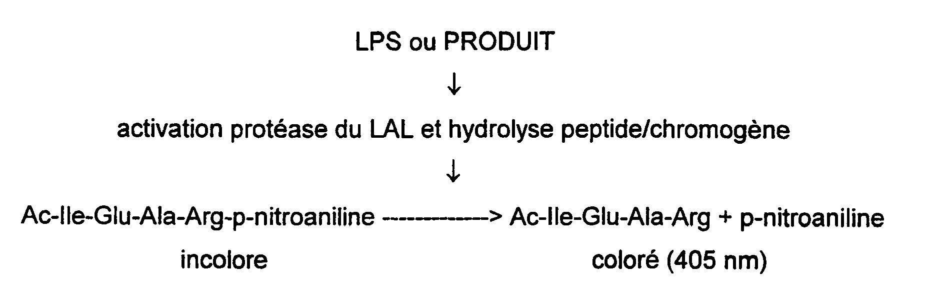

La réaction enzymatique est effectuée à 37° C et la formation du chromogène au cours du temps est mesurée à 405 nm. Dans cette méthode cinétique en point final, le temps nécessaire pour atteindre 0.2 unités de DO est enregistré et l'activité endotoxique calculée par rapport à un étalon de LPS (courbe standard).

Les résultats sont exprimés en EU (Endotoxin Unit) par rapport à une préparation standardisée de lipopolysaccharide de *E. coli.* Pour cette série de dosage 1 EU correspond à 0.08 mg d'équivalent LPS.
Les résultats présentent une variabilité relativement importante mais normale pour ce genre de dosage quantitatif qui donne surtout un ordre de grandeur. Le test LAL est surtout utilisé pour démontrer l'absence de pyrogène (limite supérieure de la concentration en endotoxine) dans des préparations pharmaceutiques. Le dosage quantitatif du contenu en pyrogène doit impérativement être comparé dans une même série d'expériences bien standardisées.

### Résultats

Les résultats (moyennes ± écart-type) obtenus pour les produits de l'invention sont présentés dans le Tab (a):

**Tab (a) Activation du limulus amebocyte lysat (LAL)**

| Produits | Activité LAL en EU/mg | Activité LAL en équivalent LPS ng éq. LPS/mg |
|---|---|---|
| OM-294-DP | 56 ± 48 | 6.2 |
| OM-294-MP | 13 ±2 | 1.4 |
| LPS de *E. coli* (référence) | 7.7 ± 1.6 x 10⁶ | 0.85 x 10⁶ |

Les composés selon l'invention sont 10⁶ fois moins actifs que le LPS dans le test LAL. L'OM-294-DP et l'OM-294-MP sont donc des produits particulièrement intéressants du fait de leur faible toxicité, associée à leur capacité à induire des activités biologiques immunomodulatrices (*in vivo* et *in vitro*).

### 2. Détermination de la prolifération des cellules souches de la moelle osseuse de souris stimulées par du LPS ou par les composés de l'invention

### Protocole

2 souris mâles C57/BL6 de 6 semaines sont sacrifiées par inhalation de CO₂ suivie d'une dislocation des cervicales. Les souris sont lavées à l'alcool, et la peau des membres postérieurs est retirée complètement. Les hanches, les fémurs et les tibias sont détachés au niveau des articulations. Les chairs sont enlevées grossièrement avec un scalpel. Les os sont nettoyés et les extrémités des os sont coupées au ciseau. La moelle est extraite de la lumière osseuse par injection en 3 fois de 1ml de milieu Eagle modifié par Dulbecco (milieu DH) par les extrémités qui ont été coupées au ciseau. Les cellules sont remises en suspension dans le milieu DH et centrifugées pendant 5 minutes à 300 x g. Le surnageant est éliminé et les cellules souches sont remises en suspension dans du milieu DH complété avec 20 % de sérum foetal (FCS). La concentration cellulaire est ajustée à 500'000 cellules par ml.

Les produits en solution dans le milieu DH supplémenté avec le FCS, les acides aminés et les antibiotiques sont dilués en série, directement dans la microplaque à 96 puits. 9 dilutions sont effectuées avec un facteur de 3.16. Les produits sont testés en sextuplicate et chaque microplaque comprend un témoin négatif composé de milieu seul. Le volume final dans chaque puits est de 100 µl. Les microplaques sont incubées pendant 1 heure à 37° C sous 8 % CO₂ dans une étuve saturée en humidité pour tamponner le milieu. Après 1 heure, 100 µl de la suspension cellulaire sont ajoutés aux produits et l'incubation est poursuivie pendant 7 jours.
La prolifération est déterminés par la mesure de l'oxydation d'un substrat chromogénique (XTT) dans les mitochondries des cellules vivantes.
Après 7 jours les microplaques sont centrifugées 5 minutes à 400 x g, et 100 µl de surnageant sont prélevés et éliminés. 50 µl d'une solution à 1 mg/ml de XTT 3-[ 1-phénylamino-carbonyl)-3,4-tétrazolium]-bis[(4-méthoxy-6-nitro)benzène sulfonate] de sodium et 0,008 mg/ml PMS ((N-méthyl dibenzopyrazine, méthyl sulfate) dans du milieu RPMI sont ajoutés à chaque puits. Après 8 heures d'incubation à 37° C sous 8 % CO₂ dans une étuve saturée en humidité, les microplaques sont lues au spectrophotomètre à 490 nm contre une référence à 690 nm.

Les résultats sont exprimés en moyenne (± écart type) sous forme d'une courbe dose/réponse. Les valeurs du contrôle négatif composé de milieu DH (moyenne ± écart type de toutes les expériences) sont également indiquées sous forme graphique.

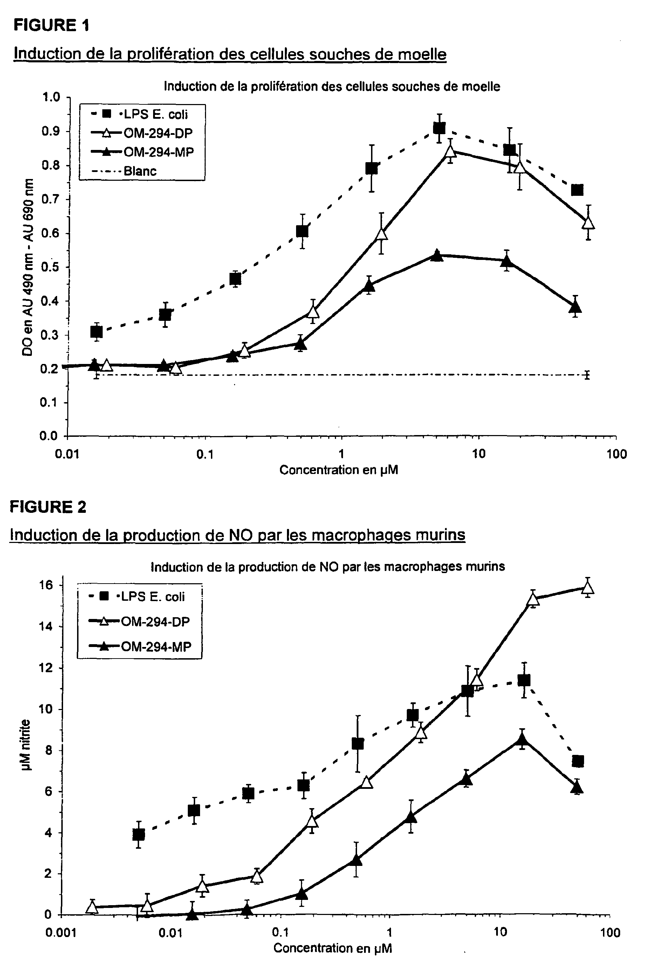

Dans cette expérience les composés selon l'invention induisent une prolifération significative des cellules-souches de moelle de souris. Celle-ci est presqueaussi importante que celle induite par le LPS de *E. coli,* mais la concentration minimale nécessaire pour induire une réponse significative est supérieure. Le produit monophosphorylé induit une réponse plus faible que celle du produit diphosphorylé. La figure 1 représente une expérience représentative tirée d'un ensemble de 3 expériences indépendantes obtenues à partie de préparations cellulaires différentes.

### 3- Détermination de la production d'oxyde nitrique dans les surnageants de macrophages

### Protocole

Deux souris C57/BL6 mâles de 6 semaines sont sacrifiées par inhalation de CO₂ suivie d'une dislocation des cervicales. Les souris sont lavées à l'alcool, et la peau des membres postérieurs est retirée complètement. Les hanches, les fémurs et les tibias sont détachés au niveau des articulations. Les chairs sont enlevées grossièrement avec un scalpel. Les os sont nettoyés et les extrémités des os sont coupées aux ciseaux. La moelle est extraite par injection en 3 fois de 1ml de milieu Eagle modifié par Dulbecco (milieu DH) dans la lumière osseuse. Les cellules sont remises en suspension dans le milieu DH et centrifugées pendant 5 minutes à 300 x g. Le surnageant est éliminé et les cellules sont remises en suspension à la concentration de 40'000 cellules/ml dans du milieu DH complété avec 20 % de sérum de cheval (HS) et 30 % de surnageant de L929. Les L929 sont une lignée de fibroblastes murins dont le surnageant est riche en facteur de croissance pour les macrophages (M-CSF). La suspension cellulaire est distribuée par 12 ml dans des boîtes de Pétri qui sont incubées pendant 8 jours à 37° C sous 8 % CO₂ dans une étuve saturée en humidité. Après 8 jours, les cellules-souches se sont différenciées en macrophages matures. Les macrophages sont détachés par une incubation de 45 minutes à 4° C dans du PBS froid. Après centrifugation et élimination, les cellules sont remises en suspension dans du milieu DH complété avec 5 % de sérum foetal (FCS), de la glutamine, de l'asparagine, de l'arginine, de l'acide folique, du mercaptoéthanol et des antibiotiques (pénicilline et streptomycine). Les cellules souches sont rassemblées et la concentration cellulaire est ajustée à 700'000 cellules par ml.
Les produits mis en solution dans le milieu DH supplémenté avec FCS, les acides aminés et les antibiotiques sont dilués en série directement dans la microplaque à 96 puits. 9 à 10 dilutions suivant les produits sont effectués avec un facteur de 3.16. Les produits sont testés en triplicate et chaque microplaque comprend un témoin négatif composé de milieu seul. Le volume final dans chaque puits est de 100 µl. Les microplaques sont incubées pendant 1 heure à 37° C sous 8 % CO₂ dans une étuve saturée en humidité pour tamponner le milieu. Après 1 heure, 100 µl de la suspension cellulaire sont ajoutés aux produits et l'incubation est poursuivie pendant 22 heures.

Après 22 heures les microplaques sont centrifugées, 5 minutes à 400 x g et 100 µl de surnageant sont prélevés et transférés dans une microplaque. 100 µl de réactif de Griess [5 mg/ml de sulfanilamide + 0.5 mg/ml de chlorhydrate de N-(1-naphthyléthylène diamine)] dans de l'acide phosphorique à 2.5 % aqueux, sont ajoutés à chaque puits. Les microplaques sont lues au spectrophotomètre à 562 nm contre une référence à 690 nm. La concentration en nitrite est proportionnelle à celle de l'oxyde nitrique. La concentration en nitritre est déterminée par rapport à une courbe standard, linéaire de 1 à 25 µM de nitrite.

Les résultats sont exprimés après soustraction du témoin négatif, en moyenne ± écart type sous forme d'une courbe dose/réponse.

Dans cette expérience les composés selon l'invention induisent une production d'oxyde nitrique par les macrophages murins avec une courbe effet-dose. Le produit diphosphorylé induit une prolifération plus importante que celle induite par le LPS de *E. coli,* mais la concentration minimale nécessaire pour induire une réponse significative est supérieure. Le produit monophosphorylé induit une réponse plus faible que celle du produit diphosphorylé et que celle du LPS de *E. coli.* La figure 2 représente une expérience représentative tirée d'un ensemble de 3 expériences indépendantes obtenues à partir de préparations cellulaires différentes.

### 4. Détermination de la capacité des composés selon l'invention à activer la production de TNF-α-de macrophages alvéolaires humains.

### Protocole

Obtention des macrophages alvéolaires : Les macrophages alvéolaires humains ont été obtenus par lavage bronchoalvéolaire (BAL) de poumons de patients atteints de cancer du poumon. Le BAL est effectué immédiatement après chirurgie sur du tissu pulmonaire provenant des parties saines du lobe pulmonaire. Les lavages sont effectués avec NaCl 0.9 % à l'aide d'une seringue de 50ml. Les cellules obtenues sont constituées à >85 % de macrophages, les autres cellules étant principalement des lymphocytes. Après centrifugation, les cellules sont remises en suspension avec du milieu RPMI et les globules rouges sont éliminés par centrifugation sur Ficoll Paque (Research Grade). Les macrophages sont lavés 3 fois avec HBSS et implantés dans des microplaques à 24 puits, à raison de 1 ml par puits contenant un total de 1'000'000 de cellules. Après une heure d'incubation à 37° C, les macrophages sont adhérents et les puits sont lavés à 3 reprises avec 1 ml de HBSS afin d'éliminer les cellules non-adhérentes. Après les lavages, 1 ml de RPMI est ajouté dans chacun des puits contenant les macrophages.

Incubation avec les produits et dosage du TNF-α : les macrophages alvéolaires sont incubés à 37° C et 5 % de CO₂ avec des concentrations de 0.1 µg/ml, 1 µg/ml et 10 µg/ml des produits suivants :
- témoin négatif : RPMI
- témoin positif : LPS de *E.coli* (serotype O5:B5, Difco, Detroit, U.S.A.)
- composé monophosphorylé selon l'invention (OM-294-MP)
- composé diphosphorylé selon l'invention (OM-294-DP)

Les surnageants des cultures sont récoltés après 24 heures et analysés pour leur teneur en TNF-α (Kit BioSource Cytoscreen, Camarillo, CA, U.S.A.) avec une limite de détection à 1 pg/ml.

### Résultats

Les dérivés monophosphorylé et diphosphorylé selon l'invention induisent une production modérée de TNF-α à partir de 10 µg/ml. Le dérivé monophosphorylé selon l'invention induit une production de TNF-α supérieure à celle du dérivé diphosphorylé. Le contrôle positif LPS induit aux 3 concentrations testées une production élevée de TNF-α.
Les résultats sont présentés dans le Tab (a).

**Tableau (a) Induction par l'OM-294-MP et l'OM-294-DP de la production de TNF-α par des macrophages alvéolaires humains.**

| | TNF-α [pg/ml] | | | |
|---|---|---|---|---|
| | moyenne ± écart type de 3 expériences indépendantes | | | |
| Produit | 0 µg/ml | 0,1 µg/ml | 1 µg/ml | 10 µg/ml |
| Témoin négatif : RPMI | 195 ± 70 | | | |
| Témoin positif : LPS de *E; coli* | | 7667 ±1155 | 9858 ±2148 | 10390±3415 |
| OM-294-MP-1 | | 246 ± 38 | 353 ± 75 | 1049 ± 295 |
| OM-294-MP-2 | | 205 ± 62 | 291 ± 70 | 1124 ± 406 |
| OM-294-DP-1 | | 156 ± 66 | 117 ± 85 | 329 ± 141 |
| OM-294-DP-2 | | 171 ± 79 | 88 ± 61 | |

### 5. Détermination de la capacité des composés selon l'invention à inhiber la production de TNF-α de macrophages alvéolaires humains, induite par le lipopolvsaccharide de E. coli (LPS)

### Protocole

Obtention des macrophages alvéolaires : Les macrophages alvéolaires humains ont été obtenus par lavage bronchoalvéolaire (BAL) de poumons de patients atteints de cancer du poumon. Le BAL est effectué immédiatement après chirurgie sur du tissu pulmonaire provenant des parties saines du lobe pulmonaire. Les lavages sont effectués avec NaCl 0.,9 % à l'aide d'une seringue de 50 ml. Les cellules obtenues sont constituées à >85 % de macrophages, les autres cellules étant principalement des lymphocytes. Après centrifugation, les cellules sont remises en suspension avec du milieu RPMI et les globules rouges sont éliminés par centrifugation sur Ficoll Paque (Research Grade). Les macrophages sont lavés 3 fois avec HBSS et implantés dans des microplaques à 24 puits, à raison de 1 ml par puits contenant un total de 1'000'000 de cellules. Après une heure d'incubation à 37° C, les macrophages sont adhérents et les puits sont lavés à 3 reprises avec 1 ml de HBSS afin d'éliminer les cellules non-adhérentes. Après les lavages, 1 ml de RPMI est ajouté dans chacun des puits contenant les macrophages.

Incubation avec les produits et dosage du TNF-α- : les macrophages alvéolaires sont incubés à 37° C et 5 % de CO₂ avec du LPS d'*E. coli* (serotype O5: B5, Difco, Detroit, U.S.A.) à 1 µg/ml additionné simultanément des produits suivants aux concentrations de 10 µg/ml :
- témoin négatif : RPMI
- composé monophosphorylé selon l'invention (OM-294-MP)
- composé diphosphorylé selon l'invention (OM-294-DP)

Les surnageants des cultures sont récoltés après 24 heures et analysés pour leur teneur en TNF-α (Kit BioSource Cytoscreen, Camarillo, CA, U.S.A.) avec une limite de détection à 1 pg/ml.

### Résultats

Le dérivé diphosphorylé inhibe de manière importante la production de TNF-α normalement induite par le LPS. Le dérivé monophosphorylé inhibe partiellement la production de TNF-α induite par le LPS.
Les résultats sont présentés dans les Tab (a).

**Tableau (a) Inhibition par l'OM-294-MP et OM-294-DP de la production de TNF-α induite sur des macrophages alvéolaires humains par le LPS.**

| Produit | TNF-α [pg/ml] | % d'inhibition |
|---|---|---|
| RPMI (témoin négatif) | 73 | --- |
| LPS de *E. coli* seul (1 µg/ml) (témoin positif) | 8170 | 0 |
| OM-294-MP-1 (10 µg/ml) + LPS de *E. coli* (1 µg/ml) | 4577 | 44 |
| OM-294-MP-2 (10 µg/ml) + LPS de *E. coli* (1 µg/ml) | 4789 | 41 |
| OM-294-DP-1 (10 µg/ml) + LPS de *E. coli* (1 µg/ml) | 1267 | 84 |
| OM-294-DP-2 (10 µg/ml) + LPS de *E. coli* (1 µg/ml) | 1280 | 84 |

### 6. Effet des produits OM-294-MP et OM-294-DP sur la maturation des cellules dendritiques humaines.

La capacité des produits OM-294-MP et OM-294-DP à induire la maturation des cellules pré-dendritiques en cellules dendritiques a été évaluée. Les paramètres suivants sont mesurés: incorporation du Dextran-FITC et expression des molécules de surface CD40, CD80, CD83, CD86.

### Protocole

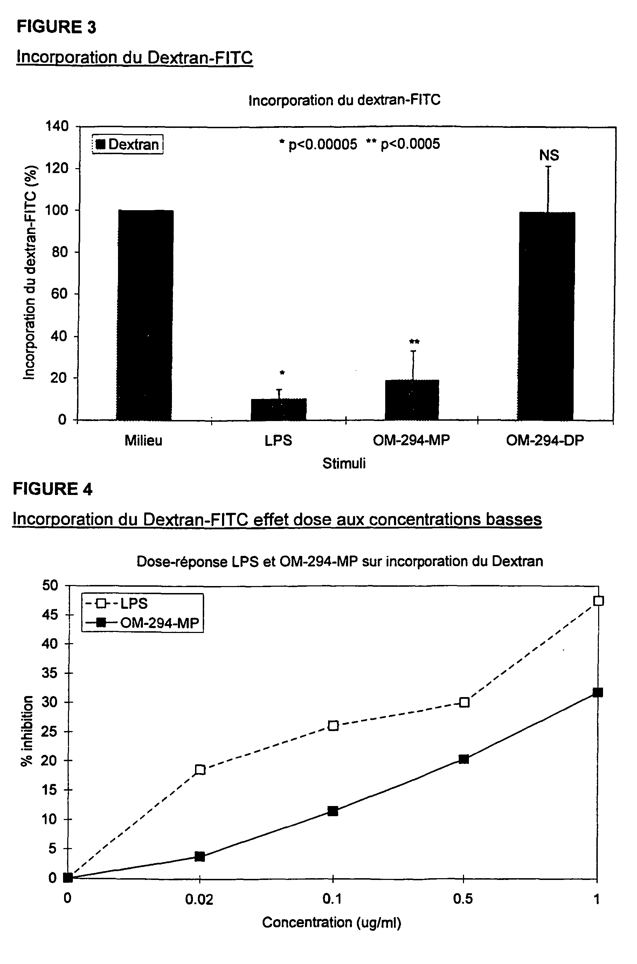

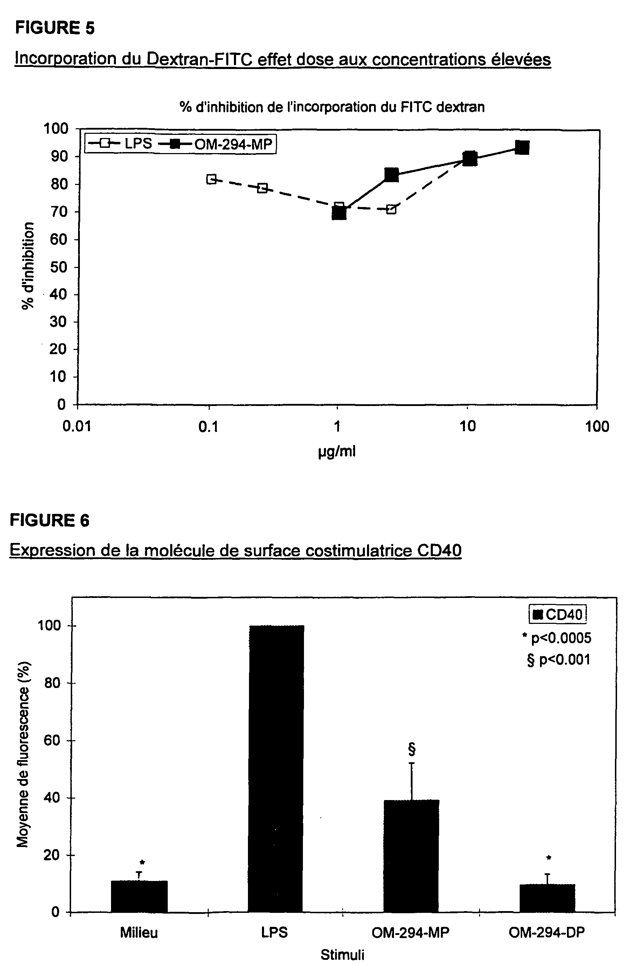

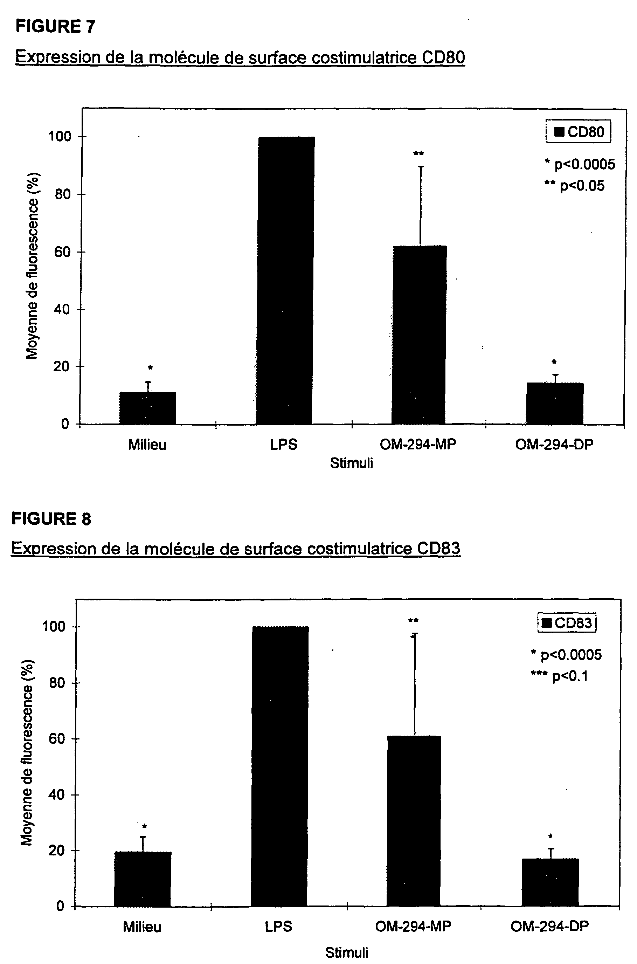

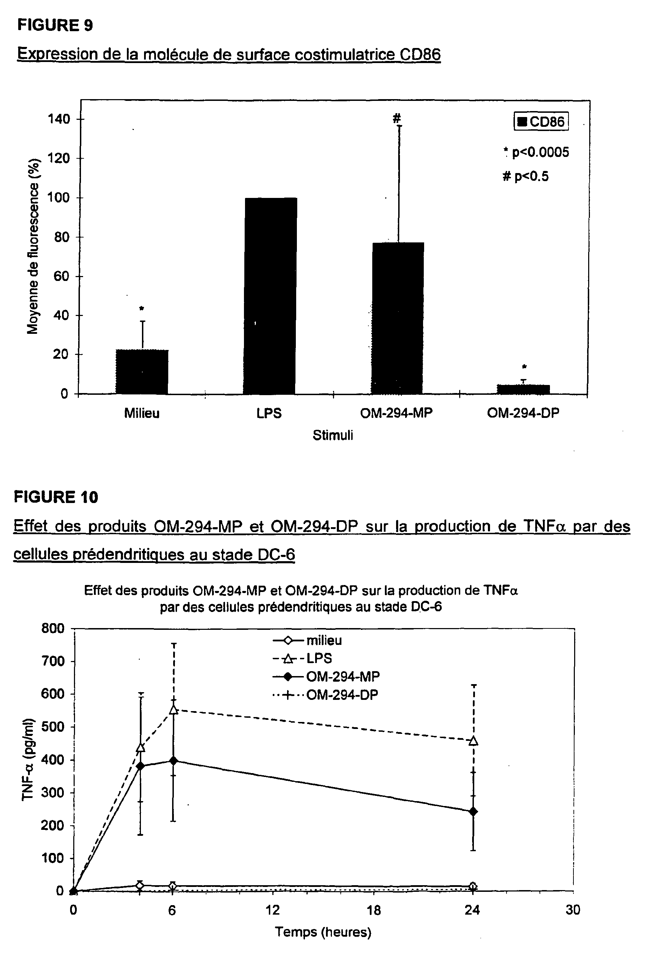

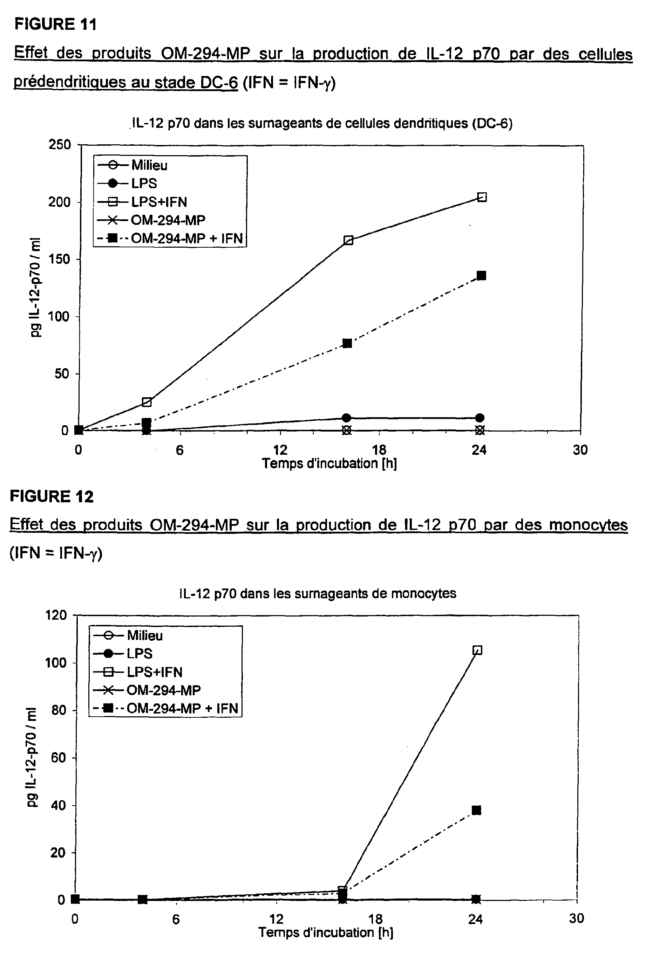

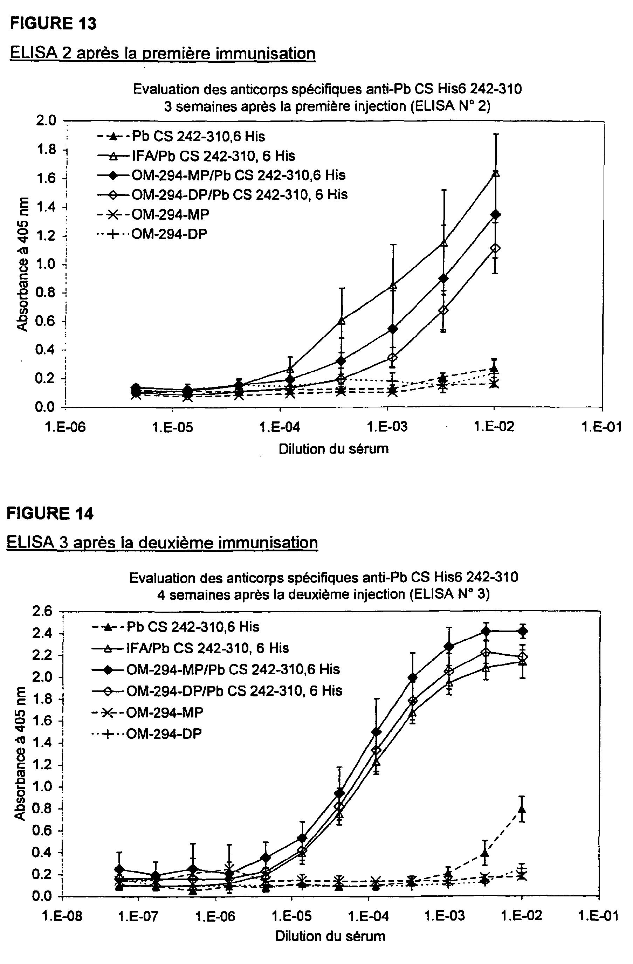

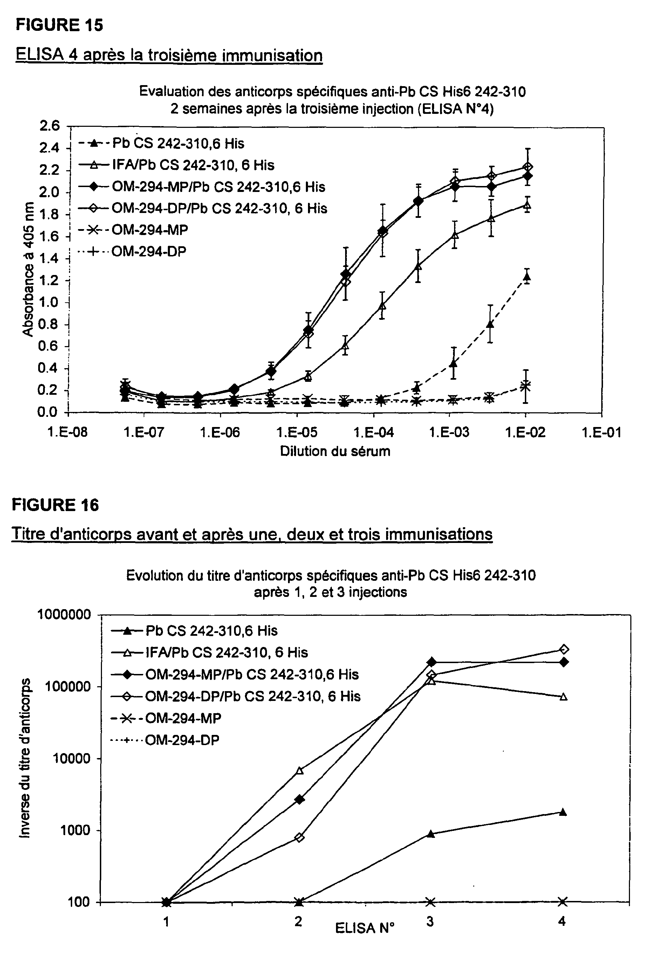

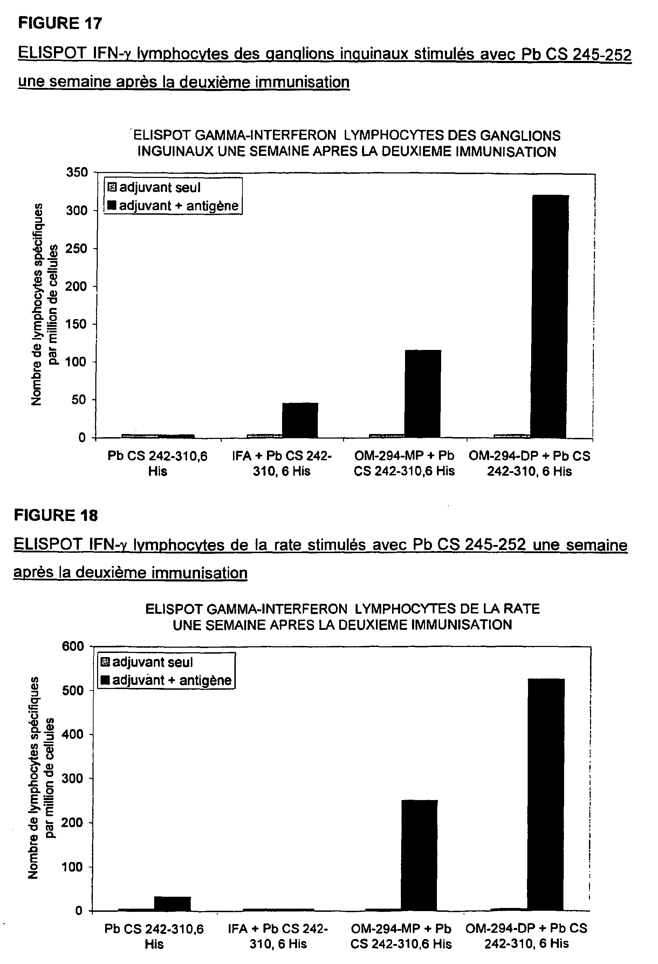

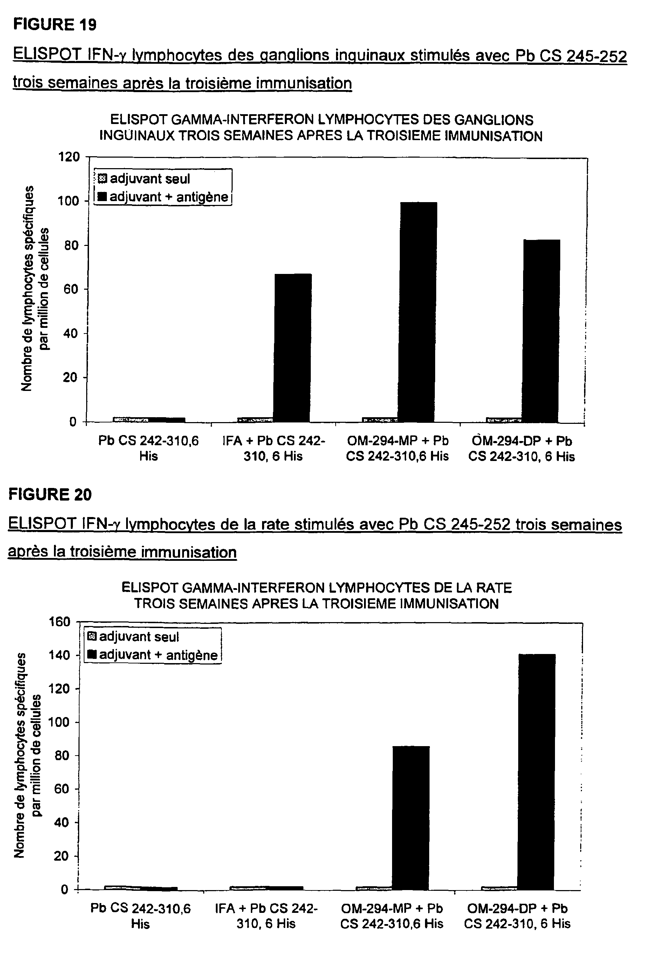

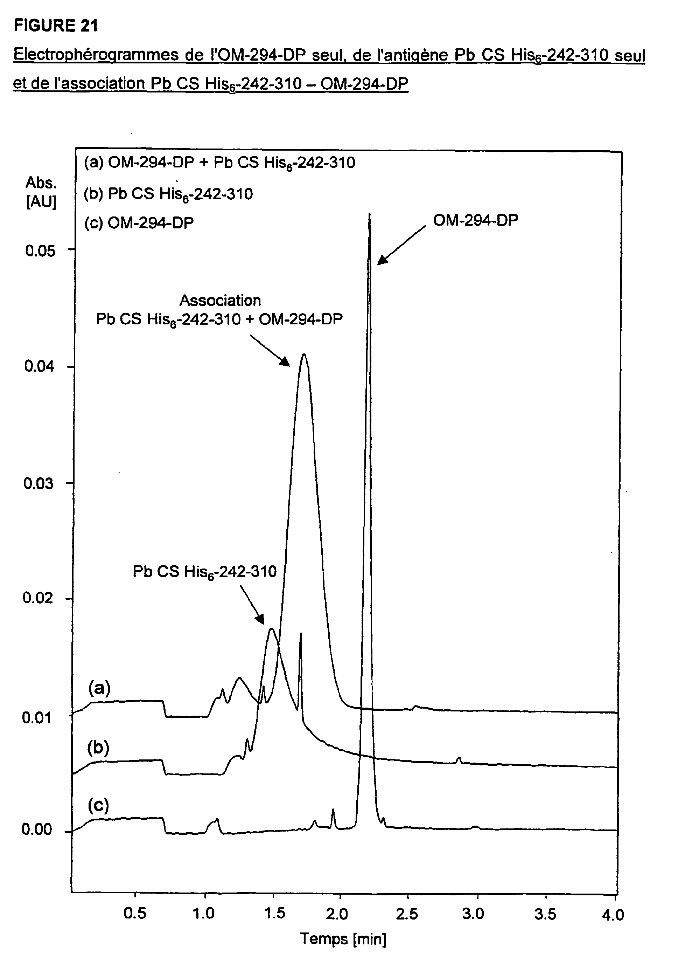

Cellules : les cellules mononucléées du sang périphérique sont isolées des *"buffy* coats" de 6 donneurs sains. Les donneurs n'ont subi aucun traitement avant le don du sang.
Préparation des cellules : les monocytes purifiés par adhérence sont remis en suspension dans du milieu RPMI-1640 (Sigma-Aldrich, St-Louis, MO, U.S.A.) contenant 10% de sérum de veau foetal, du GM-CSF (10 ng/ml; IM-HGM1, Immungenex Corp, Los Angeles, CA, U.S.A.) et de l'IL-4 (10 ng/ml; No 204-IL, R&D System, Minneapolis, MN, USA) à raison de 1 x 10⁶ cellules/ml et distribués dans des boîtes de Pétri de 10 cm de diamètre (P10, Falcon, Becton Dickinson, Plymouth, UK) (10 x 10⁶ cellules par boîte P10) pendant 6 jours (avec un changement de milieu après 3 jours). Ces cellules sont appelées cellules prédendritiques (DC-6). La maturation des cellules prédendritiques en cellules dendritiques matures est réalisée par incubation avec OM-294-MP, OM-294-DP ou LPS pendant 3 jours supplémentaires aux concentrations indiquées ci-après sous Produits. Au jour 9 (DC-9), les cellules sont récoltées pour analyser les différents paramètres indicateurs de la maturation des cellules dendritiques: les molécules de surface CD40, CD80, CD83, CD86 ainsi que la capacité d'incorporer du Dextran-FITC sont évalués. Tous ces paramètres sont analysés par FACS EPICS-XL-MCL (Coulter Immunology, Hialeah, Finlande).
Lanzavecchia *et al.,* J. Exp. Med 179 (1994) 1109; Lanzavecchia *et al.,* J. Exp. Med 182 (1995) 389.
Evaluation des résultats : l'expression des molécules de surface est exprimée en % de la fluorescence moyenne des cellules stimulées par LPS (témoin positif) ; l'incorporation du Dextran est calculée par rapport à celle des cellules maintenues dans le milieu et est exprimée en %. L'analyse statistique par le test t-student compare les valeurs obtenues dans les différents tests aux valeurs du témoin positif. Les valeurs de p<0.05 sont considérées comme significatives.
Produits : les solutions mères de OM-294-DP et OM-294-MP sont préparées à 1 mg/ml dans 0.9% NaCl/eau, additionnée de 0.1% triéthylamine pour l'OM-294-MP. Les solutions sont incubées à 37°C pendant 20 min, agités vigoureusement pendant 3 min puis dilués à 100 µg/ml dans du milieu de culture RPMI 1640 et utilisés soit à 10 µg/ml (Fig. 3, 6, 7, 8) soit à des concentrations comprises entre 0.02 et 25 µg/ml (Fig. 4, 5)
Produit de référence : lipopolysaccharide de E. coli (LPS, DIFCO, Detroit, MI, U.S.A.), solution stock 5 mg/ml dans PBS. Une solution intermédiaire est préparée à 100 µg/ml dans du milieu de culture RPMI 1640. Les concentrations testées sont soit de 10 µg/ml (Fig. 3, 6, 7, 8) soit des valeurs de 0.02 à 10 µg/ml (Fig. 4, 5)

### Résultats

Les cellules dendritiques immatures (DC-6) issues de la différenciation des monocytes, par l'effet conjugué de GM-CSF et d'IL-4, sont capables d'incorporer le Dextran-FITC. Au cours du processus de maturation, les cellules perdent la capacité d'incorporer le Dextran-FITC. Les analyses sont effectuées au stade de différenciation DC-9.
Les résultats sont exprimés en % de l'incorporation du Dextran-FITC observée dans les cellules non stimulées (milieu) Fig. (3). Les cellules traitées avec du LPS ou OM-294-MP ne conservent respectivement que 10% et 19% de phagocytose, tandis que les cellules stimulées par OM-294-DP gardent la totalité de leur capacité d'incorporer le Dextran (98 et 99%). Une courbe dose réponse indique que l'OM-294-MP montre des capacités exceptionnelles à induire une différenciation des cellules DC-6 en DC-9 à des concentrations allant de 0.02 µg à plus de 25 µg par ml, voir Fig. (4) basses concentrations et Fig. (5) concentrations plus élevées.
L'expression des molécules de surface costimulatrices est un autre critère de maturation des DC. L'expression des CD40, CD80, CD83, CD86 est testée. Les résultats sont exprimés en % de la moyenne de fluorescence par rapport à l'expression de ces marqueurs induite par le LPS.
OM-294-MP augmente l'expression de toutes les molécules de surfaces analysées : CD40 (39%), CD80 (62%), CD83 (60%), CD86 (77%) voir Fig. (6, 7, 8, 9).
OM-294-DP montre un effet similaire à celui du milieu de culture sur l'expression des marqueurs étudiés. Cet effet ne dépasse pas 20% de l'effet du LPS.

### 7. Effet des produits OM-294-MP et OM-294-DP sur la production de TNFα et d'IL-12 p70 par des monocytes et des cellules prédendritiques au stade DC-6.

Des cellules DC-6 (5 x 10⁵/500 µl de milieu) sont stimulées pendant 4h, 6h et 24h, soit par le LPS (10 µg/ml) soit par l'OM-294-MP (10 µg/ml) ou l'OM-294-DP (10 µg/ml).

### Protocole

Conditions expérimentales in vitro : Les cellules mononucléées du sang périphérique sont isolées des "buffy coats" de 6 donneurs sains (les donneurs n'ont subi aucun traitement avant le don du sang). Les monocytes sont isolés sur un gradient de Ficoll puis purifiés par adhérence. Les monocytes adhérents d'une manière lâche sont ensuite récoltés et une partie des cellules gardées comme monocytes. Les monocytes purifiés sont remis en suspension dans du milieu RPMI-1640 contenant 10% de FCS, à raison de 1 x 10⁶ cellules/ml et distribués dans des boîtes de Pétri de 10 cm de diamètre (P10 - Falcon, Becton Dickinson, Plymouth, UK) à raison de 10 x 10⁶ cellules/boîte P10. Les cellules sont mises en culture dans le milieu RPMI 1640 complet contenant du GM-CSF (10 ng/ml) et de l'IL-4 (10 ng/ml) pendant 6 jours. Au jour 6, les cellules sont récoltées, lavées 3 fois avec HBSS et réparties dans une plaque de 24-puits à raison de 5 x 10⁵ cellules par puits dans 500 µl de milieu RPMI complet et stimulées avec LPS (10 µg/ml), OM-294-MP (10 µg/ml) ou OM-294-DP (10 µg/ml). Le TNFα ainsi que l'IL-12 p70 sont dosés par ELISA dans les surnageants des cultures qui sont recueillis après 4, 6 et 24 heures.
Produits : les produits OM-294-MP et OM-294-DP (solution stock à 1 mg/ml dans de l'eau stérile) sont incubés à 37°C pendant 20 min et agités vigoureusement pendant 3 min puis dilués à 100 µg/ml et utilisés à la concentration finale de 10 µg/ml dans du milieu de culture RPMI 1640.
Produit de référence : lipopolysaccharide de *E. coli* (LPS, DIFCO, Detroit, MI, U.S.A.), solution stock 5 mg/ml dans PBS, solution intermédiaire dans le milieu de culture : 100 µg/ml, utilisé à la concentration finale de 10 µg/ml.
Dosage du TNFα et de l'IL-12 p70 : Kit TNF-α Biosource KHC3012, lot PP003-J061703 (Biosource International, Camarillo, CA, U.S.A.). Protocole ELISA selon notice du kit du fournisseur. L'IL-12 p70 est dosé dans les surnageants des cultures par ELISA en utilisant le kit pour IL-12 humaine (No D1200, lot 990 6232, R&D Systems, Minneapolis, MN, USA).

### Résultats

### TNF-α

L'OM-294-MP stimule la production de TNFα par les cellules DC-6 de façon similaire à celle du LPS tant du point de vue de la cinétique de production que de la concentration de TNFα (Fig (10)). Le pic de TNFα est situé pour les deux produits entre 6 h et 24h.
L'OM-294-DP ne stimule que marginalement la production de TNFα par les cellules DC-6.

### IL-12 p70

D'une manière générale, l'IL-12 est induite en présence d'IFN-γ (LPS+IFN-γ, OM-294-MP + IFN-γ) dans les monocytes (Fig (12)) et les DC-6 (Fig (11)). La cytokine apparaît plutôt dans les DC que dans les monocytes.

### 8. Evaluation des propriétés adjuvantes de OM-294-DP et OM-294-MP dans un modèle d'immunisation des souris avec un peptide synthétique (Pb CS His₆-242-310) de la région C-terminale de la protéine de surface du circumsporozoite de Plasmodium berghei.

### Protocole

Antigène: le peptide Pb CS (HHHHHHGGMN NKNNNNDDSY IPSAEKILEF VKQIRDSITE EWSQCNVTCG SGIRVRKRKG SNKKAEDLTL EDIDTEI), dénommé ci-après His₆-242-310, correspondant à la séquence des acides aminés 242 à 310 de la protéine du circumsporozoite de *Plasmodium berghei* souche ANKA additionnée sur la partie N-terminale de 6 résidus histidine, de 2 glycines et d'une méthionine est obtenu par synthèse selon la méthode de Merrifield et Atherton (Atherton et al., Bioorg. Chem. 8 (1979) 350-351,). Le polypeptide est préparé sur résine p-alkoxybenzylalcool (résine de Wang) avec un degré de substitution de 0.4 mmol/g. Un excès molaire de 10x des dérivés F-moc des acides aminés est utilisé avec une durée de couplage de 30 min. Le peptide est purifié par chromatographie d'exclusion de taille (Sephadex G25, Pharmacia, S), puis par chromatographie sur phase inverse (W-Porex 5 C-4, 250 x 10 mm, Phenomenex, Torrance, CA, U.S.A.) avec un gradient, en 40 min, allant d'un mélange de 10 à 50% acétonitrile dans 0.1% acide trifluoroacétique/eau (v/v), débit 3 ml/min. La composition en acides aminés du peptide purifié est déterminée selon Knecht et Chang (Anal. Chem. 58 (1986) 2373-2379,) et le poids moléculaire est confirmé par spectrométrie de masse sur Voyager-DE (Perseptive Biosystem, Framingham, MA, U.S.A.). La solution mère de l'antigène est préparée à 0.4 mg/ml dans 0.9% NaCl/eau à pH 8.0.
Adjuvants: les solutions mères de OM-294-DP et OM-294-MP sont préparées à 1 mg/ml dans 0.9% NaCl/eau, additionnée de 0.1% triéthylamine pour l'OM-294-MP. Le témoin positif est constitué par l'adjuvant incomplet de Freund (IFA de Difco, Detroit, MI, U.S.A.) et le témoin négatif est une solution de 0.9% NaCl.
Mélange antigène-adjuvant: un volume d'antigène et un volume d'adjuvant sont mélangés 3 min par Vortex.
Immunisation: des souris femelles BALB/c âgées de 6 semaines (6 souris par groupe) sont immunisées à trois reprises, par injection sous-cutanée à la base de la queue de 0.1 ml des mélanges suivants:

| groupe | adjuvant 0.05 mg/injection | antigène 0.02 mg/injection | nombre de souris |
|---|---|---|---|
| 1 | --- | Pb CS His₆ -242-310 | 6 |
| 2 | IFA | Pb CS His₆ -242-310 | 6 |
| 3 | OM-294-MP | Pb CS His₆ -242-310 | 6 |
| 4 | OM-294-DP | Pb CS His₆ -242-310 | 6 |
| 5 | OM-294-MP | --- | 6 |
| 6 | OM-294-DP | --- | 6 |

### Schéma d'immunisation et des prélèvements :

| Semaines | 0 | 3 | 4 | 7 | 9 |
|---|---|---|---|---|---|
| Immunisations | ↑ | ↑ | | ↑ | |
| Réponse anticorps spécifiques | ↑ | ↑ | | ↑ | ↑ |
| Réponse CTL | | | ↑ | | ↑ |

### Prélèvements du sang et des organes lymphoides :

Obtention du sérum: des prélèvements de sang sont effectués aux temps 0, 3, 7 et 9 semaines. Le sang est laissé reposer 60 min à 37°C, puis placé pendant une nuit à 4°C. Le sérum est ensuite congelé à -80°C jusqu'au dosage des anticorps.
Prélèvement des ganglions inguinaux et de la rate : quelques animaux de chaque groupe sont sacrifiés après 4 ou respectivement 9 semaines. Les ganglions inguinaux et la rate sont prélevés chirurgicalement.

### Détermination du titre d'anticorps anti-Pb CS His₆ 242-310:

Le dosage du titre d'anticorps dirigé spécifiquement contre l'antigène Pb CS His₆ 242-310 est réalisé par ELISA. La fixation de l'antigène est effectuée en microplaque à 96 puits (Maxisorp F 96, Nunc, DK) par incubation pendant une nuit en chambre humide à 4°C avec dans chaque puits 0.1 ml de PBS (*phosphate buffered saline*) contenant 0.001 mg/ml d'antigène Pb CS His₆-242-310. La saturation de la microplaque est effectuée avec du PBS contenant 1 % d'albumine sérique bovine (BSA, Fluka, CH). Les plaques sont lavées avec du PBS contenant 0.05% de Tween 20 (Sigma, Saint-Louis, MO, U.S.A.). Les sérums prélevés aux temps 0, 3, 7 et 9 semaines sont dilués en série avec le tampon de dilution (PBS contenant 2.5% de lait en poudre dégraissé et 0.05% de Tween 20), puis transférés dans la microplaque et laissés 1 h à température ambiante (TA). Les plaques sont ensuite lavées avec du PBS, la solution de dilution contenant l'anticorps polyclonal anti-immunoglobuline de souris conjugué à la phosphatase alcaline (Sigma, Saint-Louis, MO, U.S.A.) est ajoutée aux plaques et incubée 1 h à TA. Les plaques sont lavées avec du PBS et les anticorps spécifiques mis en évidence par réaction colorimétrique avec le substrat de la phosphatase alcaline, le p-nitrophenylphosphate (Sigma, Saint-Louis, MO, U.S.A.). L'absorbance à 405 nm est mesurée avec un lecteur de microplaque (Dynatech 25000 ELISA reader, Ashford, Middlesex, UK), chaque sérum est déterminé en duplicat. Les résultats présentés sont la moyenne des valeurs obtenues pour les souris de chaque groupe. Le titre d'anticorps est déterminé par la dernière dilution induisant une réponse positive significative, c'est-à-dire avec une valeur de densité optique supérieure à la valeur du bruit de fond additionnée de 3 écart-types.

### Dosages ELISPOT :

Les anticorps spécifiques anti-interféron-γ de souris (O1E703B2) sont fixés par incubation pendant une nuit à 4 °C en chambre humide, d'une solution d'anticorps à 50 µg/ml dans une microplaque ELISPOT dont le fond des puits est couvert de nitrocellulose (Millipore, Molsheim, F),. L'étape de saturation est effectuée par ajout de DMEM (Life Technologies, Grand Island, NY, U.S.A.) contenant 10% de sérum de veau foetal (FCS, Fakola, CH) pendant 2 heures à 37°C. Les cellules obtenues à partir des organes lymphoides (ganglions inguinaux et rate) sont mises en culture dans les microplaques à 200'000 cellules par puits, puis co-cultivées pendant 24h à 37°C avec 100'000 cellules P815 pulsées ou non avec le peptide court Pb CS 245-252. Après l'incubation, les cellules sont éliminées et après lavage, un second anticorps anti-IFN-γ de souris biotinylé (ANI, 2 µg/ml dans PBS avec 1% BSA) est ajouté pendant 2 h. La streptavidine conjuguée à la phosphatase alcaline (Boehringer Mannheim, Mannheim, RFA) est ajoutée et incubée 1 h à 37°C, 3 lavages sont effectués avec du PBS contenant 0.05% de Tween 20, suivi par 3 lavages avec du PBS. La présence des complexes immuns anti-IFN-γ est mise en évidence par l'ajout du substrat BCIP/NBT (Sigma, St-Louis, MO, U.S.A.). La réaction est arrêtée par lavage à l'eau courante. Les spots positifs à l'IFN-γ sont alors comptés au microscope binoculaire. Les spots spécifiques correspondent à la différence entre le nombre de spots comptés en présence des cellules pulsées avec le peptide et le nombre de spots comptés en l'absence de peptide. Les résultats représentent sont la moyenne des valeurs obtenues pour les souris de chaque groupe. Ils sont exprimés en nombre de spots par million de cellules mises en culture.

### Résultats

Réponse anticorps : la production spécifique des anticorps anti-Pb CS His₆-242-310, déterminée par ELISA est présentée graphiquement pour les souris ayant reçu une, deux et trois immunisations. Le contrôle effectué avec une seule injection de l'antigène seul donne un titre d'anticorps très faible. Le titre d'anticorps atteint avec une seule injection de l'antigène mélangé avec l'OM-294-MP ou respectivement l'OM-294-DP est déjà pratiquement aussi élevé qu'avec l'adjuvant de Freund incomplet (IFA) mélangé au même antigène (Fig. (13)). Après deux injections, les adjuvants OM-294-MP et OM-294-DP sont capables d'éliciter une réponse sérologique respectivement supérieure ou égale à celle de l'IFA (Fig (14)). Après trois injections les adjuvants OM-294-MP et OM-294-DP sont capables d'éliciter une réponse sérologique supérieure à celle de l'IFA (Fig. (15)).
Fig. (13) ELISA, effectué 3 semaines après la première immunisation.
Fig. (14) ELISA, effectué 4 semaines après la deuxième immunisation.
Fig. (15) ELISA, effectué 2 semaines après la troisième immunisation.
Fig. (16) Titre d'anticorps avant et après une, deux et trois immunisations.
Les titres d'anticorps des animaux de chaque groupe avant l'immunisation et après une, deux et trois immunisations sont présentés en tant que moyenne (Fig. (16)).
Réponse CTL : la reconnaissance de l'épitope T Pb CS 245-252 présent dans le peptide Pb CS His₆ -242-310 ayant servi à l'immunisation est bien mise en évidence par le test de l'ELISPOT. La réponse des lymphocytes T provenant des animaux immunisés (ganglions inguinaux et rate, prélevés une semaine après la deuxième injection et respectivement deux semaines après la troisième injection) est mise en évidence par une augmentation du nombre de spots positifs pour l'interféron γ (IFN-γ ). Les résultats présentés dans les Fig (17, 18, 19, 20) sont les moyennes des valeurs obtenues à chaque dilution et pour les souris de chaque groupe. Ils sont exprimés en nombre de spots par million de cellules mises en culture.
Les deux adjuvants OM-294-MP et OM-294-DP augmentent de façon très significative la réponse CTL des lymphocytes provenant de la rate et des ganglions inguinaux. Les réponses de la rate sont supérieures à celles des ganglions inguinaux. Les adjuvants OM-294-MP et OM-294-DP induisent une activité CTL nettement supérieure à celle de l'IFA.

### 9. Mise en évidence de l'association non-covalente OM-294 - antigène par électrophorèse capillaire.

L'électrophorèse capillaire est utilisée dans cet exemple pour mettre en évidence une association non-covalente entre l'OM-294-DP et le peptide Pb CS His₆-242-310 lors de la formulation de la préparation vaccinale.

### Protocole

Méthode d'analyse :
tampon 20 mM borate de sodium (tétraborate de di-sodium décahydraté, Merck N° 6306) pH ajusté à 7.4 avec NaOH 1N (Fluka N° 72072)
Capillaire de zone (non greffé), longueur 30 cm, diamètre 50 µm.
Détection à 200 nm sur appareil Beckman PACE MDQ (Beckman , Brea, CA, U.S.A.).

### Conditions de séparation

| Temps [min.] | Action | Pression | Solvant |
|---|---|---|---|
| 0.00 | Lavage capillaire | 20.0 psi | H₂O |
| 3.00 | Lavage capillaire | 20.0 psi | NaOH 1N |
| 6.00 | Injection de l'échantillon | 0.5 psi | Tampon borate |
| 6.08 | Séparation | 30.0 KV | Tampon borate |

Antigènes : Peptide synthétique Pb CS His₆-242-310 à 1 mg/ml dans H₂O
Adjuvant : OM-294-DP à 1 mg/ml dans H₂O
Mélange antigène - adjuvant : 250 µg/ml + 250 µg/ml

### Résultats

L'association antigène - adjuvant de cette formulation d'une préparation vaccinale est mise en évidence sur l'électrophérogramme par la disparition du pic de l'adjuvant et le déplacement du pic de l'antigène au profit d'un nouveau pic spécifique de l'association (Fig (21)).

### 10. Traitement d'une carcinomatose péritonéale induite par l'injection de cellules de la lignée tumorale syngénique PROb chez le rat BDIX.

L'objectif de cette expérience est de mettre en évidence un effet antitumoral de l'OM-294-DP administré par voie parentérale i.v. répétée aux rats porteurs de tumeurs macroscopique de quelques mm.

### Protocole

Les animaux : la souche de rats BDIX consanguins a été établie en 1937 par H. Druckrey. Un couple de rats provenant du Max Planck Institut de Fribourg (RFA) est à l'origine de la colonie maintenue depuis 1971 dans l'animalerie du laboratoire par le système de la lignée unique. Selon ce système, à chaque génération, un seul couple frère-soeur est choisi pour donner les descendants de la génération suivante. Les rats utilisés pour ce travail vient du Centre d'Elevage des Animaux de Laboratoire d'Iffa-Credo (l'Arbresle, F) à qui le laboratoire a confié l'élevage de la souche. Les rats utilisés sont des mâles âgés de 3 mois ± 1 semaine.

### Induction des tumeurs par injection des cellules PROb :

Origine des cellules PROb : une greffe d'un fragment d'un carcinome colique induit chez un rat BDIX consanguin par la 1,2-diméthylhydrazine est à l'origine de la lignée cellulaire DHD/K12. Cette lignée de cellules adhérentes a été subdivisée en deux sous-lignées en fonction de la sensibilité des cellules à la trypsine, et ont été appelées DHD/K12-TR les cellules se détachant difficilement. Les cellules DHD/K12-TR injectées à des rats BDIX syngéniques induisent des tumeurs progressives. Cette lignée est clonée, seul le clone DHD/K12-TRb désigné ultérieurement PROb est utilisé pour ce travail.
Conditions de culture : les cellules PROb, adhérentes, sont cultivées en flacons de culture fermés (Falcon, Becton Dickinson, New-Jersey, USA), à 37°C dans du milieu complet composé de milieu F10 de Ham (Bio-Whittaker, Walkersville, USA) auquel sont ajoutés 10% de sérum de veau foetal (FCS, Anval, Betton, F). Ce milieu de culture est changé tous les 3 jours. A confluence, les cellules sont détachées de leur support par 2 ml d'une solution EDTA/trypsine en 3 à 5 min, ceci après 3 rinçages de 2 ml de la même solution pendant 2 à 3 min; les cellules sont reprises par du milieu complet, le FCS bloquant l'action de la trypsine. L'absence de contamination des cellules par des mycoplasmes et bactéries est vérifiée régulièrement par coloration de l'ADN avec le fluorochrome Hoechst 33258 (Aldrich Chimie, Steinheim, RFA).

Induction de la carcinomatose péritonéale : les cellules PROb sont détachées de leur support comme indiqué au paragraphe "conditions de culture" et sont comptées dans une solution de bleu trypan, colorant qui permet d'apprécier la viabilité cellulaire. Les cellules sont mises en suspension dans du milieu F10 de Ham. Les carcinomatoses péritonéales sont induites par injection intrapéritonéale (i.p.) de 10⁶ cellules PROb viables à un rat BDIX syngénique anesthésié à l'éther. Le jour J0 correspond au jour d'injection des cellules tumorales. Dans ces conditions, tous les rats développent une carcinomatose péritonéale avec production d'ascite hémorragique et meurent entre la 6ème et la 12ème semaine après l'injection des cellules.
Traitement des carcinomatoses péritonéales : le traitement commence 13 jours après l'injection des cellules tumorales lorsque les carcinomatoses sont constituées de nodules de quelques mm de diamètre. Il consiste en 10 injections i.v. d'OM-294-DP à la dose de 1 mg/kg et à la concentration de 0.6 mg/ml dissous dans du NaCl 0.9%. Les injections sont faites 3 fois par semaine (lundi, mercredi et vendredi) dans la veine du pénis. Le groupe témoin est traité avec le véhicule seul NaCl 0.9%.
Evaluation de l'efficacité du traitement : à J42, 6 semaines après l'injection des cellules tumorales, les rats sont sacrifiés et autopsiés, les carcinomatoses sont évaluées en aveugle. II n'est pas possible de mesurer le volume d'une carcinomatose, par contre il est possible de classer les carcinomatoses en différentes catégories. Cinq classes sont définies en fonction du nombre et du diamètre des nodules:
Classe 0 : aucun nodule n'est visible
Classe 1 : des nodules de 0,1 à 0,2 cm de diamètre peuvent être dénombrés
Classe 2 : de nombreux nodules de 0,1 à 0,5 cm qui ne peuvent plus être comptés
Classe 3 : les nodules, dont certains atteignent un cm de diamètre, envahissent la cavité péritonéale
Classe 4 : la cavité est entièrement envahie par des masses tumorales de plusieurs cm.
Evolution du volume de l'ascite et du poids des animaux : le volume d'ascite est mesuré par double pesée des rats. Un groupe de rats témoins, n'ayant eu aucun traitement mais des injections de NaCl 0.9%, permet d'apprécier l'évolution normale de la carcinomatose et d'évaluer l'effet du traitement.

Détermination de l'efficacité du traitement : la durée de vie des rats des groupes traités est comparée à celle du groupe témoin; le volume des carcinomatoses et des ascites des rats des groupes traités sont comparés à ceux des rats du groupe témoin.
Etude statistique : La signification statistique de l'effet de l'immunothérapie est déterminée par un test de Kruskal-Wallis pour la classification des carcinomatoses, un test d'analyse de variance pour le volume d'ascite, un test de log rank pour les survies.

### Résultats

Carcinomatoses : l'OM-294-DP montre une activité antitumorale remarquable dans ce modèle. Cette activité est plus particulièrement mise en évidence par le nombre d'animaux sans tumeurs (classe 0) et la différence avec le témoin NaCl est significative (p<0;05) pour le volume tumoral. L'impact du traitement avec OM-294-DP sur le volume d'ascite est aussi significatif (p<0;05).

**Tab (a) Classe des carcinomatoses et volume d'ascite**

| Traitement | Nombre de rats présentant des carcinomatoses de classe | | | | | Effet du produit (*) | Volume d'ascite ml/rat | | Effet du produit (**) |
|---|---|---|---|---|---|---|---|---|---|
| | 0 | 1 | 2 | 3 | 4 | | limites | moyenne ± σ | |
| NaCl ⁽¹⁾ | 1 | 0 | 1 | 0 | 7 | --- | 0-84 | 38 ± 29 | --- |
| OM-294-DP⁽²⁾ | 4 | 1 | 2 | 1 | 2 | p<0.05 | 0-73 | 8 ± 23 | p<0.05 |

| | | | | | | | | | |
|---|---|---|---|---|---|---|---|---|---|
| (*) : test de Kruskall Wallis; (**) : analyse de variance. ⁽¹⁾ : 8 des 10 rats sont morts de leur cancer avant le sacrifice, 1 à J34 présentait des nodules et un ictère mais la classe n'a pu être déterminée (cannibalisme), 1 à J37 (classe 4), 2 à J38 (classe 4), 1 à J39 (classe 4), 2 à J40 (classe 4) et 1 à J41 (classe 4). Le rat de classe 0 présentait à l'autopsie une tumeur sous cutanée, il est probable que l'injection des cellules cancéreuses ait été manquée. ⁽²⁾ : 1 rat est mort à J14 lors de la 1 ère injection du traitement, il ne présentait pas de carcinomatose. L'un des rats sacrifiés de classe 0 comportait une tumeur sous cutanée (injection des cellules cancéreuse manquée). | | | | | | | | | |

### Survie :

Les animaux sont sacrifiés à J42. La survie déterminée au 42^{ème} jour après l'injection des cellules tumorales, 90% des animaux traités avec OM-294-DP ont survécu, alors que dans le groupe non traité seul 20 % des animaux sont encore en vie.
L'OM-294-DP prolonge de façon significative la survie des rats (p<0.001).

### Poids :

L'OM-294-DP n'a pas d'effet significatif sur l'évolution du poids par rapport aux animaux ayant reçu le NaCI seul comme l'indique les valeurs du Tab (b).

**Tab (b) évolution des poids des rats (moyennes ± écart-type)**

| Jours | NaCl | OM-294 DP |
|---|---|---|
| 0 | 314 ± 19 | 277 ± 19 |
| 13 | 337 ± 18 | 310 ± 21 |
| 20 | 342 ± 20 | 304 ± 23 |
| 29 | 361 ± 23 | 317 ± 24 |
| 41 | 314 ± 38 | 327 ± 26 |

### 11. Evaluation des propriétés de l'adjuvant OM-294-DP dans un modèle d'immunisation chez la souris par voie nasale avec la sous-unité B de l'uréase de Helicobacter pylori

Il est démontré que des souris peuvent être protégées d'une infection par *Helicobacter pylori* en les immunisant par voie orale ou nasale avec la sous-unité B de l'uréase d'*Helicobacter pylori* (UreB) en présence de l'adjuvant *cholera toxin* (CT) Corthésy-Teulaz I. et al, Gastroenterology 109 (1995) 115. ; Michetti P. et al, Gastroenterology 116 (1999) 804 ; Saldinger P.F. *et al,* Gastroenterology 115 (1998) 891. Cette réponse humorale anti-UreB mesurée dans le sérum des souris immunisées est principalement de type IgG1 (réponse Th2). L'effet adjuvant de l'OM-294-DP est évalué chez des souris BALB/c (n=6) immunisées 4 fois à une semaine d'intervalle par voie nasale avec la sous-unité B de l'uréase de *Helicobacter pylori* recombinante (UreB) en présence de l'adjuvant OM-294-DP. Des souris BALB/c témoins sont immunisées avec l'adjuvant OM-294-DP seul. Deux semaines après la dernière immunisation, du sang de chaque souris est prélevé et le dosage par ELISA d'immunoglobulines anti-UreB dans le sérum (IgG totaux, IgG1 et IgG2a) est effectué.

### Protocole

Animaux : Souris BALB/c/Ola/HsD (Harland, Horst, Hollande) : 24 souris
Antigène : *Hp*UreB 1-569, exprimée comme protéine recombinante dans *E. coli* (souche M15, Qiagen, Hilden, D) selon le protocole précédemment décrit (Michetti et al, Gastroenterology 107 (1994) 1002).
Adjuvant : OM-294-DP (solution stock à 2.2 mg/ml)

### Protocole d'immunisation :

Quatre groupes de 6 souris ont été constitués :
Groupe A: 6 souris BALB/c sont immunisées 4 fois par voie nasale avec 25 µg OM-294-DP seul (25 µl par dose) une fois par semaine durant 4 semaines consécutives.
Groupe B : 6 souris BALB/c sont immunisées 4 fois par voie nasale avec 50 µg UreB 1-569 + 25 µg OM-294-DP (25 µl par dose) une fois par semaine durant 4 semaines consécutives.
Deux semaines après la dernière immunisation nasale, du sang de chaque souris des groupes A et B est prélevé par la queue.

### Dosage des IgG dans le sérum :

Tampon de coating (pH 9.6) : pour 1 litre, Na₂CO₃ (15 mM , 1.59 g), NaHCO₃ (34.8 mM, 2.93 g), Thimerosal (0.01%); Tampon PBS-Tween pH 7.4 : pour 1 litre, NaCl (137 mM, 8.0 g), KH₂PO₄ (1.5 mM, 0.2 g), Na₂HPO₄ (8.0 mM, 1.15 g), KCl (2.7 mM, 0.2 g), Tween 20 (0.1%, 1 ml); Tampon citrate/phosphate pH 5.0 : pour 1 litre, acide citrique (44.4 mM, 9.32 g), Na₂HPO₄ (103 mM, 14.6 g); Solution substrat (O-phenyl diamine = OPD) 10 x conc : OPD (10 mg/ml dans tampon citrate); Solution d'azidure de sodium : 1%; Solution stop : 0.01% d'azidure de sodium dans du tampon citrate/phosphate 0.1 M pH 5.0
Méthode : une solution d'antigène (UreB 1-569 du 26.05.1999, solution stock 0.5 mg/ml) est préparée à la concentration de 5 µg/ml dans le tampon de coating pH 9.6 (pour 50 ml de tampon, 500 µl de la solution d'UreB). On pipète 100 µl par puits dans 3 plaques à 96 puits à fonds ronds (0.5 µg d'UreB par puit). On laisse incuber les plaques 2 heures à 37° C. On élimine le surnageant des plaques. On sature les puits en ajoutant 100 µl d'une solution de PBS-Tween 0.1% + 5% lait en poudre par puit. Incuber les plaques pendant 30 minutes à 37° C. On élimine la solution de saturation et laver 3 fois les puits avec 100 µl de PBS-Tween. On élimine le surnageant. On prépare une dilution de 1 :200 de chaque sérum de souris à tester dans tampon PBS-Tween 0.1 % (5 µl de sérum dans 1 ml de tampon PBS-Tween). Les sera (100 µl) sont répartis en dupliqués dans les 3 plaques (1 plaque pour détecter les IgG totaux, 1 plaque pour détecter les IgG1 et 1 plaque pour détecter les IgG2a). On incube toute la nuit à 4° C. On lave les puits 3 fois avec 100 µl de PBS-Tween. On prépare une dilution 1 :500 des solutions d'anticorps anti-IgG totaux couplé à la biotine (Amersham, Cat # RPN 1177), anticorps anti-IgG1 (Amersham Cat # RPN 1180) et anticorps anti-IgG2a (Pharmingen Cat # 02012D) dans le tampon PBS-Tween. On ajoute 100 µl de la solution d'anticorps anti-IgG totaux dans la plaque n°1, 100 µl de la solution d'anticorps anti-IgG1 dans la plaque n°2 et 100 µl de la solution d'anticorps anti-IgG2a dans la plaque n°3. On incube 1 heure à 37° C. Laver les puits 3 fois avec PBS-Tween. On prépare une dilution 1 :1000 de streptavidine-HRP (Dako, Cat # p0397) dans le tampon PBS-Tween et on ajoute 100 µl par puit. Incuber 30 minutes à 37° C. On lave les puits 3 fois avec 100 µl de tampon PBS-Tween. On prépare la solution de substrat en diluant 1 :10 la solution de OPD (10X) dans le tampon citrate/phosphate 0.1M. On ajoute 1 µl par ml de H₂O₂ dans la solution d'OPD diluée. On ajoute 50 µl de la solution de substrat dans chaque puits. On attend 10-20 minutes que la coloration se développe. Stopper la réaction en ajoutant 50 µl de tampon stop. L'absorbance est lue à 492 nm (avec 620 nm de référence de lecture) en utilisant le témoin négatif comme zéro.
Satistiques Les résultats sont présentés comme moyenne ± SD (n=6). Les valeurs de p sont calculées par test de Student. Les valeurs de p < 0.05 sont considérées comme significatives.

### Résultats

Les souris immunisées par voie nasale avec UreB 1-569+OM-294-DP développent une immunité humorale anti-UreB : présence d'anticorps IgG1 anti-UreB 1-569 dans le sang.
La présence d'anticorps spécifiques contre l'UreB de *Hp* dans le sérum des souris est mesuré par ELISA. L'UreB (0.5 µg/ puit) a été déposée dans des plaques à 96 à fonds ronds dans du tampon carbonate pH 9.6. Les anticorps spécifiques sont détectés à l'aide d'anticorps de lapin anti-IgG totaux, IgG1 et IgG2a. Les résultats sont donnés en densité optique (OD) mesurés à 492 nm. Les valeurs d'OD 3 fois supérieures aux valeurs mesurées dans le sérum des souris naïves sont considérées comme positives. Aucun anticorps anti-UreB n'est détecté dans le sérum des souris immunisées par OM-294-DP seul. Les souris immunisées par UreB+OM-294-DP développent également des anticorps IgG totaux anti-UreB (OD = 0.274 ± 0.130, p<0.05) et IgG1 anti-UreB (OD = 0.212 ± 0.128, p<0.05), mais ne développent pas d'anticorps IgG2a anti-UreB (OD = 0.008 ± 0.005, ns).

Des souris BALB/c immunisées par voie nasale avec la sous-unité B de l'uréase de *Helicobacter pylori* (UreB) + OM-294-DP développent, une réponse humorale anti-UreB majoritairement de type IgG1. OM-294-DP peut donc agir comme adjuvant par voie nasale et aider à développer une immunité humorale de type Th2.

### 12. OM-294-MP et OM-294-DP combinés avec l'antigène H1N1 : détermination d'anticorps spécifiques générés chez la souris après 1 ou 2 administrations par voie sous-cutanée.

### Protocole

Le but de cette étude est de montrer l'effet adjuvant de OM-294-MP et OM-294-DP pour l'antigène grippal H1N1 (haemagglutinine A/Beijing 262/95, Solvay Duphar, Weesp, NL). Pour cela 60 souris BALB/c (femelles, 8 semaines d'âge au début du traitement) sont réparties dans les 6 groupes suivants :

| GROUPES | ANTIGENE Final : 2.5 µg par animal/injection | ADJUVANTS Final : 50 µg par animal/injection | NaCl (0.9%) | VOLUME INJECTE |
|---|---|---|---|---|
| A: NaCl | - | - | 150 µl | 150 µl |
| B: H1N1 | H1N1 (100 µl) | - | 50 µl | 150 µl |
| C: H1N1 + OM-294-MP | H1N1 (100 µl) | OM-294-MP (50 µl) | - | 150 µl |
| D: H1N1 + OM-294-DP | H1N1 (100 µl) | OM-294-DP (50 µl) | - | 150 µl |
| E: OM-294-MP | - | OM-294-MP (50 µl) | 100 µl | 150 µl |
| F: OM-294-DP | - | OM-294-DP (50 µl) | 100 µl | 150 µl |

Antigène: la solution mère de H1N1 est préparée à la concentration de 25 µg/ml dans 0.9% NaCl.
Adjuvants: les solutions mères de OM-294-DP et OM-294-MP sont préparées à 1 mg/ml dans l'eau pour injection, additionnée de 0.1% triethanolamine pour l'OM-294-MP. Le témoin négatif est constitué d'une solution de 0.9% NaCl sans antigène.
Mélange antigène-adjuvant: les adjuvants sont placés 20 minutes à 37°C avant d'être vortexés pendant 3 minutes. Puis l'antigène et le NaCl (0.9%) sont ajoutés comme indiqué dans le tableau ci-dessus, et le mélange antigène/adjuvant est vortexé brièvement avant d'être mis sur un agitateur rotatif pendant 15 minutes à température ambiante, puis le tout est vortexé pendant 3 minutes
Immunisations : les injections ont lieu aux jours 0 et 14. Les mélanges indiqués dans le tableau précédent sont administrés par voie sous-cutanée (75 µl par flanc, au total 150 µl par animal). Les prises de sang ont lieu aux jours 14 et 28 (ponction orbitale).
Dosage des immunoglobulines anti-H1N1: les immunoglobulines sériques spécifiques pour H1N1 suivantes sont déterminées en duplicats par ELISA : IgG1, IgG2a, et IgM . En bref, des plaques micropuits (NUNC Immunoplate, Roskilde, DK) sont incubées (coating overnight) à 4°C avec 100 µl de H1N1 (0.5 µg) dans un tampon bicarbonate (pH 9.6). Après lavage avec 0.5% Tween-20 (Merck, Hohenbrunn, D), les sérums sont dilués 50, 200 et 800 fois (solution de dilution: phosphate buffered saline (PBS) + 1 % d'albumine sérique bovine (BSA, Sigma, St. Louis, Mo, U.S.A.) + 0.02% Tween-20)). 100 µl de chacun des sérums dilués sont ajoutés aux puits. Cette incubation dure 45 minutes à 37°C.

Après un second lavage, les IgG1, IgG2a et IgM spécifiques pour H1N1 sont incubées 30 minutes à 37°C avec 100 µl des anticorps (rat anti-souris) anti-IgG1 conjugués à la peroxydase (Serotec, Oxford, UK,) , IgG2a conjugués à la peroxydase (Pharmingen, San Diego, CA, U.S.A.) et IgM conjugués à la biotine (Pharmingen, San Diego, CA, U.S.A.), au préalable dilués dans le tampon PBS/BSA/Tween (dilutions : 250, 1000, et 500 fois respectivement). Pour les IgM, après un lavage supplémentaire, une 3^{ème} incubation est nécessaire (30 min à 37°C) avec une solution 1/100 de streptavidine conjuguée à la peroxidase (Dako, Glostrup, DK).
Après lavage, 100 µl d'une solution de phenylène 1,2-diamine (OPD, Merck, Darmstad, RFA) sont ajoutés pour détecter la peroxydase conjuguées aux anticorps secondaires anti-IgG1 et anti-IgG2a (tandis que pour les IgM, le réactif utilisé est le 3',3',5',5'-tétramethylbenzidine (TMB, Sigma, St. Louis, Mo, U.S.A.). Après une incubation de 20 minutes à température ambiante, la réaction est stoppée par l'ajout de 100 µl de 2N H₂SO₄. Les absorbances sont mesurées à 490 nm avec un lecteur de plaques Bio Rad 3550.

### Résultats

Les résultats de chaque mesure à 490 nm sont exprimés en Unités Arbitraires (U.A) par ml. Ceci est possible par comparaison de chaque échantillon avec une référence préparée à partir de dilutions d'un pool d'échantillons à 28 jours provenants du groupe B (animaux injectés avec H1N1 seul). Par définition le pool d'échantillons dilué 50 fois est à la concentration de 1000 U.A./ml. Les résultats individuels sont ensuite corrigés selon le facteur de dilution correspondant (50, 200, ou 800 fois). On ne rapporte ici que la moyenne de chaque groupe et la déviation standard (SD).

**Tab a) Immunoglobulines spécifiques contre H1N1 de la sous-classe IgG1 (Unités arbitraires/ml ± SD, ** P<0.01 (tests Anova + Dunnetts (two sided)).**

| GROUPES | Jour 14 | Jour 28 |
|---|---|---|
| A:NaCl | 3 ± 5 | 0 ± 0 |
| B : H1N1 | 11161 ± 5755 | 53950 ± 23403 |
| C : H1N1 + OM-294-MP | 34411** ± 13719 | 228467** ± 109123 |
| D : H1N1 + OM-294-DP | 30101** ± 19061 | 382325** ± 201314 |
| E : OM-294-MP | 69 ± 34 | 59 ± 31 |
| F: OM-294-DP | 59 ± 21 | 38 ± 25 |

**Tab b) Immunoglobulines spécifiques contre H1N1 de la sous-classe IgG2a (Unités arbitraires/ml ± SD, ** P<0.01 (tests Anova + Dunnetts test (two sided)).**

| GROUPES | Jour 14 | Jour 28 |
|---|---|---|
| A: NaCl | 0 ± 0 | 0 ± 0 |
| B : H1N1 | 26883 ± 20779 | 50352 ± 30846 |
| C : H1N1 + OM-294-MP | 179344** ± 139781 | 1622722** ± 986195 |
| D : H1N1 + OM-294-DP | 103630 ± 96257 | 681441 ± 1072710 |
| E : OM-294-MP | 1619 ± 743 | 1767 ± 1034 |
| F : OM-294-DP | 452 ± 584 | 782 ± 857 |

**Tab c) Immunoglobulines spécifiques contre H1N1 de la sous-classe IgM (Unités arbitraires/ml ± SD, * P<0.05 et **P<0.01 (Anova + Dunnetts test (two sided)).**

| GROUPES | Jour 14 | Jour 28 |
|---|---|---|
| A : NaCl | 22102 ± 5862 | 21531 ± 3693 |
| B : H1N1 | 37787 ± 15001 | 57306 ± 26886 |
| C : H1N1 + OM-294-MP | 67936** ± 21334 | 95108 ± 38669 |
| D : H1N1 + OM-294-DP | 598100* ± 18324 | 92920 ± 26971 |
| E: OM-294-MP | 19065 ± 4069 | 18018 ± 4016 |
| F: OM-294-DP | 20756 ± 7160 | 20944 ± 9065 |

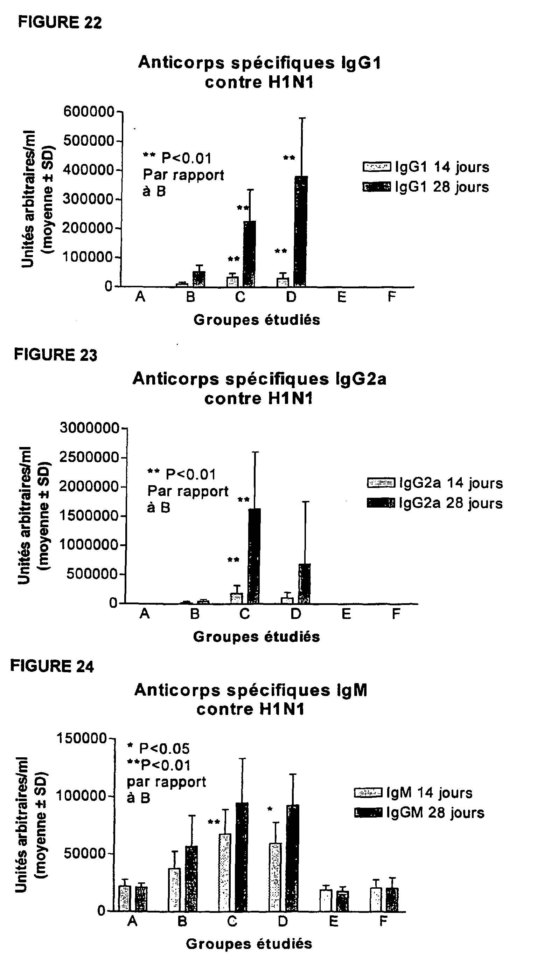

Ces résultats indiquent que les adjuvants OM-294-MP et OM-294-DP sont actifs dans le modèle étudié, puisqu'ils augmentent souvent de façon significative après une ou deux injections (voir figures 22, 23, 24) les anticorps spécifiques anti-H1N1 produits par la souris, ceci quelque soit la sous-classe d'immunoglobuline analysée (IgG1, IgG2a, et IgM).

### 13. OM-294-MP et OM-294-DP combinés avec l'antigène ovalbumine: détermination d'anticorps spécifiques générés chez la souris après 1 ou 2 administrations par voie sous-cutanée.

### Protocole

Le but de cette étude est de montrer l'effet adjuvant de OM-294-MP et OM-294-DP pour l'antigène ovalbumine (Fluka Chemie, Buchs, CH). Pour cela 50 souris BALB/c (femelles, 8 semaines d'âge au début du traitement) sont réparties dans les 5 groupes suivants :

| GROUPES | ANTIGENE Final : 50 µg par animal/injection | ADJUVANTS Final : 50 µg par animal/injection | NaCl | VOLUME INJECTE |
|---|---|---|---|---|
| A: NaCl | - | - | 150 µl | 150 µl |
| B: Ova | Ova (100 µl) | - | 50 µl | 150 µl |
| C: Ova + OM-294-MP | Ova (100 µl) | OM-294-MP (50 µl) | - | 150 µl |
| D: Ova + OM-294-DP | Ova (100 µl) | OM-294-DP (50 µl) | - | 150 µl |
| E: OM-294-MP | - | OM-294-MP (50 µl) | 100 µl | 150 µl |

Antigène : la solution mère d'ovalbumine est préparée à la concentration de 0.5 mg/ml dans 0.9% NaCl.
Adjuvants : les solutions mères de OM-294-DP et OM-294-MP sont préparées à 1 mg/ml dans de l'eau pour injection, additionnée de 0.1% triéthanolamine pour l'OM-294-MP. Le témoin négatif est constitué d'une solution de 0.9% NaCl sans antigène.
Mélange antigène-adjuvant : les adjuvants sont placés 20 minutes à 37°C avant d'être vortexés pendant 3 minutes. Puis l'antigène et le NaCl (0.9%) sont ajoutés comme indiqué dans le tableau ci-dessus, et le mélange antigène/adjuvant est vortexé brièvement avant d'être mis sur un agitateur rotatif pendant 15 minutes à température ambiante, puis le tout est vortexé pendant 3 minutes
Immunisations : les injections ont lieu aux jours 0 et 14. Les mélanges indiqués dans le tableau précédent sont administrés par voie sous-cutanée (75 µl par flanc, au total 150 µl par animal). Les prises de sang ont lieu aux jours 14 et 28 (ponction orbitale).

Dosage des immunoglobulines anti-ovalbumine : les immunoglobulines sériques suivantes spécifiques pour l'ovalbumine sont déterminées en duplicats par ELISA : IgG1, IgG2a, et IgM. En bref, des plaques de micropuits (NUNC Immunoplate, Roskilde, DK) sont incubées (*coating overnight*) à 4°C avec 100 µl de ovalbumine (0.5 µg) dans un tampon bicarbonate (pH 9.6). Après lavage avec 0.5% Tween-20 (Merck, Hohenbrunn, D) les sérums sont dilués 50, 200 et 800 fois (solution de dilution: phosphate buffered saline (PBS) + 1 % d'albumine sérique bovine (BSA, Sigma, St. Louis, MO, U.S.A.) + 0.02% Tween-20). 100 µl de chacun des sérums dilués sont ajoutés aux puits. Cette incubation dure 45 minutes à 37°C.
Après un second lavage, les IgG1, IgG2a et IgM spécifiques pour l'ovalbumine sont incubées 30 minutes à 37°C avec 100 µl des anticorps (rat anti-souris) anti-IgG1 conjugués à la peroxydase (Serotec, Oxford, UK), IgG2a conjugués à la peroxydase (Pharmingen, San Diego, CA, U.S.A.) et IgM conjugués à la biotine (Pharmingen, San Diego, CA, U.S.A.), au préalable dilués dans le tampon PBS/BSA/Tween (dilutions : 250, 1000, et 500 fois respectivement). Pour les IgM, après un lavage supplémentaire, une 3^{ème} incubation est nécessaire (30 min à 37°C) avec une solution 1/100 de streptavidine conjuguée à la peroxidase (Dako, Glostrup, DK).
Après lavage, 100 µl d'une solution de phenylène 1,2-diamine (OPD, Merck, Darmstad, RFA) sont ajoutés pour détecter la peroxydase conjuguées aux anticorps secondaires anti-IgG1 et anti-IgG2a (tandis que pour les IgM, le réactif utilisé est la 3',3',5',5'-tétramethylbenzidine (TMB, Sigma, St. Louis, MO, U.S.A.). Après une incubation de 20 minutes à température ambiante (40 minutes pour le TMB), la réaction est stoppée par l'ajout de 100 µl de 2N H₂SO₄. Les absorbances sont mesurées à 490 nm avec un lecteur de plaques Bio Rad 3550.

### Résultats

Les résultats de chaque mesure à 490 nm sont exprimés en Unités Arbitraires (U.A.) par ml. Ceci est possible par comparaison de chaque échantillon avec une référence préparée à partir de dilutions d'un pool d'échantillons à 28 jours provenants du groupe B (animaux injectés avec ovalbumine seule). Par définition le pool d'échantillons dilué 50 fois est à la concentration de 1000 U.A./ml. Les résultats individuels sont ensuite corrigés selon le facteur de dilution correspondant (50, 200, ou 800 fois). Nous ne rapportons ici que la moyenne de chaque groupe et la déviation standard (SD).

**Tab (a) Immunoglobulines spécifiques contre ovalbumine de la sous-classe IgG1 (Unités arbitraires/ml ± SD, ** P<0.01 (test Anova + Dunnetts (two sided)).**

| GROUPES | Jour 14 | Jour 28 |
|---|---|---|
| A : NaCl | 6 ± 6 | 16 ± 12 |
| B : Ova | 728 ± 589 | 47743 ± 46294 |
| C : Ova + OM-294-MP | 4361** ± 2513 | 284121** ± 164822 |
| D : Ova + OM-294-DP | 3240** ± 1794 | 277025** ± 173737 |
| E : OM-294-MP | 19 ± 8 | 40 ± 69 |

**Tab (b) Immunoglobulines spécifiques contre ovalbumine de la sous-classe IgG2a (Unités arbitraires/ml ± SD, * P<0.05 et ** P<0.01 (test Anova + Dunnetts (two sided)).**

| GROUPES | Jour 14 | Jour 28 |
|---|---|---|
| A : NaCl | 2996 ± 898 | 5414 ± 1554 |
| B : Ova | 5201 ± 1880 | 73162 ± 107954 |
| C : Ova + OM-294-MP | 9524 ± 6809 | 625663* ± 681232 |
| D : Ova + OM-294-DP | 18108** ± 14958 | 601434* ± 624166 |
| E : OM-294-MP | 11253 ± 12169 | 4192 ± 2104 |

**Tab (c) Immunoglobulines Spécifiques contre ovalbumine de la sous-classe IgM (Unités arbitraires/ml ± SD).**

| GROUPES | Jour 14 | Jour 28 |
|---|---|---|
| A : NaCl | 14009 ± 6158 | 12288 ± 7136 |
| B : Ova | 19423 ± 13778 | 47998 ± 34035 |
| C : Ova + OM-294-MP | 21652 ± 9524 | 38240 ± 8822 |
| D : Ova + OM-294-DP | 25762 ± 10975 | 74399 ± 119781 |
| E : OM-294-MP | 19742 ± 5667 | 9827 ± 2021 |

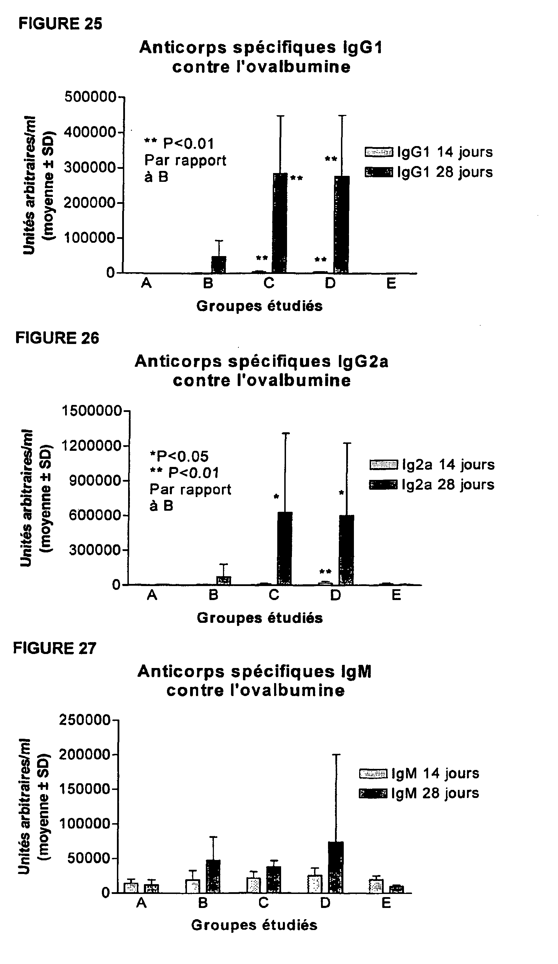

Ces résultats indiquent que les adjuvants OM-294-MP et OM-294-DP sont actifs dans le modèle étudié, puisqu'ils augmentent significativement (pour les sous-classes IgG1 et IgG2a) après 1 ou 2 injections (voir figures 25, 26, 27) les anticorps spécifiques anti-ovalbumine produits par la souris.

### 14. OM-294-MP et OM-294-DP combinés avec l'antigène TT (Tetanus Toxoid) : détermination d'anticorps spécifiques générés chez la souris après 1 ou 2 administrations par voie sous-cutanée.

### Protocole

Le but de cette étude est de montrer l'effet adjuvant de OM-294-MP et de OM-294-DP pour l'antigène TT (Massachusetts Biologic Laboratories, MA, U.S.A.). Pour cela 40 souris BALB/c (femelles, 8 semaines d'âge au début du traitement) sont réparties dans les 4 groupes suivants :

| GROUPES | ANTIGENE Final : 20 µg par animal/injection | ADJUVANTS Final : 50 µg par animal/injection | NaCl (0.9%) | VOLUME INJECTE |
|---|---|---|---|---|
| A: NaCl | - | - | 150 µl | 150 µl |
| B: TT | TT (100 µl) | - | 50 µl | 150 µl |
| C: TT + OM-294-MP | TT (100 µl) | OM-294-MP (50 µl) | - | 150 µl |
| D: TT + OM-294-DP | TT (100 µl) | OM-294-DP (50 µl) | - | 150 µl |

Antigène : la solution mère de TT est préparée à la concentration de 0.2 mg/ml dans 0.9% NaCl.
Adjuvants : les solutions mères de OM-294-DP et de OM-294-MP sont préparées à 1 mg/ml dans de l'eau pour injection, additionnée de 0.1% triethanolamine pour l'OM-294-MP. Le témoin négatif est constitué d'une solution de 0.9% NaCl sans antigène.
Mélange antigène-adjuvant : les adjuvants sont placés 20 minutes à 37°C avant d'être vortexés pendant 3 minutes. Puis l'antigène et le NaCl (0.9%) sont ajoutés comme indiqué dans le tableau ci-dessus, et le mélange antigène/adjuvant est vortexé brièvement avant d'être mis sur un agitateur rotatif pendant 15 minutes à température ambiante, puis le tout est vortexé pendant 3 minutes
Immunisations : les injections ont eu lieu aux jours 0 et 14. Les mélanges indiqués dans le tableau précédent sont administrés par voie sous-cutanée (75 µl par flanc, au total 150 µl par animal). Les prises de sang ont eu lieu aux jours 14 et 28 (ponction orbitale).
Dosage des immunoglobulines anti-TT : les immunoglobulines sériques spécifiques pour TT suivantes sont déterminées en duplicats par ELISA : IgG1, IgG2a, et IgM. Des plaques micropuits (NUNC Immunoplate, Roskilde, DK) sont incubées (coating overnight) à 4°C avec 100 µl de TT (0.5 µg) dans un tampon bicarbonate (pH 9.6). Après lavage avec 0.5% Tween-20 (Merck, Hohenbrunn, RFA) les sérums sont dilués 50, 200 et 800 fois (solution de dilution: phosphate buffered saline (PBS) + 1 % d'albumine sérique bovine (BSA, Sigma, St. Louis, MO, U.S.A.) + 0.02% Tween-20)). 100 µl de chacun des sérums dilués sont ajoutés aux puits. Cette incubation dure 45 minutes à 37°C.
Après un second lavage, les IgG1, IgG2a et IgM spécifiques pour TT sont incubées 30 minutes à 37°C avec 100 µl des anticorps (rat anti-souris) anti-IgG1 conjugués à la peroxydase (Serotec, Oxford, UK,) , IgG2a conjugués à la peroxydase (Pharmingen, San Diego, CA, U.S.A.) et IgM conjugués à la biotine (Pharmingen, San Diego, CA, U.S.A.), au préalable dilués dans le tampon PBS/BSA/Tween (dilutions : 250, 1000, et 500 fois respectivement). Pour les IgM, après un lavage supplémentaire, une 3^{ème} incubation est nécessaire (30 min à 37°C) avec une solution 1/100 de streptavidine conjuguée à la peroxidase (Dako, Glostrup, DK).
Après lavage, 100 µl d'une solution de phenylène 1,2-diamine (OPD, Merck, Darmstad, RFA) sont ajoutés pour détecter la peroxydase conjuguées aux anticorps secondaires anti-IgG1 et anti-IgG2a (tandis que pour les IgM, le réactif utilisé est la 3',3',5',5'-tétramethylbenzidine (TMB, Sigma, St. Louis, Mo)). Après une incubation de 20 minutes à température ambiante (40 minutes pour le TMB), la réaction est stoppée par ajout de 100 µl de 2N H₂SO₄. Les absorbances sont mesurées à 490 nm avec un lecteur de plaques Bio Rad 3550.

### Résultats

Les résultats de chaque mesure pour le immunoglobulines IgG1 et IgG2a à 490 nm sont exprimés en Unités Arbitraires (U.A) par ml. Ceci est possible par comparaison de chaque échantillon avec une référence préparée à partir de dilutions d'un pool d'échantillons à 28 jours provenant du groupe B (animaux injectés avec TT seule). Par définition le pool d'échantillons dilué 50 fois est à la concentration de 1000 U.A./ml. Les résultats individuels sont corrigés selon le facteur de dilution correspondant (50, 200, ou 800 fois). On ne rapporte ici que la moyenne de chaque groupe et la déviation standard (SD).
En ce qui concerne la détermination des IgM spécifiques pour TT, comme le « bruit de fond » de la mesure est trop élevé, aucune différence claire entre le groupe B (TT seule) et les groupes C et D (TT avec adjuvants) ne peut être décelée en mesurant les IgM spécifiques de la même manière que les IgG1 et les IgG2a. Par contre, on a pu mesurer le titre des IgM spécifiques, en utilisant des dilutions successives de chaque échantillon (plutôt que les U.A. décrites précédemment), et on a pris en considération, pour chaque échantillon, la dilution maximale qui donne une absorbance supérieure à la moyenne + 3 SD des absorbances du groupe A (NaCl). Le titre ainsi obtenu indique le nombre de fois qu'un échantillon de sérum peut être dilué avant que l'absorbance obtenue à 490 nm ne se confonde avec le bruit de fond. C'est cette dilution qui est rapportée dans le Tab (c) IgM ci-après.

**Tab (a) Immunoglobulines spécifiques contre TT de la sous-classe IqG1 (Unités arbitraires/ml ± SD, ** P<0.01 (Anova + Dunnetts test (two sided)).**

| GROUPES | Jour 14 | Jour 28 |
|---|---|---|
| A: NaCl | 1 ± 2 | 0 ± 1 |
| B : TT | 2871 ± 1633 | 34367 ± 15018 |
| C : TT + OM-294-MP | 8502** ± 2020 | 78506** ± 21660 |
| D : TT + OM-294-DP | 11620** ± 2348 | 136463** ± 41025 |

**Tab (b) Immunoglobulines spécifiques contre TT de la sous-classe IgG2a (Unités arbitraires/ml ± SD, ** P<0.01 (Anova + Dunnetts test (two sided)).**

| GROUPES | Jour 14 | Jour 28 |
|---|---|---|
| A : NaCl | 351 ± 506 | 539 ± 1046 |
| B : TT | 2547 ± 2539 | 61387 ± 82269 |
| C : TT + OM-294-MP | 8869 ± 6979 | 65881 ± 46635 |
| D : TT + OM-294-DP | 21969** ± 25067 | 148365 ± 134196 |

**Tab (c) Titre des immunoglobulines spécifiques contre TT de la sous-classe IgM (dilutions pour chaque échantillon donnant un signal à 490 nm supérieur à celui de la moyenne + 3 SD de celui du groupe A ).**

| GROUPES | Jour 14 | Jour 28 |
|---|---|---|
| A: NaCl | Référence | Référence |
| B : TT | 9 animaux <25 | 10 animaux<25 |
| | 1 animal <50 | |
| C : TT + OM-294-MP | 9 animaux <25 | 7 animaux <25 |
| | 1 animal >1600 | 1 animal <100 |
| | | 2 animaux >1600 |
| D : TT + OM-294-DP | 7 animaux <25 | 10 animaux<25 |
| | 1 animal <200 | |
| | 2 animaux >1600 | |

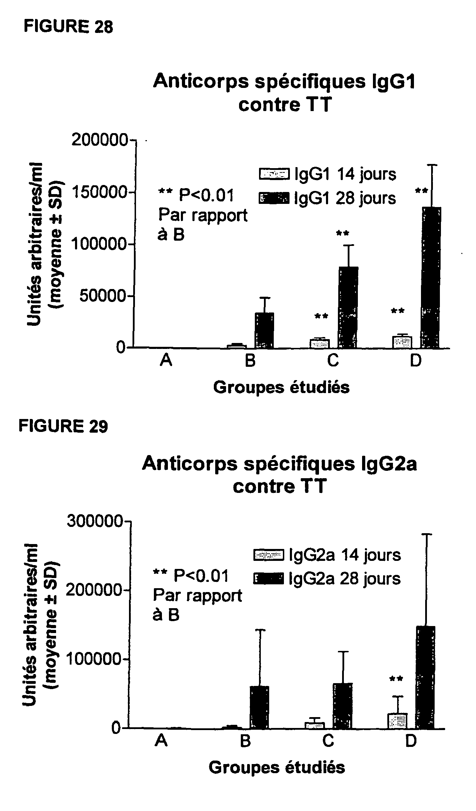

Ces résultats indiquent que les adjuvants OM-294-MP et OM-294-DP sont actifs dans le modèle étudié, puisqu'ils augmentent souvent de façon significative après 1 ou 2 injections (voir figures 28, 29) les anticorps spécifiques IgG1 et IgG2a anti-TT, produits par la souris. Par contre seuls quelques animaux ont produit des IgM spécifiques contre TT.

### 15. Evaluation des propriétés de l'adjuvant OM-294-MP dans un modèle d'immunisation chez la souris CBA par voie s.c. avec l'antigène de Leishmania gp63.

### Protocole

Des souris CBA reçoivent dans la queue à 8 jours d'intervalle deux injections sous-cutanées de 2 µg de gp63. L'adjuvant OM-294-MP est mélangé avec les deux doses d'antigène, le BCG est mélangé seulement avec la première. Chaque souris reçoit 2 x 50 µg d'OM-294-MP ou 200 µg de BCG. Un groupe témoin est injecté avec l'antigène seul (sans adjuvant). Dix jours après la deuxième injection, les cellules des ganglions lymphatiques inguinaux et périaortiques (groupes de 3 souris) sont mis en culture et la réponse proliférative à l'antigène purifié gp63 évaluée par mesure de l'incorporation de thymidine (³H-TdR). La production des cytokines IFN-γ et IL-4 in vitro par les lymphocytes ganglionnaires restimulés in vitro par l'antigène de gp63 a également été déterminée par ELISA (kits IFN-γ MIF00 et IL-4 M4000, R&D Systems Europe LtD, Abingdon, UK) sur un prélèvement de chacun des surnageants des cultures des lymphocytes ganglionnaires avant l'ajout de ³H-TdR.
Les valeurs reportées dans les tableaux représentent pour l'incorporation de ³H-TdR, la moyenne arithmétique ± la déviation standard (triplicats) exprimée en cpm et pour les cytokines dans les surnageants, la moyenne arithmétique ± la déviation standard (triplicats) exprimée en pg par ml.
Antigène : la solution mère de gp63 est préparée à la concentration de 40 µg/ml dans 0.9% NaCl.
Adjuvants : la solution mère de OM-294- MP est préparée à 1 mg/ml dans de l'eau pour injection additionnée de 0.1% triethanolamine. Le contrôle négatif est constitué d'une solution de PBS sans antigène.
Mélange antigène-adjuvant : les adjuvants sont placés 10 minutes à 37°C avant d'être vortexés pendant 3 minutes. Puis l'antigène (1 volume) et le l'adjuvant (1 volume) sont mélangés et vortexés brièvement avant d'être mis à incuber pendant 20 minutes à 37°C, puis le tout est vortexé pendant 3 minutes

### Résultats

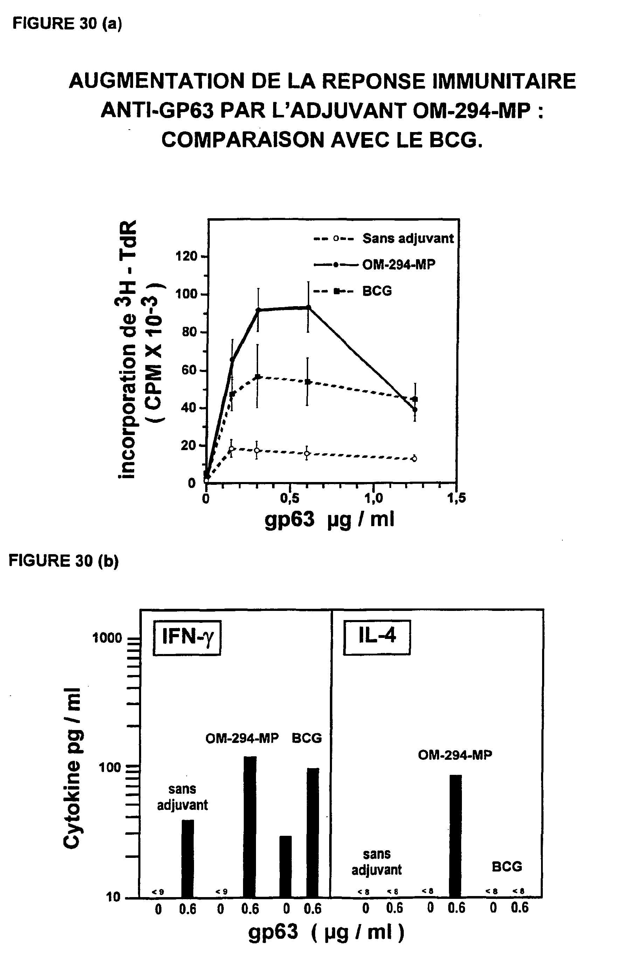

Chez des souris immunisées avec l'antigène gp63, l'adjuvant OM-294-MP induit une meilleure réponse de prolifération lymphocytaire (Tab (a) et Fig 30 (a)) que le BCG. En effet, par rapport aux cultures provenant d'animaux immunisés sans adjuvant, l'augmentation de la prolifération est comprise entre 3.1 et 6 pour le produit OM-294-MP alors qu'elle ne dépasse pas 3.5 dans le cas du BCG (2.6 à 3.5).
Dans ces mêmes cultures lymphocytaires, la production de cytokines est mesurée dans le surnageant (Tab (b) et Fig 30 (b)). Il est observé que l'antigène gp63 induit la sécrétion d'IFN-γ en quantités équivalentes que les souris sont traitées par l'adjuvant OM-294-MP ou par le BCG. Par contre, l'adjuvant OM-294-MP semble prédisposer les lymphocytes immuns (anti-gp63) à sécréter des quantités importantes d'IL-4, alors que les lymphocytes de souris traitées par le BCG sécrètent des quantités faibles, voire indétectables de cette cytokine.

**Tableau (a). Effet de l'adjuvant OM-294-MP sur la réponse immunitaire contre l'antigène gp63, mesurée par la prolifération de lymphocytes T murins en réponse à l'antigène gp63 in vitro.**

| gp63 in vitro (µg/ml) | Sans adjuvant (cpm x 10⁻³/ml) | OM-294-MP (cpm x 10⁻³/ml) | BCG (cpm x 10⁻³/ml) |
|---|---|---|---|
| 0 | 1.4 ± 0.4 | 2.8 ± 0.7 | 5.3 ± 1.1 |
| 0.16 | 18 ± 5 | 65 ± 11 | 47 ± 9 |
| 0.31 | 17 ± 5 | 92 ± 11 | 57 ± 17 |
| 0.62 | 16 ± 4 | 93 ± 13 | 54 ± 13 |
| 1.25 | 13 ± 2 | 39 ± 7 | 44 ± 9 |

Les valeurs reportées dans le Tab (a) représentent la moyenne arithmétique ± la déviation standard de l'incorporation (cultures en triplicats).

**Tableau (b). Effet de l'adjuvant OM-294-MP administré in vivo avec l'antigène gp63 sur la production in vitro de cytokines par les lymphocytes ganglionnaires.**

| Concentration de IFN-γ (pg/ml) | | | |
|---|---|---|---|
| gp63 µg/ml | Sans adjuvant | 294-MP | BCG |
| 0 | < 9 | < 9 | 27 |
| 0.3 | 78 | 135 | 200 |
| 0.6 | 38 | 120 | 105 |

| Concentration de IL-4 (pg/ml) | | | |
|---|---|---|---|
| gp63 µg/ml | Sans adjuvant | 294-MP | BCG |
| 0 | <8 | <8 | <8 |
| 0.3 | < 15 | 125 | 15 |
| 0.6 | < 8 | 83 | < 8 |

L'adjuvant OM-294-MP potentialise chez des souris CBA immunisées avec gp63 (un antigène amphiphile du parasite *Leishmania*) une réponse T spécifique évaluée *in vitro* par la mesure de la prolifération lymphocytaire et de la production d'IFN-γ et d'IL-4 induite par l'antigène.

### 16. Efficacité de l'adjuvant OM-294-MP au cours de la réponse primaire T anti-LmCPb dans un modèle d'immunisation chez la souris CBA par voie s.c. avec l'antigène de Leishmania mexicana LmCPb.

### Protocole

Des souris CBA ont reçu dans la queue une seule injection de 2 µg de LmCPb avec ou sans 50 µg de l'adjuvant OM-294-MP. Un groupe contrôle a reçu une injection de tampon physiologique (non immunisé). Onze jours plus tard, les cellules des ganglions lymphatiques inguinaux et périaortiques (groupes de 3 souris) sont mises en culture et la réponse proliférative à l'antigène purifié LmCPb, à une préparation totale d'amastigotes de *Leishmania mexicana* et à la concanavaline A (Con A) est évaluée par mesure de l'incorporation de thymidine tritiée (³H-TdR). La production des cytokines IFN-γ et IL-4 par les lymphocytes ganglionnaires restimulés in vitro par l'antigène LmCPb de *Leishmania mexicana* ou par les amastygotes est également déterminée par ELISA (kits IFN-γ MIF00 et IL-4 M4000, R&D Systems Europe LtD, Abingdon, UK) sur un prélèvement de chacun des surnageants des cultures des lymphocytes ganglionnaires avant l'ajout de ³H-TdR.
Les valeurs reportées dans les tableaux représentent pour l'incorporation de ³H-TdR, la moyenne arithmétique ± la déviation standard (triplicats) exprimée en cpm et pour les cytokines dans les surnageants, la moyenne arithmétique ± la déviation standard (triplicats) exprimée en pg par ml.
Antigène : la solution mère de LmCPb est préparée à la concentration de 40 µg/ml dans le PBS deux fois concentré.
Adjuvants : la solution mère d'OM-294- MP est préparée à 1 mg/ml dans de l'eau pour injection additionnée de 0.1% triethanolamine. Le contrôle négatif est constitué d'une solution de PBS sans antigène.
Mélange antigène-adjuvant : les adjuvants sont mis 10 minutes à 37°C avant d'être vortexés pendant 3 minutes. Puis l'antigène (1 volume) et l'adjuvant (1 volume) sont mélangés et vortexés brièvement avant d'être mis à incuber pendant 20 minutes à 37°C, puis le tout est vortexé pendant 3 minutes

### Résultats

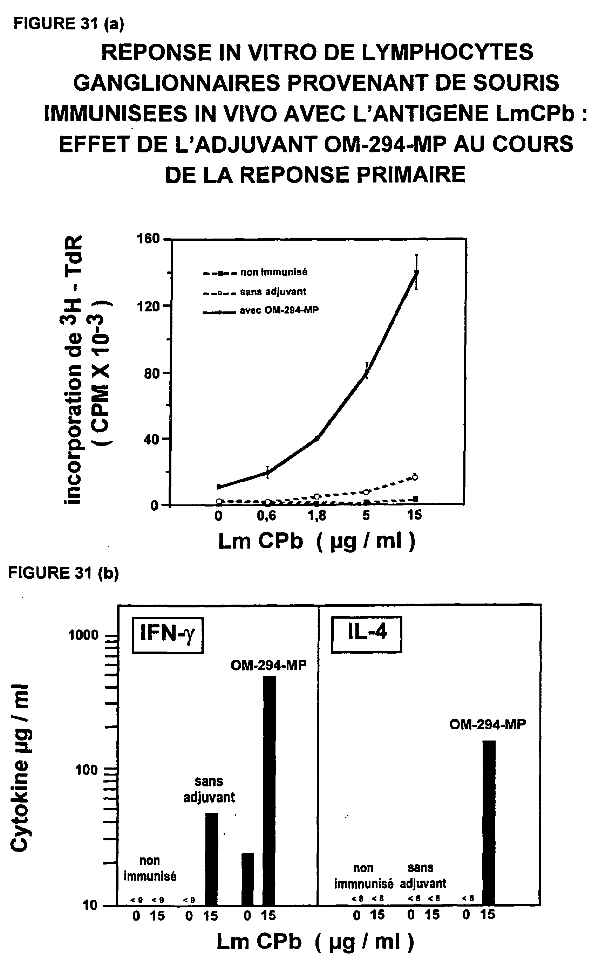

En l'absence de tout stimulant ajouté au milieu de culture, l'adjuvant OM-294-MP favorise le développement de lymphocytes proliférant spontanément (Tab (a) et Fig 31 (a)) et sécrétant des traces d'INF-γ (Tab (b) et Fig 31 (b)). Cette réponse est fortement potentialisée lorsque l'antigène purifié LmCPb ou un extrait total de parasites est ajouté aux cultures. Dans cette expérience, il y influence nette de l'adjuvant sur l'induction de lymphocytes sensibilisés (anti-LmCPb) capables de sécréter des quantités importantes d'IL-4 (Tab. (b) et Fig 31 (b)).

**Tableau (a). Réponse proliférative in vitro de cellules ganglionnaires immunisées in vivo avec LmCPb : effet de l'adjuvant OM-294-MP**

| | Incorporation de ³H-TdR (cpmx10⁻³/ml) | | |
|---|---|---|---|
| Antigène *in vitro* | Non immunisé | Sans Adjuvant | OM-294-MP |
| Pas de stimulant | 0.9 ± 0.3 | 2.2 ± 0.7 | 11 ± 2 |
| LmCPb 0.6 µg/ml | 0.8 ± 0.4 | 1.7 ± 0.1 | 19 ± 4 |
| LmCPb 1.7 µg/ml | 0.9 ± 0.1 | 4.6 ± 1.6 | 40 ± 4 |
| LmCPb 5 µg/ml | 1.3 ± 0.8 | 7.2 ± 0.6 | 80 ± 5 |
| LmCPb 15 µg/ml | 2.6 ± 0.4 | 16.2 ± 2.1 | 140 ± 10 |
| Amastigotes 1.9 x 10⁻⁶/ml | 0.8 ± 0.2 | 1.7 ± 0.1 | 44 ± 7 |
| Amastigotes 6 x 10⁻⁶/ml | 1.5 ± 0.2 | 4.6 ± 0.6 | 79 ± 6 |
| Amastigotes 17 x 10⁻⁶/ml | 2.9 ± 0.6 | 7.2 ± 0.6 | 119 ± 4 |
| Con A 5 µg/ml | 123 ± 33 | 193 ± 17 | 196 ± 10 |

**Tableau (b). Sécrétion in vitro de cytokines par les lymphocytes ganglionnaires immunisés in vivo avec LmCPb : Effet de l'adjuvant OM-294-MP sur la réponse primaire.**

| Production de IFN-γ (pg/ml) | | | |
|---|---|---|---|
| Stimulant *in vitro* | Non immunisé | Sans adjuvant | OM-294-MP |
| Pas de stimulant | < 9 | < 9 | 25 |
| LmCPb 15 µg/ml | < 9 | 46 | 480 |
| Amastigotes 17 x 10⁻⁶/ml | < 9 | 95 | 320 |
| Con A 5 µg/ml | > 1800 | > 1800 | > 1800 |

| Production de IL-4 (pg/ml) | | | |
|---|---|---|---|
| Stimulant *in vitro* | Non immunisé | Sans adjuvant | OM-294-MP |
| Pas de stimulant | < 8 | < 8 | < 8 |
| LmCPb 15 µg/ml | < 8 | < 8 | 130 |
| Amastigotes 17 x 10⁻⁶/ml | < 8 | < 8 | 65 |
| Con A 5 µg/ml | 92 | 190 | 360 |

L'adjuvant OM-294-MP est aussi très efficace au cours de la réponse T primaire (suite à une seule injection vaccinale). Les caractéristiques de l'effet adjuvant sur cette réponse (augmentation de la prolifération lymphocytaire, induction de cytokines) sont similaires à celles observées lors de la réponse à deux injections vaccinales.

### 17. Evaluation des propriétés des adjuvants OM-294-MP et OM-294-DP dans un modèle d'immunisation chez la souris CBA par voie s.c. avec l'antigène de Leishmania mexicana LmCPb: comparaison avec le BCG

### Protocole

Des souris CBA (8 souris par groupe) ont reçu dans la queue à 8 jours d'intervalle deux injections sous-cutanées de 3 à 5 µg de LmCPb purifié. Les adjuvants OM-294-DP, OM-294-MP sont mélangés avec les deux doses d'antigène, alors que le BCG a été mélangé seulement avec la première. Chaque souris a reçu 2 x 50 µg d'adjuvant OM ou 200 µg de BCG. Huit jours après la deuxième injection les ganglions lymphatiques inguinaux et périaortiques (3 souris par groupe) sont prélevés et les cellules mises en culture pour déterminer la réponse proliférative à l'antigène purifié LmCPb, ou respectivement, la réponse proliférative à une préparation totale d'amastigotes *Leishmania mexicana* ou à la concanavaline A (Con A). La réponse protiférative est évaluée par mesure de l'incorporation de thymidine tritiée (³H-TdR). La production des cytokines IFN-γ et IL-4 par les lymphocytes ganglionnaires restimulés in vitro par l'antigène LmCPb de *Leishmania mexicana* ou par les amastygotes ou par la Con A est déterminée par ELISA (kits IFN-γ MIF00 et IL-4 M4000, R&D Systems Europe LtD, Abingdon, UK) sur un prélèvement de chacun des surnageants des cultures des lymphocytes ganglionnaires avant l'ajout de ³H-TdR.
Les valeurs reportées dans les tableaux représentent pour les titres d'anticorps, la moyenne arithmétique ± la déviation standard exprimés en % du standard, pour l'incorporation de ³H-TdR, la moyenne arithmétique ± la déviation standard (triplicats) exprimée en cpm et pour les cytokines dans les surnageants, la moyenne arithmétique ± la déviation standard (triplicats) exprimée en pg par ml.
Antigène : la solution mère de LmCPb est préparée à une concentration comprise entre 60 et 100 µg/ml dans 0.9% NaCl.
Adjuvants : les solutions mères de OM-294-DP et OM-294-MP sont préparées à 1 mg/ml dans de l'eau pour injection, additionnée de 0.1% triethanolamine pour l'OM-294-MP. Le témoin négatif est constitué d'une solution de PBS sans antigène.
Mélange antigène-adjuvant : les adjuvants sont placés 10 minutes à 37°C avant d'être vortexés pendant 3 minutes. Puis l'antigène (1 volume) et l'adjuvant 0.9% (1 volume) sont mélangés et vortexés brièvement avant d'être mis à incuber pendant 20 minutes à 37°C, puis le tout est vortexé pendant 3 minutes

### Résultats

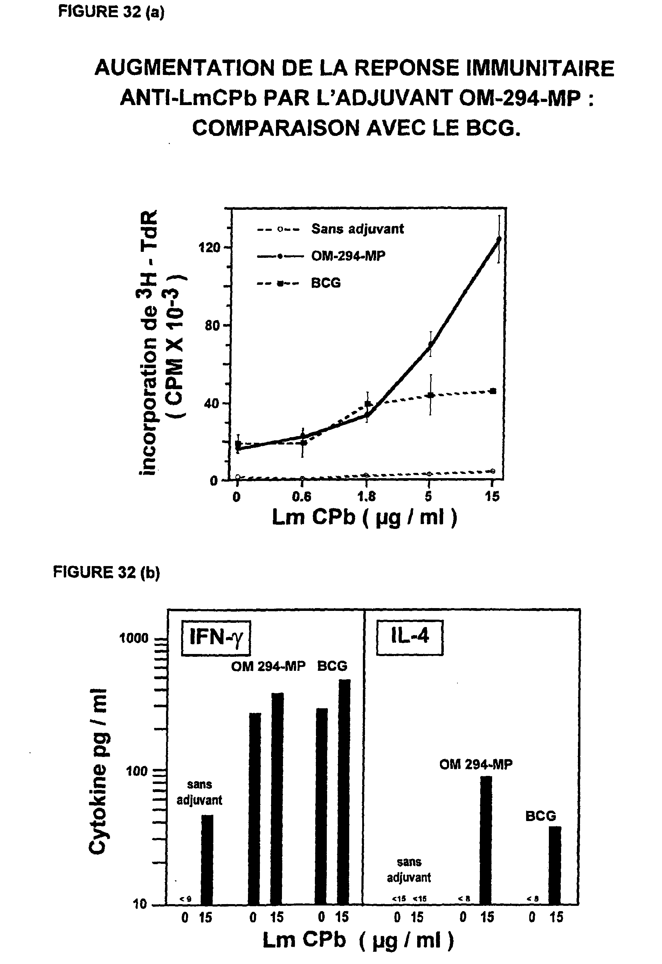

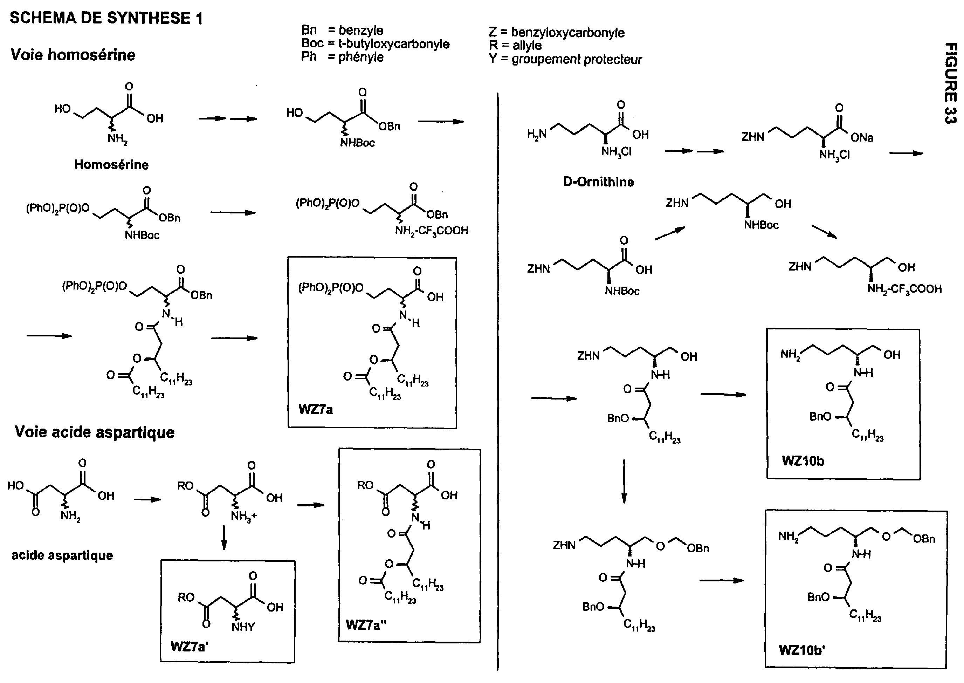

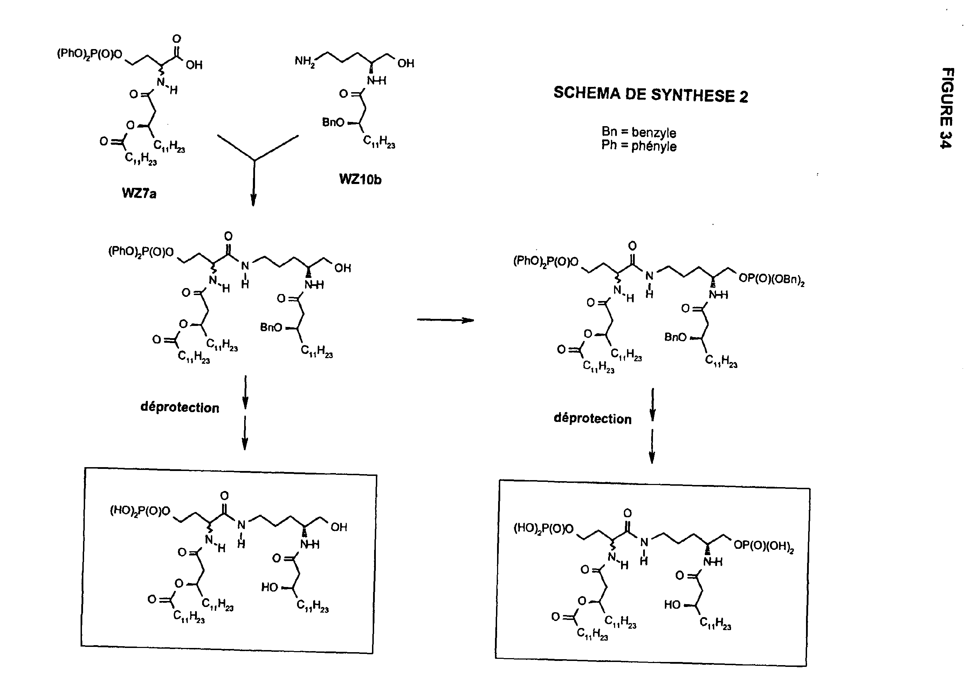

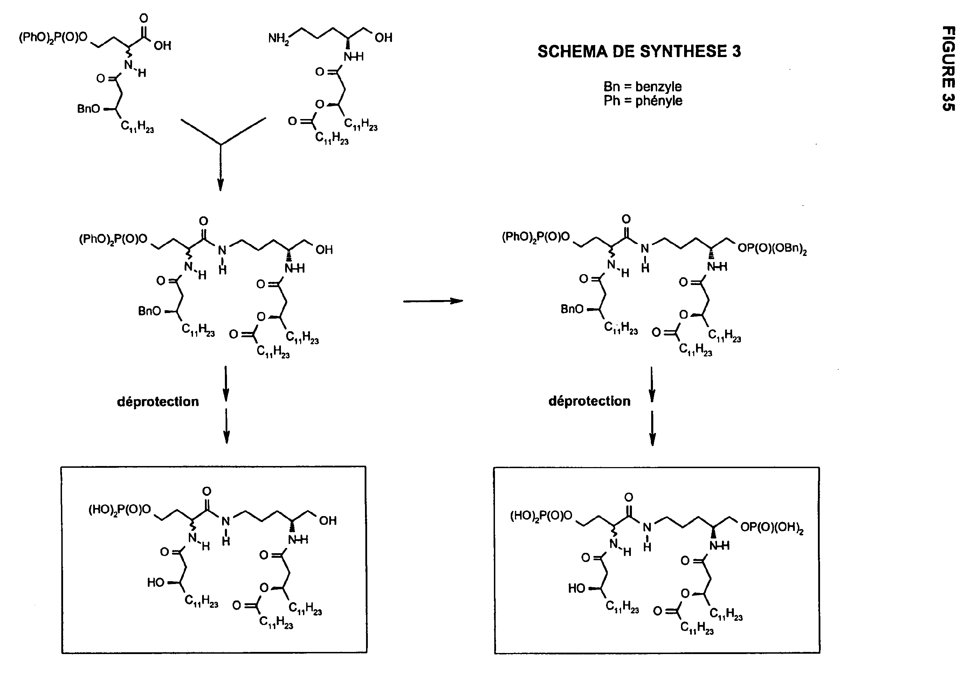

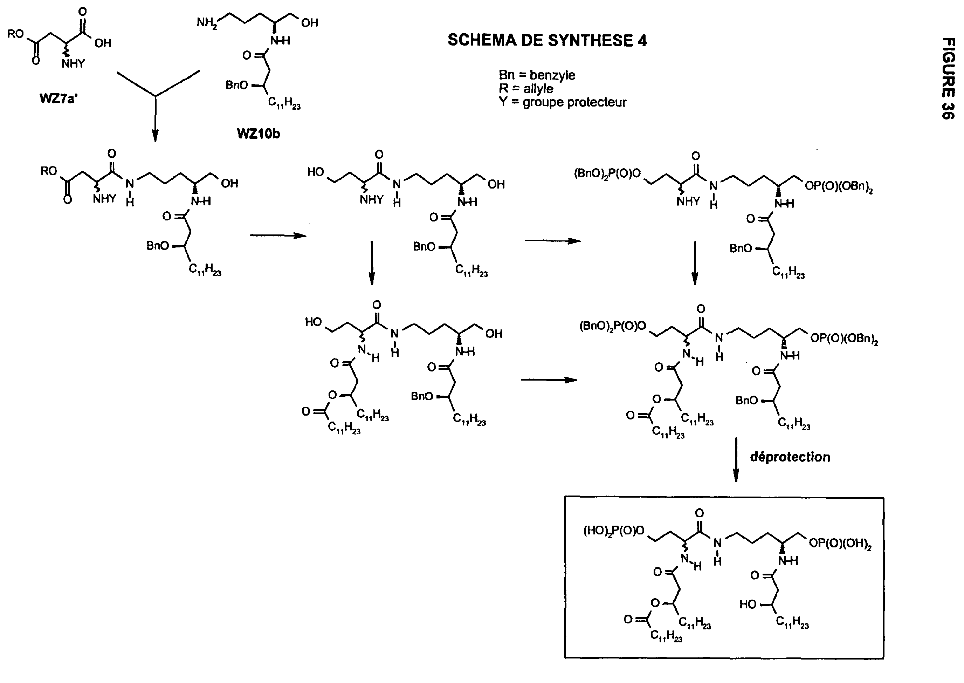

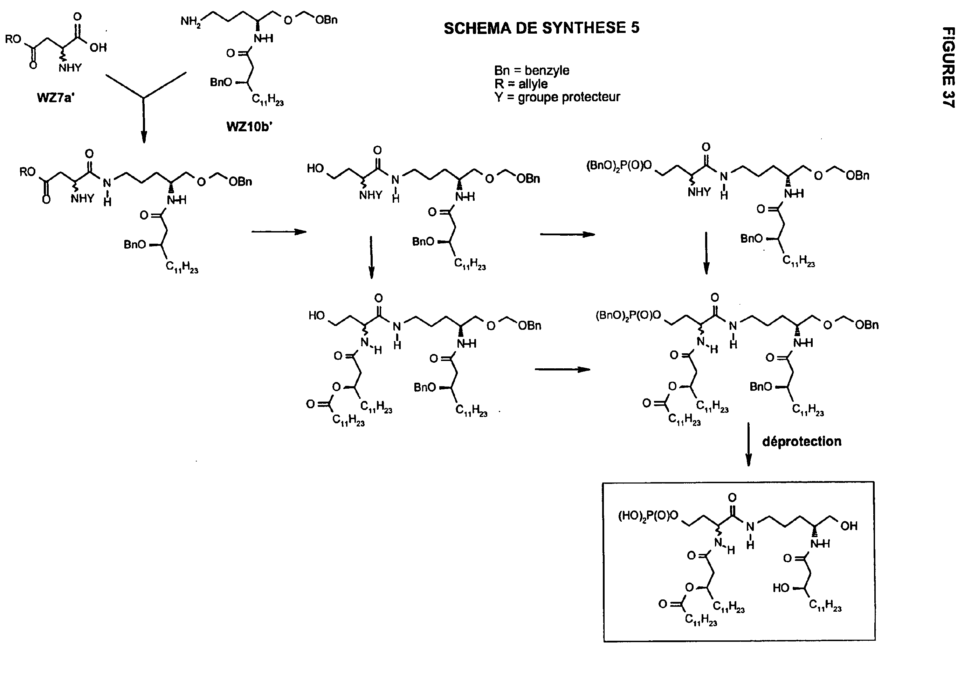

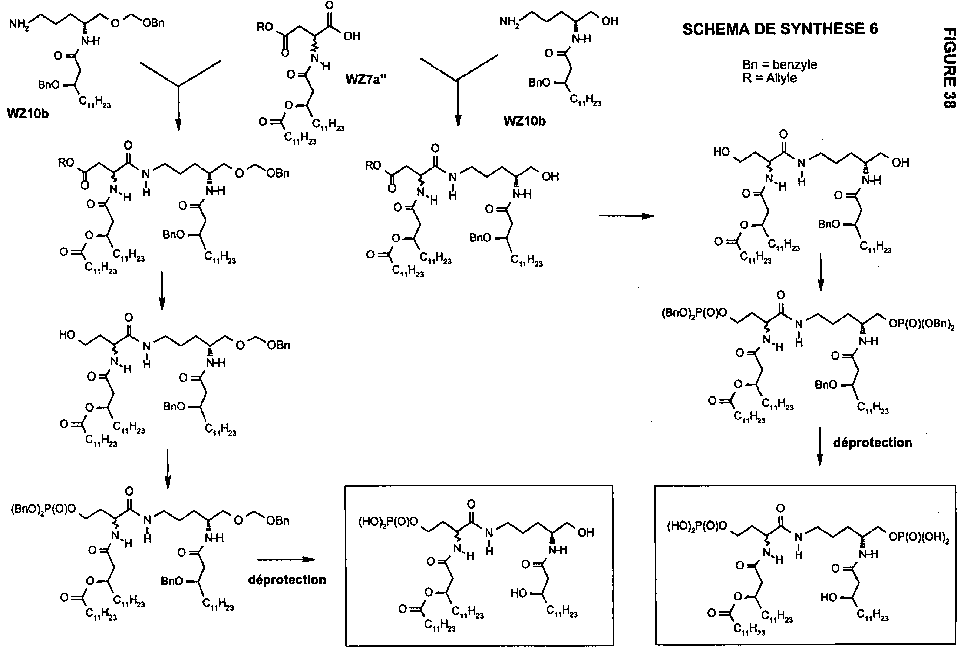

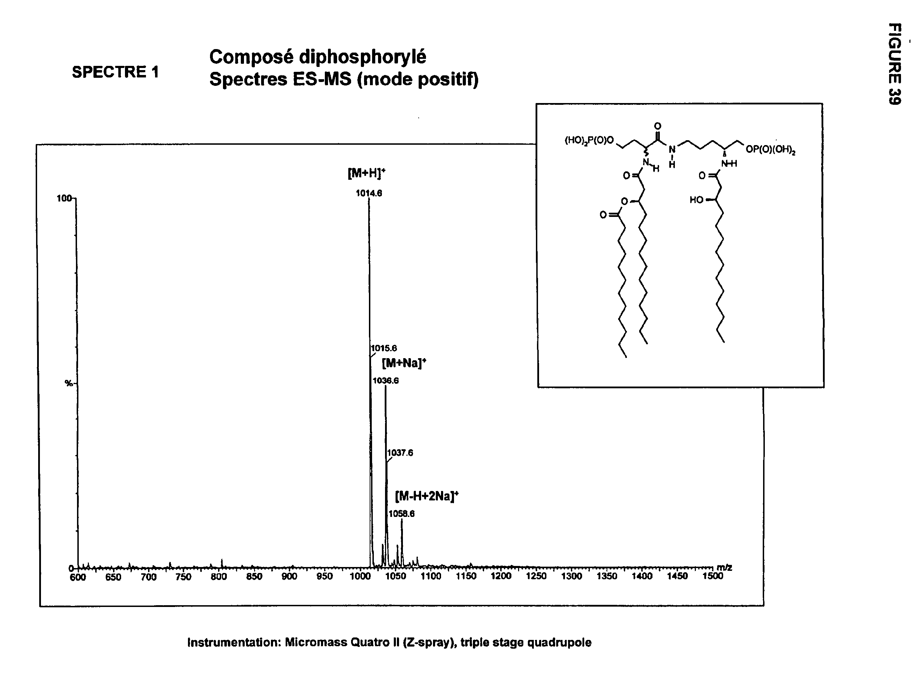

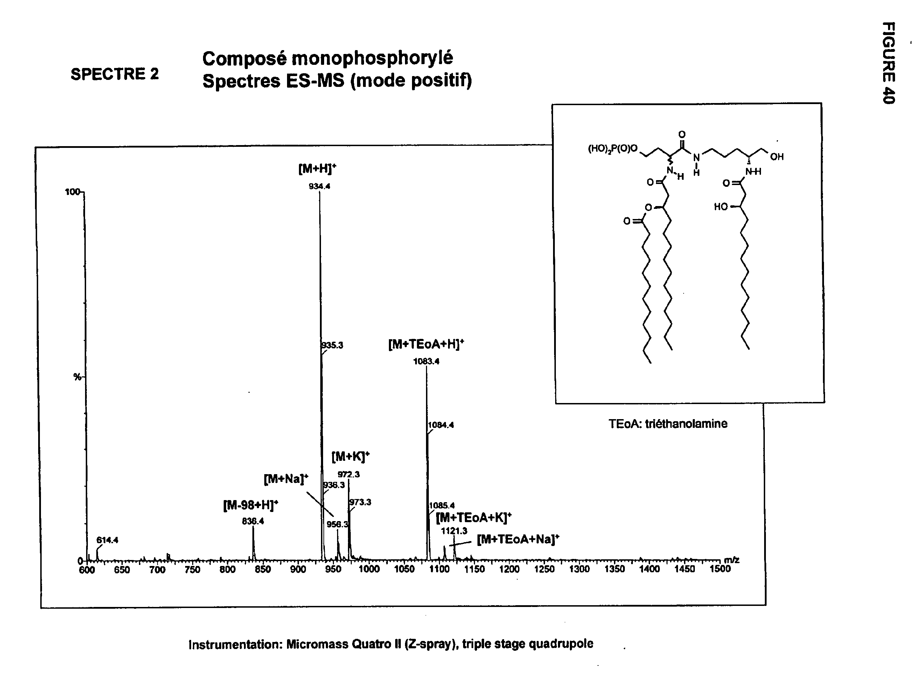

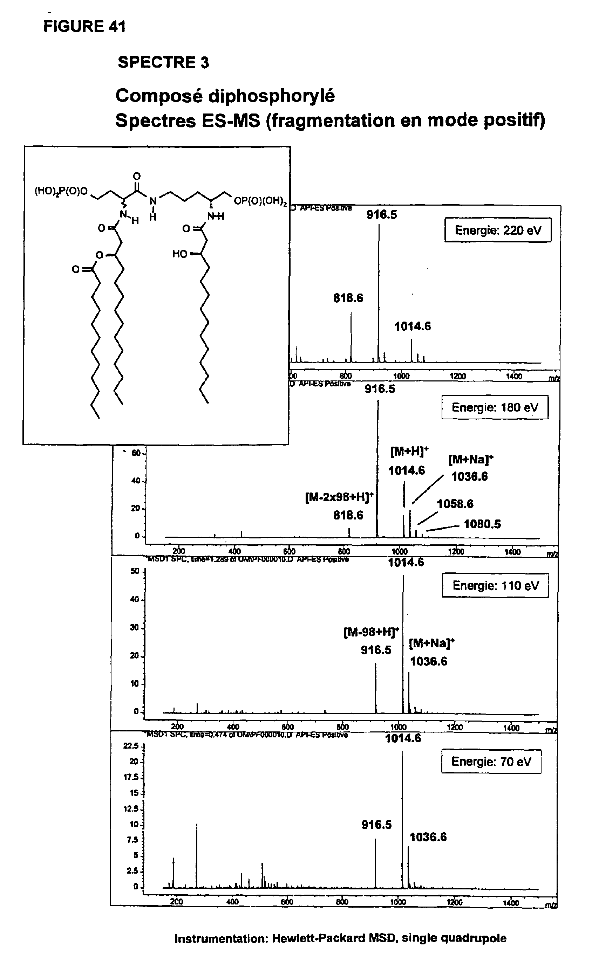

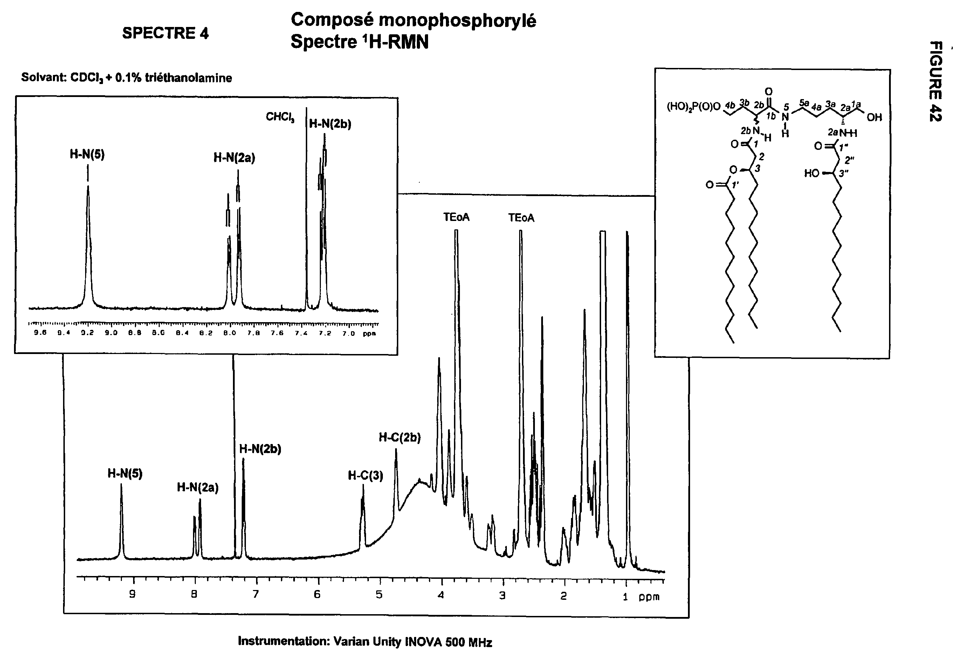

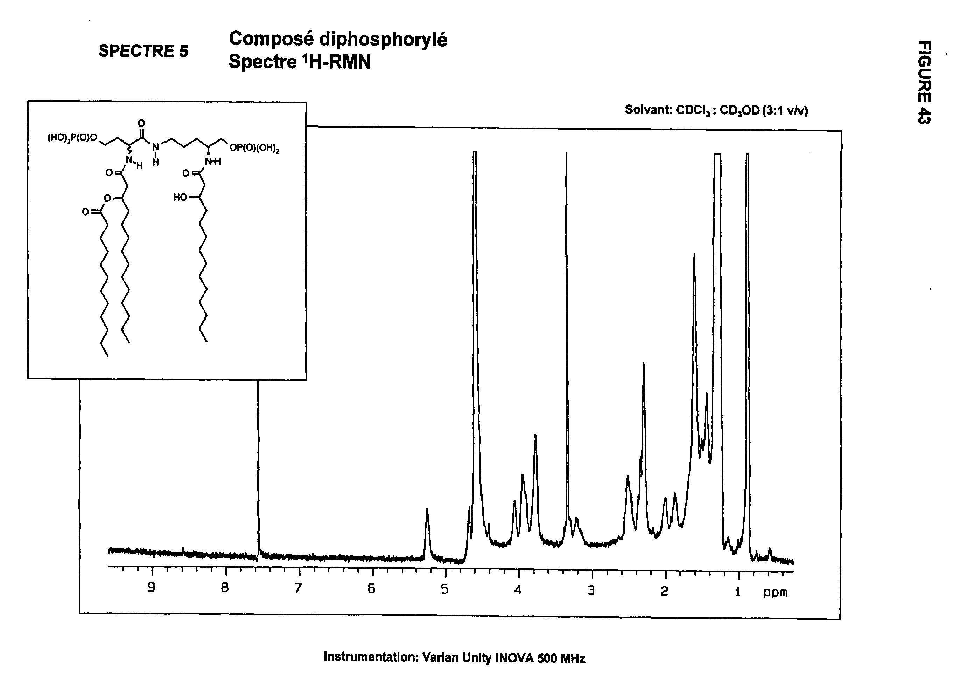

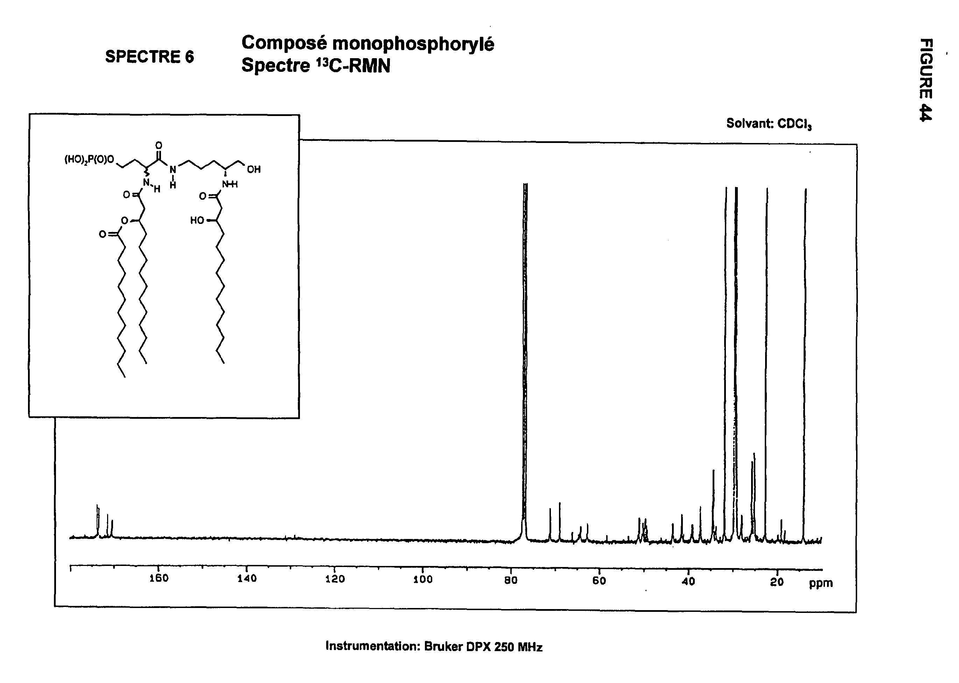

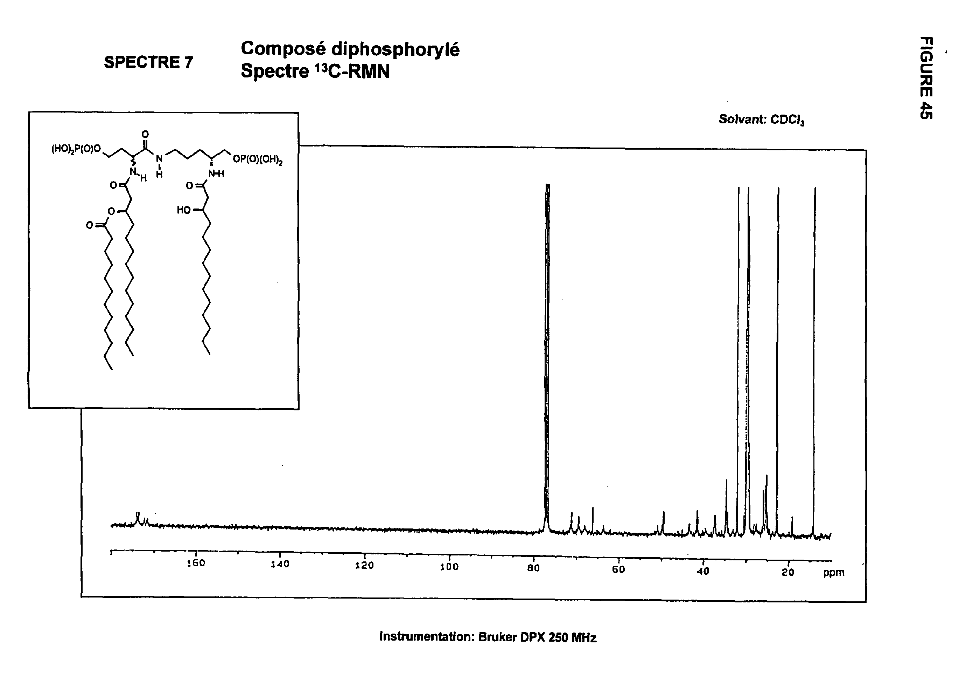

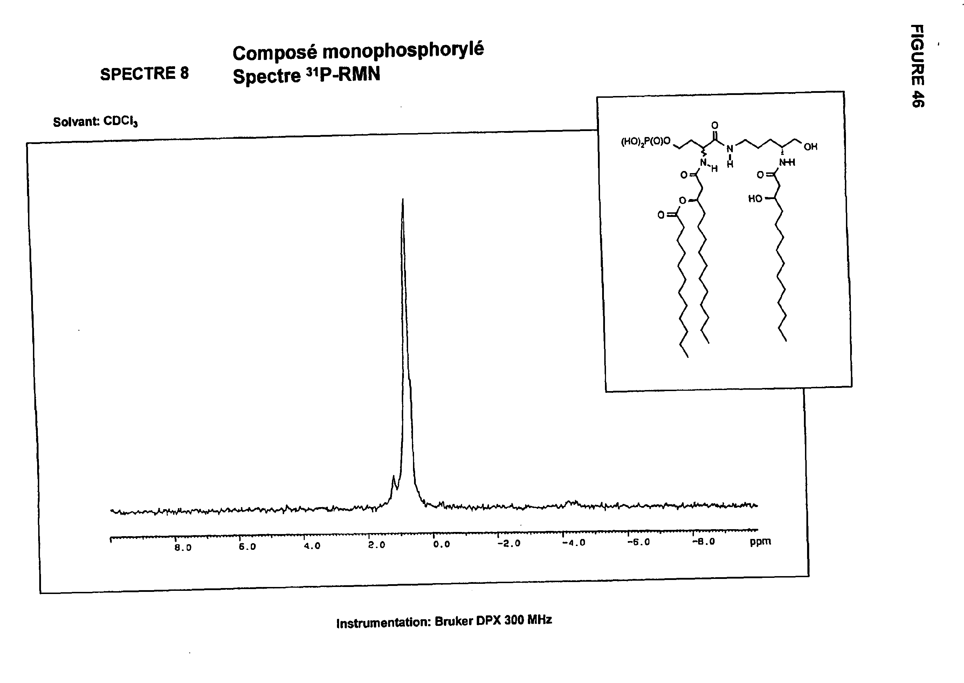

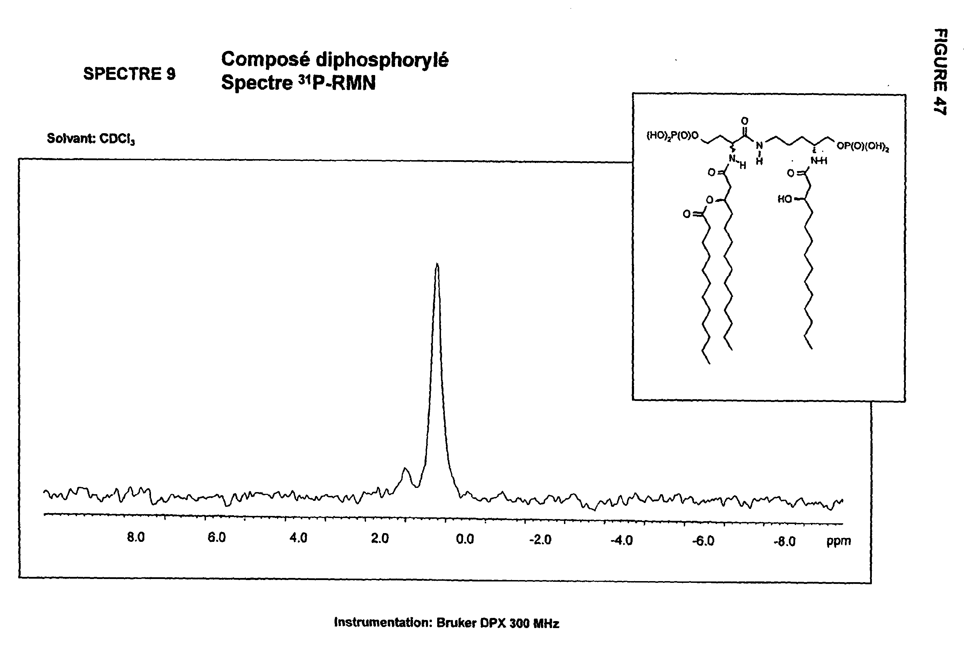

Chez les souris immunisées avec l'antigène LmCPb (Tab (a) et (b) et Fig 32 (a, b)), les produits OM-294-MP et OM-294-DP produisent un effet semblable à celui qui a été observé chez les souris développant une réponse immune contre gp63. Ainsi, en présence de LmCPb (15 µg/ml), les cultures proviennent des souris immunisées avec l'antigène plus les adjuvants OM-294-MP et OM-294-DP présentent une prolifération respectivement 23 et 28 fois plus intense que les cultures proviennent de souris ayant reçu l'antigène seul (sans adjuvant). L'influence du BCG dans ces conditions est moindre, puisque l'augmentation de la prolifération est de 11 fois seulement. Des effets analogues se retrouvent que les cultures sont stimulées avec l'antigène purifié ou avec un extrait total du parasite *Leishmania,* et à toutes les concentrations d'antigène testées.
La production d'IFN-γ en réponse à l'antigène LmCPb a tendance à être un peu plus élevée avec le produit OM-294-DP qu'avec le BCG (Tab (b) et Fig 32 (b)). II est à noter que dans cette expérience, les lymphocytes prolifèrent et sécrètent des quantités importantes d'IFN-γ même si de l'antigène n'est pas ajouté au milieu de culture. Dans ce cas aussi l'adjuvant OM-294-DP tend à être un peu plus efficace que le BCG. Une différence nette entre les adjuvants OM-294-MP, respectivement OM-294-DP et le BCG apparaît comme précédemment pour ce qui concerne le développement de lymphocytes capables de produire de l'IL-4. La quantité d'IL-4 produite sous l'effet des adjuvants OM-294-MP et OM-294-DP est importante puisqu'elle équivaut à la quantité sécrétée par des lymphocytes exposés à la Con A, un puissant stimulant non spécifique des lymphocytes (voir Tab (a) et (b)).

**Tableau (a): Réponse proliférative in vitro de cellules ganglionnaires provenant de souris immunisées in vivo avec LmCPb : effet de différents adjuvants.**

| | Incorporation de ³H-TdR (cpmx10⁻³/m) | | | |
|---|---|---|---|---|
| Stimulant *in vitro* | Sans adjuvant | OM-294-DP | OM-294-MP | BCG |
| Pas de stimulant | 1.7 ± 0.6 | 21.8 ± 2.2 | 17.1 ± 2.5 | 18.6 ± 4.8 |
| LmCPb 0.6 µg/ml | 0.8 ± 0.3 | 40.9 ± 12.7 | 22.9 ± 2.8 | 19.0 ± 7.4 |
| LmCPb 1.7 µg/ml | 2.4 ± 0.1 | 57.2 ± 10.9 | 34.1 ± 4.1 | 39.8 ± 5.7 |
| LmCPb 5 µg/ml | 2.8 ± 0.6 | 70.2 ± 9.2 | 70.3 ± 6.4 | 44.0 ± 10.4 |
| LmCPb 15 µg/ml | 4.3 ± 0.1 | 100.0 ± 6.5 | 124.2 ± 12.0 | 46.2 ± 0.3 |
| Amastigotes 2 x 10⁻⁶/ml | 2.4 ± 0.6 | 61.0 ± 1.7 | 28.4 ± 8.3 | 24.4 ± 4.3 |
| Amastigotes 6 x 10⁻⁶/ml | 2.3 ± 0.7 | 81.1 ± 5.5 | 66.1 ± 4.5 | 23.6 ± 2.5 |
| Amastigotes 17 x 10⁻⁶/ml | 1.7 ± 0.4 | 78.2 ± 7.8 | 68.7 ± 2.3 | 23.4 ± 4.0 |
| ConA5µg/ml | 188.1 ± 21.0 | 135.9 ± 3.7 | 151.4 ± 3.7 | 119.7 ± 28.5 |

Les valeurs reportées dans le tableau représentent la moyenne arithmétique ± la déviation standard de l'incorporation (cultures en triplicats).

**Tableau (b): Production de cytokines in vitro par des lymphocytes ganglionnaires de souris immunisés in vivo par l'antigène LmCPb : effet de différents adjuvants.**

| Concentration de IFN-γ en (pg/ml) | | | | |
|---|---|---|---|---|
| Stimulant *in vitro* | Sans adjuvant | OM-294-DP | OM-294-MP | BCG |
| Pas de stimulant | < 9 | 460 | 240 | 280 |
| LmCPb 15 µg/ml | 44 | 520 | 360 | 460 |
| Amastigotes 17 x 10⁻⁶/ml | 14 | > 600 | > 600 | 480 |
| Con A 5 µg/ml | 1200 | 1200 | 1900 | > 3000 |

| Concentration de IL-4 en (pg/ml) | | | | |
|---|---|---|---|---|
| Stimulant *in vitro* | Sans adjuvant | OM-294-DP | OM-294-MP | BCG |
| Pas de stimulant | < 15 | < 8 | < 8 | < 8 |
| LmCPb 15 µg/ml | < 15 | 110 | 88 | 36 |
| Amastigotes 17 x 10⁻⁶/ml | < 8 | 130 | 110 | 29 |
| Con A 5 µg/ml | 40 | 230 | 85 | 105 |

Les adjuvants OM-294-MP et OM-294-DP potentialisent de façon efficace la réponse immune à un antigène soluble de *Leishmania,* la protéase LmCPb. Cela se marque *in vitro* par une augmentation de la réponse proliférative suivie de l'induction de la production d'IFN-γ et d'IL-4 en quantités significatives.

### EXEMPLE VI

### Solution aqueuse injectable

| | |
|---|---|
| Composé de l'exemple III | 1 g |
| Polysorbate 80 | 0.2 g |
| Chlorure de sodium | 9 g |
| Eau distillée pour injection qsp | 1'000 ml |

La solution est ajustée à pH 7.4 avec HCl 0.1M et puis elle est stérilisée par filtration sur membrane 0.22 µm Steritop Express 1000 (membrane PES, 90 mm, SCGP T10 RE, Millipore Corporation, Bedford, MA, U.S.A.). La solution stérile est répartie en ampoules stériles de 1 ml.

### Lyophilisat

| | |
|---|---|
| Composé de l'exemple IV | 2 g |
| Polysorbate 80 | 0.2 g |
| Chlorure de sodium | 9 g |
| Mannitol | 10 g |
| Acide ascorbique | 0.1 g |
| Eau distillée pour injection qsp | 1'000 ml |

La solution est ajustée à pH 7.4 avec HCl 0.1M, puis elle est stérilisée par filtration sur membrane 0.22 µm Steritop Express 1000 (membrane PES, 90 mm, SCGP T10 RE, Millipore Corporation, Bedford, MA, U.S.A.). La solution stérile est répartie en flacons multidose stériles à raison de 1 ml par flacon, puis la solution est lyophilisée.

## Revendications

1. Les composés de formule générale I : 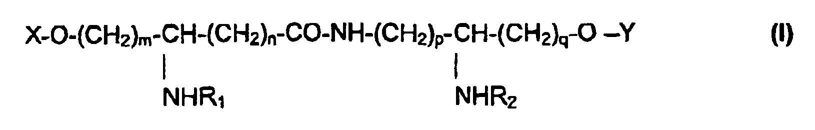 dans laquelle R₁ et R₂ représentent chacun un groupe acyle dérivé d'un acide carboxylique ayant de 2 à 24 atomes de carbone, saturé ou insaturé, linéaire ou ramifié, non-substitué ou portant un ou plusieurs substituants choisis dans le groupe formé de hydroxyle, alkyle, alkoxy, acyloxy, amino, acylamino, acylthio et (alkyle en C₁-C₂₄) thio,
le descripteur m possède une valeur allant de 1 à 4,
le descripteur n possède la valeur 0,
le descripteur p possède une valeur de 3 ou 4 et q possède une valeur de 1
X et Y représentent chacun un hydrogène ou un groupe phosphono, avec la limitation que l'un au moins des substituants X et Y représente un groupe phosphono,

2. Un composé selon la revendication 1, dans lequel le ou les groupe(s) phosphono est (sont) salifié(s) par une base minérale ou organique.

3. Un composé selon la revendication 1 ou la revendication 2, à savoir les 1 et/ou 10-dihydrogénophosphate de 3-(3-dodécanoyloxytétradécanoylamino) 9-(3-hydroxytétradécanoylamino)-4-oxo-5-azadecane-1,10-diols et leurs sels d'addition avec une base minérale ou organique.

4. Un composé selon la revendication 1 ou la revendication 2, choisi parmi le 1,10-bis-(dihydrogénophosphate) de 3-(3-dodécanoyloxytétradécanoylamino) 9-(3-hydroxytétradécanoylamino) 4-oxo-5-azadecane-1,10-diol et ses sels d'addition avec une base minérale ou organique.

5. Un composé selon la revendication 1 ou la revendication 2, choisi parmi le 1,10-bis(dihydrogénophosphate) de 3-(3-hydroxytétradécanoylamino) 9-(3-dodécanoyloxytétradécanvylamino) 4-oxo-5-azadécane-1,10-diol et ses sels d'addition avec une base minérale ou organique.

6. Un composé selon la revendication 1 ou la revendication 2, choisi parmi le 1-dihydrogénophosphate de 3-(3-dodécanoyloxytétradécanoylamino) 9-(3-hydroxytétradécanoylamino) 4-oxo-5-azadécane 1,10-diol et ses sels d'addition avec une base minérale ou organique.

7. Un composé selon la revendication 1 ou la revendication 2, choisi parmi le 1-dihydrogénophosphate de 3-(3-hydroxytétradécanoylamino)-9-(3-dodécanoyloxytétradécanoylamino)-4-oxo-5-azadécane-1,10-diol et ses sels d'addition avec une base minérale ou organique.

8. Un composé selon la revendication 1 ou la revendication 2, à savoir un composé choisi parmi le 10-dihydrogénophosphate de 3-(3-hydroxytétradécanoylamina)-9-(3-dodécanoyloxytétradécanoylamino)-4-oxo-5-azadecane -1,10-diol et ses sels d'addition avec une base minérale ou organique.

9. Procédé d'obtention des phosphopseudodipeptides de formule générale I 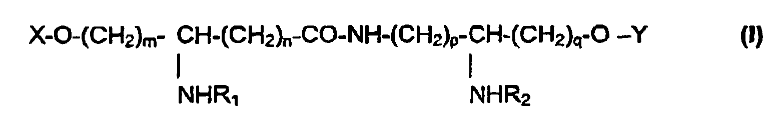 dans laquelle R₁ et R₂ représentent chacun un groupe acyle dérivé d'un acide carboxylique ayant de 2 à 24 atomes de carbone, saturé ou insaturé, linéaire ou ramifié, non-substitué ou portant un ou plusieurs substituants choisis dans le groupe formé de hydroxyle, alkyle, alkoxy, acyloxy, amino, acylamino, acylthio et (alkyl en C₁-C₂₄) thio
le descripteur m prend une valeur allant de 1 à 4,
le descripteur n prend une valeur 0,
le descripteur p est égal à 3 ou 4 et le descripteur q possède la valeur 1
X et Y représentent chacun un hydrogène ou un groupe phosphono, avec la limitation que l'un au moins des substituants X et Y représente un groupe phosphono,
qui consiste en ce qu'on bloque les fonctions amine en position non-terminale (q + 1) et en position terminale ω d'un acide diaminé de formule H₂N(CH₂)ₚ CHNH₂(CH₂)_{q-1}COOH par des réactifs de blocage labiles par acidolyse et hydrogénolyse, respectivement, soumet la fonction carboxylique restée libre à l'action d'un agent réducteur pour former l'alcool correspondant, libère la fonction amine non-terminale en (q+1) que l'on acyle à l'aide d'un dérivé fonctionnel d'un acide carboxylique de formule R₂OH, dans laquelle R₂ est défini comme précédemment, puis libère la fonction amine terminale en m par hydrogénolyse pour obtenir l'amino alcool de formule générale II 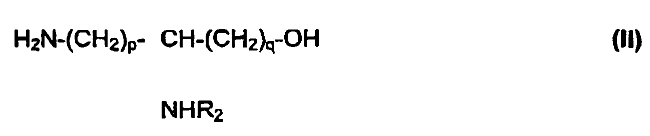 dans laquelle R₂ représente un groupe acyle dérivé d'acide carboxylique ayant de 2 à 24 atomes de carbone, saturé ou non-saturé, non-substitué ou portant un ou plusieurs substituants définis comme ci-dessus,
p prend la valeur 1, 3 ou 4
et q est égal à 1
que l'on condense en présence d'un agent de condensation peptidique dans un solvant inerte, avec un dérivé fonctionnel d'acide ω-hydroxy aminé de formule générale III 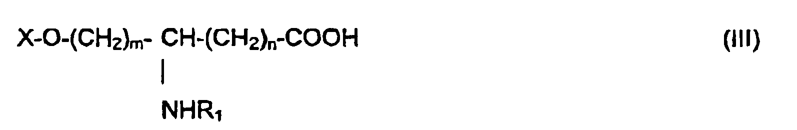 dans laquelle R₁ est un groupe acyle dérivé d'un acide carboxylique ayant de 2 à 24 atomes de carbone, saturé ou non-saturé, non-substitué ou portant un ou plusieurs substituants définis comme précédemment
m est un nombre entier variant de 1 à 4,
n est égal à 0,
et X est un radical dialkyloxy- ou diaryloxy-phosphoryle de formule 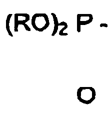 pour former le pseudodipeptide de formule générale IV 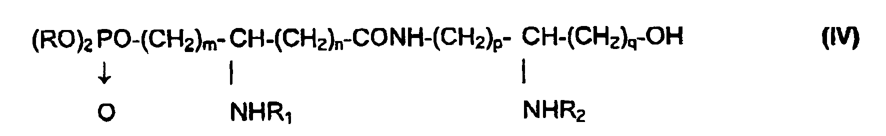 dans laquelle les substituants R₁, R₂ et les descripteurs m, n, p et q sont définis comme précédemment, et R est un radical labile par hydrogénolyse,
dont on peut -si désiré- phosphoryler l'autre fonction alcool par un agent de phosphorylation, si nécessaire, en présence d'un agent de couplage, et soumettre à une hydrogénation catalytique d'une part pour débloquer la fonction alcool éventuellement présente sur le groupe acyle R₂ et, d'autre part, libérer la fonction phosphate puis débloquer par hydrogénolyse la deuxième fonction phosphate éventuellement présente, de façon à obtenir le dérivé de formule générale V 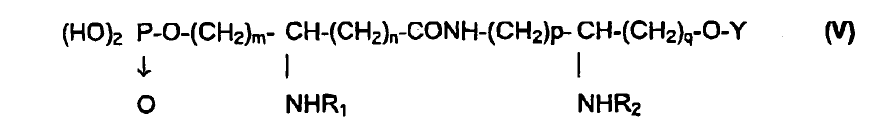 dans laquelle Y représente soit un hydrogène soit un groupe phosphono.et les descripteurs m, n, p et q ont les valeurs fournies précédemment et si désiré, on effectue l'étape supplémentaire de salification à l'aide d'une base minérale ou organique.

10. A titre d'intermédiaires de la synthèse des composés de formule I, les dérivés fonctionnels d'ω-hydroxy amino acide de formule III 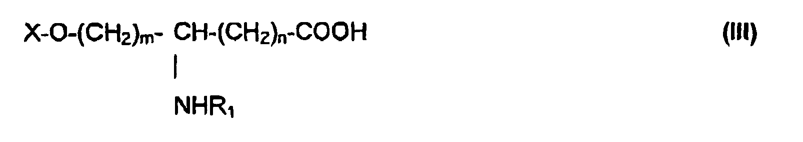 dans laquelle R₁ est un groupe acyle dérivé d'un acide carboxylique ayant de 2 à 24 atomes de carbone, saturé ou non-saturé, non-substitué ou portant un ou plusieurs substituants définis comme précédemment
m est un nombre entier variant de 1 à 4
n est égal à 0
et X est un radical dialkyloxy- ou diaryloxy-phosphoryle de formule

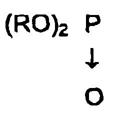

11. A titre d'intermédiaires de la synthèse des composés de formule I selon la revendication 1, les pseudodipeptides de formule générale IV 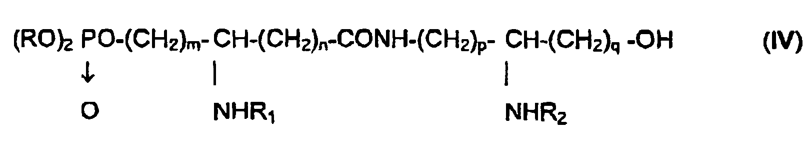 dans laquelle les substituants R₁, R₂ et les descripteurs m, n, p et q sont définis comme à la revendication 1, et R est un radical alkyle ou aryle, labile par hydrogénolyse.

12. A titre d'intermédiaires de la synthèse des composés de formule I selon la revendication 1,
les aminoalcools de formule générale II 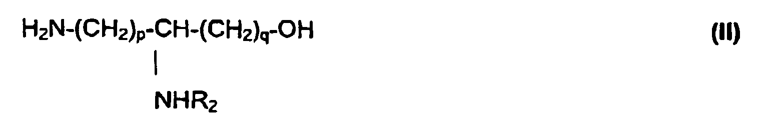 dans laquelle R₂ représente un groupe acyle dérivé d'un acide carboxylique ayant de 2 à 24 atomes de carbone, saturé ou non-saturé, non-substitué ou portant un ou plusieurs substituants définis comme ci-dessus,
p représente un nombre entier égal à 3 ou 4
et q est égal à 1

13. Les compositions pharmaceutiques à visée immunologique renfermant, à titre de principe actif, au moins un composé de formule générale I selon la revendication 1 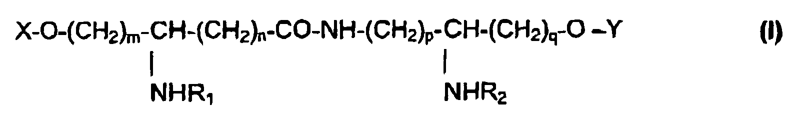 dans laquelle R1 et R2 représentent chacun un groupe acyle dérivé d'un acide carboxylique ayant de 2 à 24 atomes de carbone, saturé ou insaturé, linéaire ou ramifié, non-substitué ou portant un ou plusieurs substituants choisis dans le groupe formé de hydroxyle, alkyle, alkoxy, acyloxy, amino, acylamino, acylthio et (alkyle en C₁-C₂₄)thio
le descripteur m prend une valeur allant de 1 à 4,
le descripteur n est égal à 0, p prend une valeur de 3 ou 4 et q est égal à 1
X et/ou Y représentent chacun un hydrogène ou un groupe phosphono sous forme neutre ou chargée avec la limitation que l'un au moins des substituants X et Y représente un groupe phosphono,
en association ou en mélange avec un excipient ou un véhicule inerte, non toxique, pharmaceutiquement acceptable.

14. Compositions pharmaceutiques selon la revendication 13, dans lesquelles le composé de formule I est un de ceux pour lesquels X et/ou Y représentent un radical phosphono.

15. Compositions pharmaceutiques selon la revendication 13, dans lesquelles le principe actif est sous forme salifiée avec une base minérale ou organique thérapeutiquement compatible.

16. Compositions pharmaceutiques selon l'une des revendications 13 et 15, dans lesquelles le principe actif est sous forme énantiomériquement pure ou sous forme de mélange de stéréoisomères.

## Claims

1. The compounds of general formula **I**: 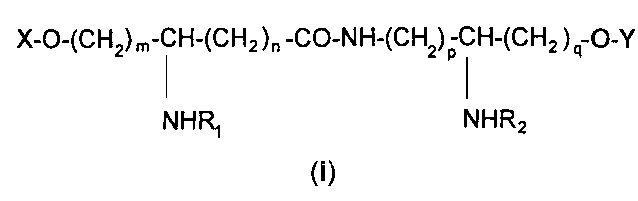 wherein R₁ and R₂ each designate an acyl group derived from a saturated or unsaturated, straight- or branched-chain carboxylic acid having from 2 to 24 carbon atoms, which is unsubstituted or bears one or several substituents selected from the group comprised of hydroxyl, alkyl, alkoxy, acyloxy, amino, acylamino, acylthio and ((C₁-C₂₄)alkyl)thio groups, subscript m takes a value ranging from 1 to 4,
subscript n is 0,
subscript p takes a value of 3 or 4 and subscript q is 1
X and Y each designate a hydrogen atom or a phosphono group with the limitation that at least one of the substituents X and Y represent a phosphono group.

2. A compound in accordance with claim 1, in which the phosphono group(s) is(are) made into salt form with an inorganic or organic base.

3. A compound in accordance with claim 1 or with claim 2, namely the 3-(3-dodecanoyloxytetradecanoylamino) 9-(3-hydroxytetradecanoylamino)-4-oxo-5-azadecan-1,10-diol 1- and/or 10-dihydrogenophosphates and their addition salts formed with an organic or an inorganic base.

4. A compound in accordance with claim 1 or with claim 2 selected from the group consisting of 3-(3-dodecanoyloxytetradecanoylamino) 9-(3-hydroxytetradecanoylamino)-4-oxo-5-azadecan-1, 10-diol 1,10-bis-(dihydrogenophosphate) and its addition salts formed with an organic or an inorganic base.

5. A compound in accordance with claim 1 or with claim 2 selected from the group consisting of 3-(3-hydroxytetradecanoylamino) 9-(3-dodecanoyloxytetradecanoylamino)-4-oxo-5-azadecan-1,10-diol 1,10-bis-(dihydrogenophosphate) and its addition salts formed with an organic or an inorganic base.

6. A compound in accordance with claim 1 or with claim 2 selected from the group consisting of 3-(3-dodecanoyloxytetradecanoylamino) 9-(3-hydroxytetradecanoylamino)-4-oxo-5-azadecan 1,10-diol 1-dihydrogenophosphate and its addition salts formed with an organic or an inorganic base.

7. A compound in accordance with claim 1 or with claim 2 selected from the group consisting of 3-(3-hydroxytetradecanoylamino) 9-(3-dodecanoyloxytetradecanoylamino)-4-oxo-5-azadecan-1,10-diol 1-dihydrogenophosphate and its addition salts formed with an organic or an inorganic base.

8. A compound in accordance with claim 1 or with claim 2 selected from the group consisting of 3-(3-hydroxytetradecanoylamino) 9-(3-dodecanoyloxytetradecanoylamino)-4-oxo-5-azadecan-1,10-diol 10-dihydrogenophosphate and its addition salts formed with an organic or an inorganic base.

9. A method for obtaining phosphodipeptide-like compounds of general formula **I** in accordance with claims 1 or 2 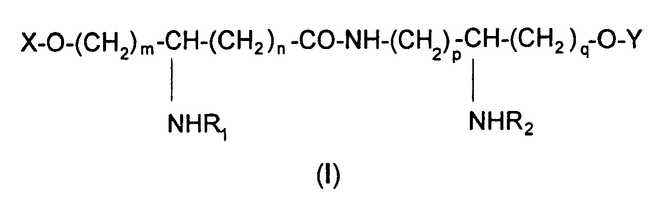 wherein R₁ and R₂ each designate an acyl group derived from a saturated or unsaturated, straight- or branched-chain carboxylic acid having from 2 to 24 carbon atoms, which is unsubstituted or bears one or several substituents selected from the group comprised of hydroxyl, alkyl, alkoxy, acyloxy, amino, acylamino, acylthio and ((C₁-C₂₄)alkyl)thio groups, subscript m takes a value ranging from 1 to 4,
subscript n is 0,
subscript p takes a value of 3 or 4 and subscript q is 1
X and Y each designate a hydrogen atom or a phosphono group with the limitation that at least one of the substituents X and Y represent a phosphono group,
wherein the amine functional groups in non-terminal position (q+1) and in terminal position ω of the diamino acid of formula
H₂N(CH₂)ₚCHNH₂(CH₂)_{q-1}COOH are protected by blocking groups which readily undergo acidolysis and hydrogenolysis, respectively, the carboxylic functional group still in free form is reacted with a reducing agent to yield the corresponding alcohol, the amine functional group in non-terminal position (q+1) is deprotected and then acylated by means of an active derivative of a carboxylic acid of formula R₂OH wherein R₂ is as defined above, the amine functional group in terminal position ω is subsequently deprotected by hydrogenolysis to yield the amino-alcohol of general formula **II** 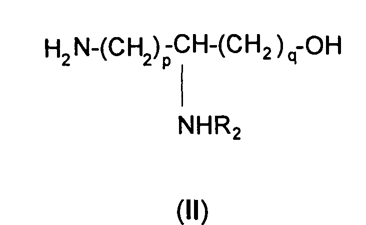 wherein R₂ designates an acyl group derived from a saturated or unsaturated, straight- or branched-chain carboxylic acid having from 2 to 24 carbon atoms, which is unsubstituted or bears one or several substituents as specified above,
subscript p takes a value of 3 or 4
and subscript q is 1
which amino-alcohol is condensed in the presence of a peptide condensing agent in an inert solvant with a ω-hydroxy amino acid derivative of general formula **III** : 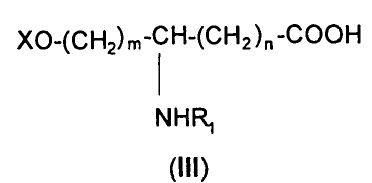 wherein R₁ is an acyl group derived from a saturated or unsaturated, straight- or branched-chain carboxylic acid having from 2 to 24 carbon atoms, which is unsubstituted or bears one or several substituents as specified above,
subscript m is an integer ranging from 1 to 4,
subscript n is 0,
and X is a dialkyloxy- or diaryloxy- phosphoryl radical of formula 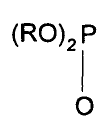 to yield the dipeptide-like compound of general formula **IV** 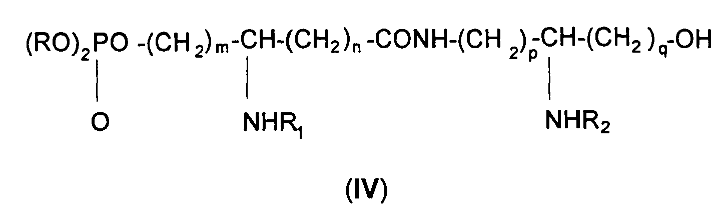 wherein substituents R₁, R₂ and descriptors m, n, p and q are as defined above, and R is a radical which is readily cleaved by hydrogenolysis, the free alcohol functional group of which can be - if need be - phosphorylated by a phosphorylating agent in the presence of a coupling agent, if needed, and perform a catalytic hydrogenation in order to unblock the alcohol functional group optionally present in acyl group R₂ as well as the phosphate functional group followed by a second hydrogenolysis to deprotect the second phosphate group optionally present, in order to obtain the derivative of general formula **V** 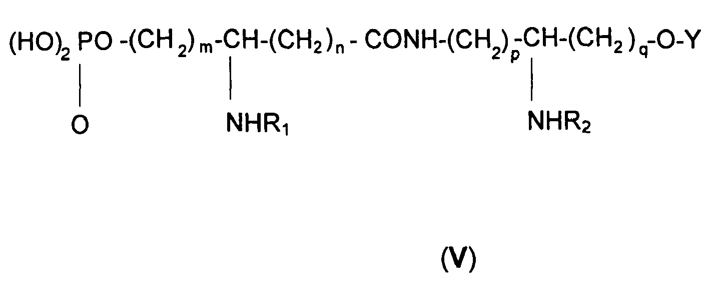 wherein Y designates either a hydrogen atom or a phospono group and the descriptors m, n, p, and q are as defined above
and, if needed, the subsequent step of salt formation is performed with an inorganic or an organic base.

10. As intermediates in the synthesis of compounds of general formula I, ω-hydroxy amino acid derivatives of formula III 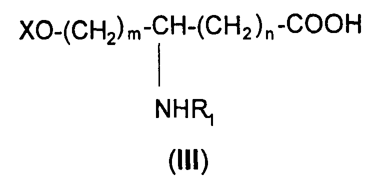 wherein R₁ is an acyl group derived from a saturated or unsaturated, straight- or branched-chain carboxylic acid having from 2 to 24 carbon atoms, which is unsubstituted or bears one or several substituents as
specified above,
subscript m is an integer ranging from 1 to 4,
subscript n is 0,
and X is a dialkyloxy- or diaryloxy-phosphoryl radical of formula

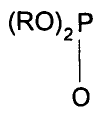

11. As intermediates in the synthesis of compounds of general formula I according to claim 1, dipeptide-like compounds of general formula **IV** 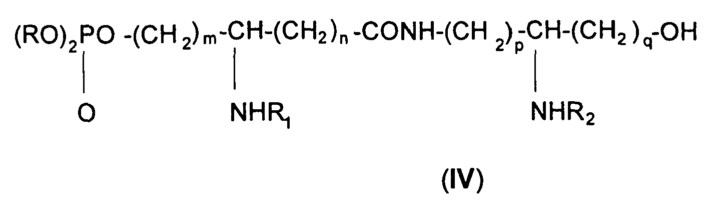 wherein substituents R₁, R₂ and descriptors m, n, p and q are defined as in claim 1, and R is an alkyl or aryl radical which is readily cleaved by hydrogenolysis.

12. As intermediates in the synthesis of compounds of general formula **I** according to claim 1, amino alcohols of general formula **II** 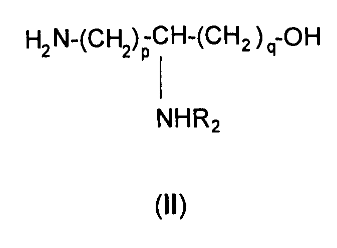 wherein R₂ designates an acyl group derived from a saturated or unsaturated, straight- or branched-chain carboxylic acid having from 2 to 24 carbon atoms, which is unsubstituted or bears one or several substituents as specified above,
subscript p is an integer taking a value of 3 or 4
and subscript q is 1

13. Pharmaceutical compositions intended for modulation of immune response containing as an active ingredient at least one compound of general formula **I** in accordance with claim 1 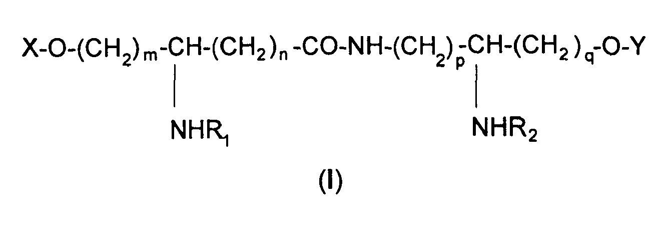 wherein R₁ and R₂ each designate an acyl group derived from a saturated or unsaturated, straight- or branched-chain carboxylic acid having from 2 to 24 carbon atoms, which is unsubstituted or bears one or several substituents selected from the group comprised of hydroxyl, alkyl, alkoxy, acyloxy, amino, acylamino, acylthio and ((C₁-C₂₄)alkyl)thio groups, subscript m takes a value ranging from 1 to 4,
subscript n is 0,
subscript p takes a value of 3 or 4 and subscript q is 1
X and/or Y each designate a hydrogen atom or a phosphono group in neutral or salt form with the limitation that at least one of the substituents X and Y represent a phosphono group.
together or in admixture with a non toxic, pharmaceutically acceptable, inert excipient or carrier

14. The pharmaceutical compositions in accordance with claim 13, wherein the compound of formula **I** is a compound of the type where X and/or Y designate a phosphono radical.

15. The pharmaceutical compositions in accordance with claim 13, wherein the active ingredient is in salt form made with an organic or inorganic base intended for therapeutic use.

16. The pharmaceutical compositions in accordance with one of claims 13 and 15, wherein the active ingredient is in the form of a pure enantiomer or in the form of a mixture of stereoisomers.

## Patentansprüche

1. Verbindungen mit der allgemeinen Formel I: 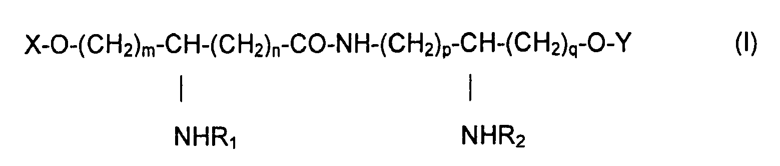 in der R₁ und R₂ jeweils eine Acylgruppe darstellen, die von einer Carboxylsäure abgeleitet ist, die 2 bis 24 Kohlenstoffatome aufweist, gesättigt oder ungesättigt ist, linear oder verzweigt ist, nicht substituiert ist oder einen oder mehrere Substituenten trägt, die in der Gruppe gewählt werden, die aus Hydroxyl, Alkyl, Alkoxy, Acyloxy, Amino, Acylamino, Acylthio und (C₁-C₂₄)Alkylthio gewählt wird, der Deskriptor m einen Wert von 1 bis 4 besitzt,
der Deskriptor n den Wert 0 besitzt,
der Deskriptor p einen Wert von 3 oder 4 besitzt und q einen Wert von 1 besitzt, X und Y jeweils einen Wasserstoff oder eine Phosphongruppe darstellen, mit der Einschränkung, dass mindestens einer der Substituenten X und Y eine Phosphongruppe darstellt.

2. Verbindung nach Anspruch 1, in der die Phosphongruppe(n) durch eine mineralische oder organische Base in ein Salz umgewandelt ist (sind).

3. Verbindung nach Anspruch 1 oder Anspruch 2, nämlich 1 und/oder 10-Dihydrogenphosphat von 3-(3-Dodecanoyloxytetradecanoylamino)9-(3-hydroxytetradecanoylamino)-4-oxo-5-azadecan-1,10-diolen und ihren Additionssalzen mit einer mineralischen oder organischen Base.

4. Verbindung nach Anspruch 1 oder Anspruch 2, gewählt unter 1,10-bis-(Dihydrogenphosphat) von 3-(3-Dodecanoyloxytetradecanoylamino)9-(3-hydroxytetradecanoylamino)4-oxo-5-azadecan-1,10-diol und seinen Additionssalzen mit einer mineralischen oder organischen Base.

5. Verbindung nach Anspruch 1 oder Anspruch 2, gewählt unter 1,10-bis-(Dihydrogenphosphat) von 3-(3-Hydroxytetradecanoylamino)9-(3-dodecanoyloxytetradecanoylamino)4-oxo-5-azadecan-1,10-diol und seinen Additionssalzen mit einer mineralischen oder organischen Base.

6. Verbindung nach Anspruch 1 oder Anspruch 2, gewählt unter 1-Dihydrogenphosphat von 3-(3-Dodecanoyloxytetradecanoylamino)9-(3-hydroxytetradecanoylamino)4-oxo-5-azadecan-1,10-diol und seinen Additionssalzen mit einer mineralischen oder organischen Base.

7. Verbindung nach Anspruch 1 oder Anspruch 2, gewählt unter 1-Dihydrogenphosphat von 3-(3-Hydroxytetradecanoylamino)9-(3-dodecanoyloxytetradecanoylamino)-4-oxo-5-azadecan-1,10-diol und seinen Additionssalzen mit einer mineralischen oder organischen Base.

8. Verbindung nach Anspruch 1 oder Anspruch 2, nämlich eine Verbindung, gewählt unter 10-Dihydrogenphosphat von 3-(3-Hydroxytetradecanoylamino)-9-(3-dodecanoyloxytetradecanoylamino)-4-oxo-5-azadecan-1,10-diol und seinen Additionssalzen mit einer mineralischen oder organischen Base.

9. Verfahren zur Herstellung von Phosphopseudodipeptiden mit der allgemeine Formel I 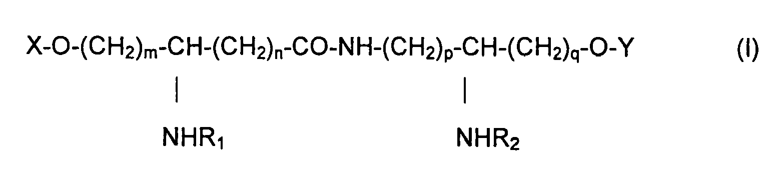 in der R₁ und R₂ jeweils eine Acylgruppe darstellen, die von einer Carboxylsäure abgeleitet ist, die 2 bis 24 Kohlenstoffatome aufweist, gesättigt oder ungesättigt ist, linear oder verzweigt ist, nicht substituiert ist oder einen oder mehrere Substituenten trägt, die in der Gruppe gewählt werden, die aus Hydroxyl, Alkyl, Alkoxy, Acyloxy, Amino, Acylamino, Acylthio und (C₁-C₂₄)Alkylthio gewählt wird, der Deskriptor m einen Wert von 1 bis 4 annimmt,
der Deskriptor n einen Wert 0 annimmt,
der Deskriptor p gleich 3 oder 4 ist und der Deskrptor q den Wert 1 besitzt,
X und Y jeweils einen Wasserstoff oder eine Phosphongruppe darstellen, mit der Einschränkung, dass mindestens einer der Substituenten X und Y eine Phosphongruppe darstellt,
das darin besteht, dass die Aminogruppen in der nicht endständigen Position (q + 1) und in der endständigen Position ω einer Diaminosäure mit der Formel H₂N(CH₂)ₚCHNH₂(CH₂)_{q-1}COOH durch Blockierungsreagenzien blockiert werden, die durch Acidolyse bzw. Hydrogenolyse labil sind, die frei gebliebene Carboxylgruppe der Wirkung eines Reduktionsmittels ausgesetzt wird, um den entsprechenden Alkohol zu bilden, die nicht endständige Aminogruppe bei (q + 1) freigegeben wird, die mit Hilfe eines funktionellen Derivats einer Carboxylsäure mit der Formel R₂OH acyliert wird, in der R₂ wie oben definiert wird, anschließend die endständige Aminogruppe bei ω durch Hydrogenolyse freigegeben wird, um den Aminoalkohohl mit der allgemeinen Formel II 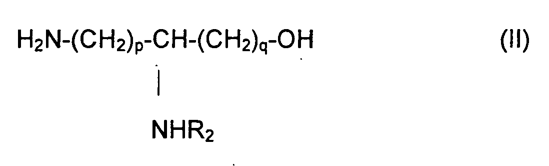 zu bilden, in der R₂ eine Acylgruppe darstellt, die von einer Carboxylsäure abgeleitet ist, die 2 bis 24 Kohlenstoffatome aufweist, gesättigt oder ungesättigt ist, nicht substituiert ist oder einen oder mehrere Substituenten trägt, die wie oben definiert sind,
p den Wert 1, 3 oder 4 annimmt,
und q gleich 1 ist,
der in Gegenwart eines peptidischen Kondensationsmittels in einem inaktiven Lösemittel mit einem funktionellen Derivat von ω-Hydroxyaminosäure mit der allgemeinen Formel III 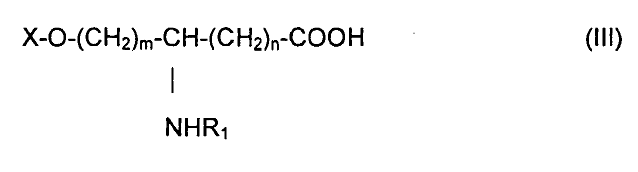 kondensiert wird, in der R₁ eine Acylgruppe darstellt, die von einer Carboxylsäure abgeleitet ist, die 2 bis 24 Kohlenstoffatome aufweist, gesättigt oder ungesättigt ist, nicht substituiert ist oder einen oder mehrere Substituenten trägt, die wie oben definiert sind,
m eine ganze Zahl von 1 bis 4 ist,
n gleich 0 ist,
und X ein Dialkyloxy- oder Diaryloxy-phosphorylradikal mit der Formel 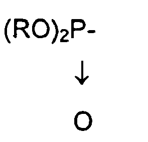 ist, um das Pseudodipeptid mit der allgemeinen Formel IV 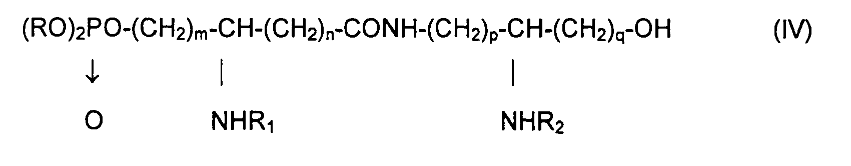 zu bilden, in der die Substituenten R₁, R₂ und die Deskriptoren m, n, p und q wie oben definiert sind und R ein durch Hydrogenolyse labiles Radikal ist,
dessen andere Alkoholgruppe - wenn gewünscht - durch ein Phosphorylierungsmittel phosphoryliert werden kann, wenn notwendig, in Gegenwart eines Kopplungsmittels, wobei dies einerseits einer katalytischen Hydrierung unterzogen werden kann, um die Alkoholgruppe zu deblockieren, die eventuell an der Acylgruppe R₂ vorhanden ist, und andererseits die Phosphatgruppe freigegeben und anschließend durch Hydrogenolyse die zweite eventuell vorhandene Phosphatgruppe deblockiert werden kann, um das Derivat mit der allgemeinen Formel V 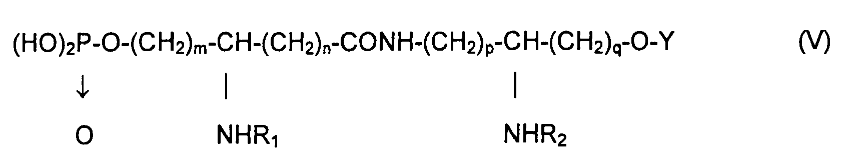 zu erhalten, in der Y entweder ein Wasserstoff oder eine Phosphongruppe darstellt und die Deskriptoren m, n, p und q die oben angegebenen Werte aufweisen
und wobei, wenn gewünscht, der zusätzliche Salzbildungsschritt mit Hilfe einer mineralischen oder organischen Base durchgeführt wird.

10. Als Zwischenstufen der Synthese der Verbindungen mit der Formel I funktionelle Derivate von ω-Hydroxyaminosäure mit der Formel III 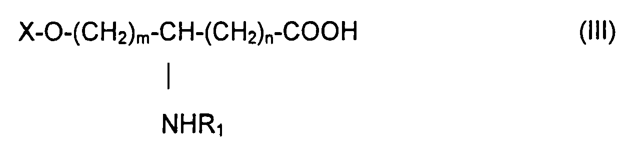 in der R₁ eine Acylgruppe ist, die von einer Carboxylsäure abgeleitet ist, die 2 bis 24 Kohlenstoffatome aufweist, gesättigt oder ungesättigt ist, nicht substituiert ist oder einen oder mehrere Substituenten trägt, die wie oben definiert sind,
m eine ganze Zahl von 1 bis 4 ist,
n gleich 0 ist,
und X ein Dialkyloxy- oder Diaryloxy-phosphorylradikal mit der Formel 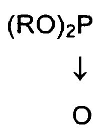 ist.

11. Als Zwischenstufen der Synthese der Verbindungen mit der Formel I nach Anspruch 1 Pseudodipeptide mit der allgemeinen Formel IV 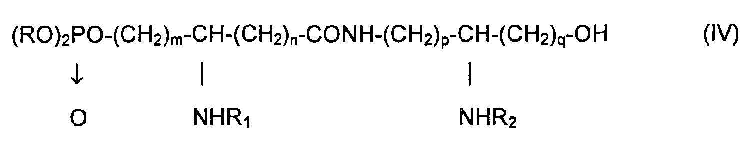 in der die Substituenten R₁, R₂ und die Deskriptoren m, n, p und q wie in Anspruch 1 definiert sind und R ein durch Hydrogenolyse labiles Alkyl- oder Arylradikal ist.

12. Als Zwischenstufen der Synthese der Verbindungen mit der Formel I nach Anspruch 1 Aminoalkohole mit der allgemeinen Formel II 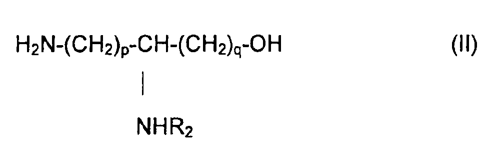 in der R₂ eine Acylgruppe darstellt, die von einer Carboxylsäure abgeleitet ist, die 2 bis 24 Kohlenstoffatome aufweist, gesättigt oder ungesättigt ist, nicht substituiert ist oder einen oder mehrere Substituenten trägt, die wie oben definiert sind,
p eine ganze Zahl gleich 3 oder 4 darstellt,
und q gleich 1 ist.

13. Pharmazeutische Zusammensetzungen mit immunologischem Zweck, die als Wirkstoff mindestens eine Verbindung mit der allgemeinen Formel I nach Anspruch 1 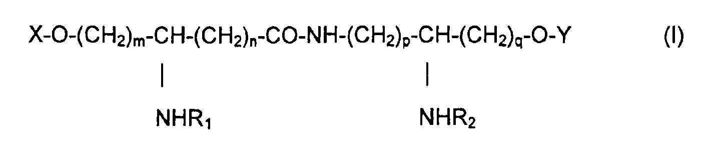 enthalten, in der R1 und R2 jeweils eine Acylgruppe darstellen, die von einer Carboxylsäure abgeleitet ist, die 2 bis 24 Kohlenstoffatome aufweist, gesättigt oder ungesättigt ist, linear oder verzweigt ist, nicht substituiert ist oder einen oder mehrere Substituenten trägt, die in der Gruppe gewählt werden, die aus Hydroxyl, Alkyl, Alkoxy, Acyloxy, Amino, Acylamino, Acylthio und (C₁-C₂₄)Alkylthio gewählt wird,
der Deskriptor m einen Wert von 1 bis 4 annimmt,
der Deskriptor n gleich 0 ist, p einen Wert von 3 oder 4 annimmt und q gleich 1 ist, X und/oder Y jeweils einen Wasserstoff oder eine Phosphongruppe in neutraler oder geladener Form darstellen, mit der Einschränkung, dass mindestens einer der Substituenten X und Y eine Phosphongruppe darstellt,
in Verbindung oder gemischt mit einem inerten, nicht toxischen, pharmazeutisch akzeptablen Exzipienten oder Träger.

14. Pharmazeutische Zusammensetzungen nach Anspruch 13, in denen die Verbindung mit der Formel I eine von jenen ist, bei denen X und/oder Y ein Phosphonradikal darstellen.

15. Pharmazeutische Zusammensetzungen nach Anspruch 13, in denen der Wirkstoff in einer Form vorliegt, die mit einer therapeutisch kompatiblen mineralischen oder organischen Base in ein Salz umgewandelt wurde.

16. Pharmazeutische Zusammensetzungen nach einem der Ansprüche 13 und 15, in denen der Wirkstoff in einer enantiomerisch reinen Form oder in Form eines Stereoisomer-Gemisches vorliegt.
